# FASCICULE DE BREVET EUROPEEN

(11) **EP 3 855 908 B1**
(45) Date de publication et mention de la délivrance du brevet: **15.05.2024**
(21) Numéro de dépôt: 19773452.8
(22) Date de dépôt: 27.09.2019
(51) Int. Cl.: A01K 63/00, A01K 1/03, A01K 29/00

(54) **METHODE ET DISPOSITIF D'OBTENTION DE POISSONS MODELES CONDITIONNES**
VERFAHREN ZUR HERSTELLUNG KONDITIONIERTER FISCHMODELLE
METHOD FOR OBTAINING CONDITIONED FISH MODELS

(30) Priorité: 28.09.2018 FR 1858985
(43) Date de publication de la demande: 04.08.2021
(73) Titulaire: CENTRE NATIONAL DE LA RECHERCHE SCIENTIFIQUE (CNRS), 75016 Paris (FR); Université Paris-Saclay, 91190 Gif-sur-Yvette (FR)
(72) Inventeur: FROC, Cynthia, 91400 Saclay (FR); YAMAMOTO, Kei, 91120 Palaiseau (FR); BLOCH, Solal, 91400 Orsay (FR)
(74) Mandataire: Novagraaf Technologies
(86) Numéro de dépôt international: PCT/EP2019/076177
(87) Numéro de publication internationale: WO 2020/065006

(56) Documents cités:
- US-A- 4 194 620
- SCHLUESSEL V ET AL: "Irrespective of size, scales, color or body shape, all fish are just fish: object categorization in the gray bamboo sharkChiloscyllium griseum", ANIMAL COGNITION, SPRINGER, BERLIN, DE, vol. 18, no. 2, 11 novembre 2014 (2014-11-11), pages 497-507, XP035445964, ISSN: 1435-9448, DOI: 10.1007/S10071-014-0818-0 [extrait le 2014-11-11]
- EVIN DANISMAN ET AL: "Do cleaner fish learn to feed against their preference in a reverse reward contingency task?", ANIMAL COGNITION, SPRINGER, BERLIN, DE, vol. 13, no. 1, 16 juin 2009 (2009-06-16), pages 41-49, XP019763577, ISSN: 1435-9456

## Description

### DOMAINE DE L'INVENTION

La présente invention se situe dans le domaine de la biologie animale, notamment dans les domaines de la biologie du comportement (ou éthologie) et de la pathologie. Elle concerne une méthode d'obtention de poissons modèles conditionnés. Elle concerne également les poissons modèles conditionnés ainsi obtenus, ainsi que leur utilisation pour caractériser une atteinte ou une déficience cognitive, résultant par exemple de maladies neuropsychiatriques ou neurologiques (telles que les maladies neurodégénératives, les addictions ou les lésions au cerveau), s'agissant notamment des effets sur la mémoire de travail et/ou sur les capacités cognitives et/ou sur les capacités d'apprentissage chez un poisson modèle ; ou pour étudier la capacité de régénération du cerveau chez un poisson modèle ; ou encore pour caractériser la mémoire de travail et/ou les capacités cognitives et/ou les capacités d'apprentissage chez un poisson modèle ; ou encore pour identifier des molécules, des composés, des compositions ou des formulations modifiant la mémoire de travail et/ou les capacités cognitives et/ou les capacités d'apprentissage chez un poisson modèle.

Il est également décrit des méthodes pour caractériser une atteinte ou une déficience cognitive, résultant par exemple de maladies neuropsychiatriques ou neurologiques (telles que les maladies neurodégénératives, les addictions ou les lésions au cerveau), s'agissant notamment des effets sur la mémoire de travail et/ou sur les capacités cognitives et/ou sur les capacités d'apprentissage chez un poisson modèle ; ou pour étudier la capacité de régénération du cerveau chez un poisson modèle. La présente invention concerne également un dispositif de conditionnement d'un poisson modèle.

### ART ANTERIEUR

Les études de l'étiologie, de la pathogénie, de la physiopathologie et de la sémiologie des maladies, des syndromes ou des affections touchant les animaux au sens large, incluant les humains, ainsi que la mise au point de thérapies, sont pour une grande part basées sur des études chez des animaux modèles de laboratoire, tels que les rongeurs, les lapins, les singes, etc. En effet, l'utilisation des animaux de laboratoire a permis de comprendre de nombreux mécanismes fondamentaux impliqués dans ces maladies, syndromes ou affections. Elle a également permis l'identification, la mise au point et le test de nombreuses molécules ou formulations thérapeutiques et de méthodes de traitement.

L'utilisation des animaux de laboratoire a notamment joué un rôle fondamental dans la compréhension des capacités cognitives des animaux, mais également des déficiences, des maladies ou des affections résultant en une diminution des capacités cognitives (telles que les maladies neuropsychiatriques ou neurologiques, incluant notamment les maladies neurodégénératives, les addictions ou les lésions au cerveau) et la mise au point de traitements curatifs et/ou préventifs efficaces contre ces maladies.

Dans ce contexte, les poissons modèles de laboratoire, tels que le *Danio rerio* (ou poisson zèbre) présentent de nombreux atouts. En effet, du fait de leur taille (en général inférieure à celle d'un rongeur), leur élevage nécessite peu de place, ce qui permet de maintenir plus d'animaux sur une surface réduite. En outre, leur élevage est facile et peu coûteux. Les poissons modèles se reproduisent facilement et en grand nombre. Leur développement est uniforme entre les individus et rapide (par exemple, le poisson zèbre est considéré adulte à l'âge de trois mois). Certains poissons modèles sont transparents ou peuvent être rendus transparents à l'aide de mutations génétiques bien maitrisées à l'heure actuelle. Enfin, ces poissons modèles peuvent être modifiés par génie génétique.

Les études des capacités cognitives des animaux et de leurs modifications dans un contexte pathologique reposent en général sur un conditionnement de l'animal de laboratoire. De nombreuses méthodes de conditionnement ont été décrites pour la majorité des animaux modèles, tels que le rongeurs, les singes, ou encore les oiseaux. Ces méthodes de conditionnement reposent en général sur la réalisation d'une ou plusieurs tâches, plus ou moins complexes selon l'espèce utilisée.

Des méthodes de conditionnement simples, utilisées chez les rongeurs, les mammifères ou les oiseaux, sont basées sur la reconnaissance et l'association d'un indice visuel à son identique (tâche également appelée « simultaneous matching to sample » ou SMTS). Toutefois, de telles méthodes de conditionnement n'ont pas été décrites ou mises en oeuvre avec le succès attendu chez les poissons.

Des travaux réalisés sur la plupart des poissons modèles ont montré que ceux-ci sont capables de discriminer les stimuli ou indices visuels. Par exemple, la discrimination de la couleur et de l'orientation des lignes a été testée avec succès chez le poisson-zèbre (*Danio rerio* ; Colwill et al., 2005 ; Arthur and Levin, 2001). Il a été par ailleurs montré que le poisson faiblement électrique, *Gnathonemus petersii,* ou encore le cichlidé africain (du genre *Pseudotropheus* sp.), sont capable de distinguer les formes géométriques (Schuster and Amtsfeld, 2002 ; Schluessel et al., 2012). Il a également été montré que le poisson *Xenotoca eiseni* et le poisson rouge (*Carassius auratus*) sont capables de distinguer les contours subjectifs et illusoires (Sovrano and Bisazza, 2009 ; Wyzisk and Neumeyer, 2007).

Toutefois, les travaux visant à transposer, chez les poissons, les méthodes de conditionnement de type SMTS utilisées chez les rongeurs, les mammifères ou les oiseaux, n'ont pas abouti. Par exemple, une étude réalisée sur le cichlidé Malawi (*Pseudotropheus* sp.) n'est pas parvenue à trancher sur sa capacité à associer une forme simple à une forme simple identique (Gierszewski et al., 2013).

En revanche une étude a montré que le poisson rouge (*Carassius auratus*) peut effectuer une tâche de type SMTS (Goldman and Shapiro, 1979). Toutefois, seul un faible nombre d'individus a atteint et maintenu un taux de réussite satisfaisant, malgré un nombre important de répétitions, sur une période supérieure à 2 mois.

Une étude de capacité des requins à distinguer et catégoriser des objets et un dispositif selon le préambule de la revendication 13 sont connus du document SCHLUESSEL V ET AL: "Irrespective of size, scales, color or body shape, all fish are just fish: object categorization in the gray bamboo sharkChiloscyllium griseum", ANIMAL COGNITION, SPRINGER, BERLIN, DE, vol. 18, no. 2, 11 novembre 2014 (2014-11-11), pages 497-507.

Une étude de la capacité des poissons labres nettoyeurs (Labroides dimidiatus) à contrôler un comportement impulsif et un dispositif selon le préambule de la revendication 13 sont connus du document EVIN DANISMAN ET AL: "Do cleaner fish learn to feed against their préférence in a reverse reward contingency task?",ANIMAL COGNITION, SPRINGER, BERLIN, DE, vol. 13, no. 1, 16 juin 2009 (2009-06-16), pages 41-49.

Il existe donc toujours le besoin de développer de nouvelles méthodes efficaces de conditionnement des poissons modèles, par exemple dans le but de caractériser leurs capacités cognitives, étudier l'impact des atteintes ou déficiences cognitives, résultant par exemple de maladies neuropsychiatriques ou neurologiques (telles que les maladies neurodégénératives, les addictions ou les lésions au cerveau) et identifier et mettre au point des traitements visant à prévenir, limiter ou résorber ces atteintes ou déficiences cognitives. La présente invention permet de répondre à ce besoin. Les présents Inventeurs ont démontré pour la première fois que le poisson zèbre était capable de réaliser des tâches de type SMTS. Les Inventeurs ont ainsi mis au point une méthode permettant de conditionner un poisson modèle de manière efficace afin qu'il réalise de telles tâches de façon reproductible. Les Inventeurs ont également montré que, de manière surprenante, le poisson modèle est capable de réaliser des tâches de type DMTS (« delayed matching to sample »), nécessitant de mémoriser une consigne pendant plusieurs secondes. Cette invention permet ainsi de tester la mémoire de travail, ce qui ouvre de nouvelles perspectives de modélisation thérapeutique. Les Inventeurs ont ainsi révélé pour la première fois que le poisson modèle peut être efficacement conditionné, et ce d'une manière approfondie, grâce à la mise au point de méthodes inédites de conditionnement d'un poisson modèle.

### RESUME DE L'INVENTION

Dans le cadre de la présente invention, les Inventeurs ont mis au point, de manière tout à fait inattendue, une nouvelle méthode permettant d'obtenir des poissons modèles conditionnés. Cette nouvelle méthode permet notamment d'obtenir des poissons modèles conditionnés capables d'associer une consigne, ou stimulus, à une réponse correspondante. Les Inventeurs ont pu montrer de manière surprenante que ces poissons modèles conditionnés sont également capables de mémoriser une consigne pendant plusieurs secondes. Les Inventeurs ont notamment montré que ces poissons modèles conditionnés sont capables d'associer une consigne à une réponse correspondante, même après leur avoir imposé un temps de pause (ou délai) suivant le retrait de la consigne (Bloch et al., 2019). De telles capacités n'avaient encore été démontrées chez aucun poisson modèle.

La présente invention concerne donc une méthode d'obtention de poissons modèles conditionnés selon la revendication 1.

Dans le cadre de la présente invention, les Inventeurs ont également mis au point un nouveau dispositif, particulièrement adapté à la mise en oeuvre de la méthode d'obtention de poissons modèles conditionnés.

La présente invention concerne donc également un dispositif de conditionnement d'un poisson modèle selon la revendication 13.

Les poissons modèles conditionnés ou conditionnés de manière approfondie sont particulièrement adaptés à la réalisation d'études de caractérisation de l'effet d'atteintes ou de déficiences cognitives, résultant par exemple de maladies neuropsychiatriques ou neurologiques (telles que les maladies neurodégénératives, les addictions ou les lésions au cerveau, sur la mémoire de travail et/ou les capacités cognitives et/ou les capacités d'apprentissage chez un poisson modèle ; ou d'études de la capacité de régénération du cerveau chez un poisson modèle ; ou encore d'études d'identification de molécules, de composés, de compositions ou de formulations modifiant la mémoire de travail et/ou les capacités cognitives et/ou les capacités d'apprentissage chez un poisson modèle .

L'invention concerne donc l'utilisation du poisson modèle conditionné ou conditionné de manière approfondie à ces effets. Il est également décrit des méthodes de caractérisation de l'effet d'atteintes ou de déficiences cognitives, résultant par exemple de maladies neuropsychiatriques ou neurologiques (telles que les maladies neurodégénératives, les addictions ou les lésions au cerveau), sur la mémoire de travail et/ou les capacités cognitives et/ou les capacités d'apprentissage chez un poisson modèle ; ou des méthodes d'études de la capacité de régénération du cerveau chez un poisson modèle ; ou encore des méthodes d'identification de molécules, de composés, de compositions ou de formulations modifiant la mémoire de travail et/ou les capacités cognitives et/ou les capacités d'apprentissage chez un poisson modèle.

### DESCRIPTION DES FIGURES

D'autres caractéristiques et avantages ressortiront encore de la description qui suit, laquelle est purement illustrative et non limitative. Les figures annexées aident à lire les parties de la description qui y font référence.
La **Figure 1** représente de manière schématique, en vue de dessus, un exemple de dispositif utilisé pour la mise en oeuvre de la méthode d'obtention d'un poisson modèle conditionné.
Les **Figures 2A** et **2B** représentent de manière schématique, le dispositif comprenant une première cloison respectivement en position ouverte et en position fermée.
La **Figure 3** montre un exemple du déroulement d'un essai de la phase de pré-conditionnement d'un poisson modèle.
La **Figure 4** résume un exemple de déroulement d'un essai de la phase de conditionnement. ITI signifie « intertrial interval » et correspond à la période ou intervalle entre les essais.
La **Figure 5** montre un exemple de déroulement d'un essai de la phase de conditionnement, lorsque le poisson choisit une réponse correcte (A et B) ou lorsque le poisson choisit une réponse incorrecte (C et D). La Figure 5 E récapitule les différentes possibilités de réponses présentées dans les figures 5 A à D.
La **Figure 6** résume un exemple de déroulement d'un essai de la phase de conditionnement approfondi.
La **Figure 7** montre un exemple de déroulement d'un essai de la phase de conditionnement approfondi, lorsque le poisson choisit une réponse correcte.
La **Figure 8** montre les pourcentages de réponses correctes de l'animal n°7 au cours de la phase de conditionnement (ou entraînement au SMTS ; la progression est visualisée sous forme de courbe) et de la phase de conditionnement approfondi (ou test/entrainement de DMTS) avec différents délais (visualisé sous forme de points) dans l'Expérience 1. Le seuil de 70% de réponses correctes est indiqué par une ligne pointillée.
La **Figure 9** montre les pourcentages de réponses correctes de l'animal n°1 au cours de la phase de conditionnement (ou test/entraînement au SMTS ; la progression est visualisée sous forme de courbe) et de la phase de conditionnement approfondi (ou test/entrainement au DMTS) avec un délai de 4 secondes (visualisé sous forme d'un point) dans l'Expérience 2. Le seuil de 70% de réponses correctes est indiqué par une ligne pointillée.

### DESCRIPTION DETAILLEE DE L'INVENTION

### Définitions

Par « **environ égal à** », on entend proche, du même ordre de grandeur, sans différence significative.

Par « **poisson** », on entend un animal vertébré aquatique à branchies, pourvus de nageoires et dont le corps est, en général, couvert d'écailles. Un poisson selon l'invention peut être un poisson d'eau douce (c'est-à-dire un poisson dont l'habitat naturel est principalement l'eau douce), un poisson d'eau salée ou encore un poisson d'eau saumâtre. Un poisson s'entend ici comme appartenant aux taxons Pétromyzontides (lamproies), Chondrichtyens (les raies et requins), Actinoptérygiens (les plus communs), Sarcoptérygiens (Dipneustes et Actinistiens). On y associe parfois les Myxinoïdes.

Par « **poisson modèle** », on entend un poisson communément utilisé en laboratoire de recherche à des fins de recherche scientifique. Ainsi, un poisson modèle est étudié en détail pour comprendre un phénomène biologique particulier, en postulant que les résultats et les observations qui seront faites seront au moins partiellement valables pour d'autres organismes, notamment les mammifères dont l'humain. Les poissons modèles appartiennent notamment aux genres *Danio* sp. (par exemple le *Danio rerio*)*, Pseudotropheus* sp., *Gnathonemus* sp. (par exemple le *Gnathonemus petersii*), *Pomacentrus* sp. (par exemple le *Pomacentrus amboinensis*), *Phoxinus* sp. (par exemple le *Phoxinus phoxinus*), *Xenotoca* sp. (par exemple le *Xenotoca eiseni), Astronotus* sp. (par exemple le *Astronotus ocellatus*)*, Oryzias sp.* (par exemple le *Oryzias latipes*), *Nothobranchius sp.* (par exemple le *Nothobranchius furzeri*), Astyanax sp. (par exemple le *Astyanax* mexicanus) et *Carassius* sp. (par exemple le *Carassius auratus*)*.*

Par « **essai** » ou « **trial** » (« essai » en anglais) (les deux termes étant ici considérés comme synonymes) on entend un ensemble d'étapes réalisées successivement, comprenant la détection d'une réponse choisie par un poisson modèle.

Par « **deux essais consécutifs** », on désigne deux essais qui sont réalisés l'un après l'autre :
- soit immédiatement l'un après l'autre, en observant éventuellement un temps de pause (donc selon la séquence « Essai 1 - Essai 2 » ou « Essai 1 - Pause - Essai 2 »). Dans ce cas, les essais sont enchaînés les uns après les autres ;
- soit l'un à la suite de l'autre, en observant une durée d'interruption entre les deux tests (donc selon la séquence « Test 1 - - - Interruption - - - Test 2 ») ;
ledit temps de pause étant généralement plus court que ladite durée d'interruption, comme indiqué dans la description détaillée qui suit. La pause entre deux essais est également appelée « intervalle entre essai » ou « **ITI** » (pour « intertrial interval »).

Par « **série** » ou « **session** » (les deux termes étant ici considérés comme synonymes), on entend la répétition d'au moins deux essais consécutifs.

Par « **deux séries consécutives** », ou « **deux sessions consécutives** », on désigne deux séries ou deux sessions qui sont réalisées l'une après l'autre :
- soit immédiatement l'une après l'autre, en observant éventuellement un temps de pause (donc selon la séquence « Session 1 - Session 2 » ou « Session 1 - Pause - Session 2 ») ;
- soit l'une à la suite de l'autre, en observant une durée d'interruption entre les deux sessions (donc selon la séquence « Session 1 - - - Interruption - - - Session 2 » ;
ledit temps de pause étant généralement plus court que ladite durée d'interruption, comme indiqué dans la description détaillée qui suit.

Par « **temps de pause** » ou « **pause** », on désigne un temps observé entre deux essais consécutifs ou entre deux sessions consécutives, durant lequel aucun essai ou aucune session n'est réalisé. Le temps de pause observé entre deux essais consécutifs (ou ITI) est généralement (mais pas systématiquement) plus court que le temps de pause observé entre deux sessions consécutives, comme indiqué dans la description détaillée qui suit.

Par « **interruption** », ou « **durée d'interruption** » on désigne une durée observée entre deux essais consécutifs ou entre deux sessions consécutives, durant laquelle aucun essai ou aucune session n'est réalisé(e). La durée de l'interruption est généralement plus longue que le temps de pause.

Par « **consigne** », « **indice** », « **sample** » (« échantillon » en anglais), ou encore « **instruction** », on entend un stimulus, de préférence visuel, présenté à un poisson modèle durant un test. Le stimulus de consigne est une instruction ou un indice qui indique au poisson la réponse attendue pour réussir le test, à savoir au moins une réponse identique à la consigne.

Par « **réponse** », on entend un stimulus, de préférence visuel, présenté à un poisson modèle durant un test. On présente au moins deux stimuli de réponse audit poisson modèle, dont au moins un est identique au stimulus de consigne et au moins un est différent du stimulus de consigne. Dans un essai, on s'attend à ce que le poisson choisisse une réponse (un stimulus de réponse parmi les au moins deux stimuli de réponse qui lui seront présentés). Dans un essai réussi, le poisson choisit une réponse qui est identique à la consigne. Dans un essai échoué, le poisson choisit une réponse qui est différente de la consigne.

Par « **réponse correcte** » ou « **bonne réponse** », on entend donc une réponse identique à la consigne, choisie par un poisson modèle durant un essai. Si le poisson modèle choisit une réponse correcte, alors l'essai est réussi. Avantageusement, si le poisson modèle choisit une réponse correcte dans le temps qui lui est imparti pour choisir une réponse durant un essai, alors l'essai est réussi.

Par « **réponse incorrecte** » ou « **réponse fausse** », on entend une réponse différente de la consigne, choisie par un poisson modèle durant un essai, ou une absence de réponse du poisson modèle dans le temps qui lui est imparti pour choisir une réponse durant un essai. Si le poisson modèle choisit une réponse incorrecte, ou ne choisit aucune réponse dans le temps imparti, alors l'essai a échoué.

Par « **taux de réussite** », on entend le pourcentage de réponses correctes choisies/obtenues par un poisson modèle par session. Le taux de réussite se calcule donc en additionnant le nombre total de réponses correctes obtenu lors d'un essai, pour chaque session.

Par « **test** » ou « **tâche** », on entend l'ensemble des sessions et essais menés sur le poisson modèle avec une procédure précise pour évaluer des paramètres comportementaux. Par exemple, le SMTS et le DMTS sont deux tests ou tâches distinctes.

### Méthode d'obtention de poissons modèles conditionnés

Les Inventeurs ont mis au point de manière tout à fait surprenante, une nouvelle méthode permettant d'obtenir des poissons modèles conditionnés. Les Inventeurs ont notamment pu montrer pour la première fois que cette méthode permet notamment d'obtenir des poissons modèles conditionnés, capables d'associer une consigne, ou stimulus, à une réponse correspondante.

La présente invention concerne donc une méthode d'obtention de poissons modèles conditionnés, comprenant une phase de conditionnement a) ; ladite phase de conditionnement étant caractérisée en ce qu'elle consiste en au moins une session d'au moins deux essais consécutifs ; chaque essai comprenant les étapes consistant à :
(a-i) présenter une consigne audit poisson modèle ;
(a-ii) présenter simultanément au moins deux réponses distinctes au poisson modèle de l'étape (a-i) pendant une durée Dᵣₐ, lesdites au moins deux réponses distinctes comprenant au moins une réponse identique à ladite consigne et au moins une réponse différente de ladite consigne ; et
(a-iii) détecter la réponse choisie par le poisson modèle de l'étape (a-ii) parmi lesdites au moins deux réponses distinctes, pendant ladite durée Dᵣₐ,
ledit poisson modèle étant conditionné lorsqu'il choisit au moins une de ladite ou desdites réponses identiques à ladite consigne avec un taux de réussite T1 prédéterminé par session.

En d'autres termes, le poisson modèle conditionné choisit au moins une de ladite ou desdites réponses identiques à ladite consigne avec un taux de réussite T1 prédéterminé par session.

La méthode selon l'invention est donc une méthode d'apprentissage, d'entrainement cognitif, ou encore d'éducation, d'un poisson modèle.

En particulier, l'invention se rapporte à une méthode d'apprentissage de poissons modèles, comprenant une phase de conditionnement a) ; ladite phase de conditionnement étant caractérisée en ce qu'elle consiste en au moins une session d'au moins deux essais consécutifs ; chaque essai comprenant les étapes consistant à :
(a-i) présenter une consigne audit poisson modèle ;
(a-ii) présenter simultanément au moins deux réponses distinctes au poisson modèle de l'étape (a-i) pendant une durée Dᵣₐ, lesdites au moins deux réponses distinctes comprenant au moins une réponse identique à ladite consigne et au moins une réponse différente de ladite consigne ; et
(a-iii) détecter la réponse choisie par le poisson modèle de l'étape (a-ii) parmi lesdites au moins deux réponses distinctes, pendant ladite durée Dᵣₐ,
ledit poisson modèle étant conditionné lorsqu'il choisit au moins une de ladite ou desdites réponses identiques à ladite consigne avec un taux de réussite T1 prédéterminé par session.

Il est possible de présenter ladite consigne durant toute la durée de déroulement d'un essai de ladite phase de conditionnement a). Il est également possible de retirer la consigne, soit en même temps ou pendant l'étape (a-ii) (par exemple au moment de présenter les réponses de l'étape (a-ii), ou pendant la présentation des réponses de l'étape (a-ii)), soit entre les étapes (a-ii) et (a-iii), soit après l'étape (a-iii).

Selon un mode de réalisation, la consigne de l'étape (a-i) de ladite phase de conditionnement a) est présentée durant une durée déterminée D_{ca}, puis retirée, soit en même temps ou pendant l'étape (a-ii) (par exemple au moment de présenter les réponses de l'étape (a-ii), ou pendant la présentation des réponses de l'étape (a-ii)), soit entre les étapes (a-ii) et (a-iii). Ladite durée déterminée D_{ca} peut par exemple aller de 1 seconde à 5 minutes, de préférence de 5 secondes à 4 minutes, de préférence de 10 secondes à 3 minutes, de préférence de 15 secondes à 2 minutes, de préférence de 20 secondes à 1 minute.

Selon un mode de réalisation préféré, ladite durée déterminée D_{ca} va de 1 seconde à 300 secondes, de préférence de 3 seconde à 240 secondes, de préférence de 5 secondes à 240 secondes, de préférence de 10 secondes à 180 secondes, de préférence encore de 15 secondes à 120 secondes, de préférence de 20 secondes à 100 secondes, de préférence de 25 secondes à 90 secondes, de préférence de 30 secondes à 60 secondes, de préférence encore de 6 secondes à 35 secondes, de préférence encore de 7 secondes à 30 secondes, de préférence encore de 8 secondes à 25 secondes, de préférence encore de 9 secondes à 20 secondes, de préférence encore de 10 secondes à 15 secondes. De manière préférée, ladite durée déterminée D_{ca} est choisie parmi 5, 6, 7, 8, 9, 10, 11, 12, 13, 14, 15, 20, 25, 30, 35, 40, 45, 50, 55, 60, 70, 80, 90, 100, 110, 120, 130, 150, 180, 200, 240 et 300 secondes. De manière particulièrement préférée, ladite durée déterminée D_{ca} est choisie parmi environ 30 secondes, 30 secondes, environ 45 secondes, 45 secondes, environ 60 secondes, 60 secondes, environ 90 secondes, 90 secondes, environ 120 secondes, et 120 secondes.

Il est également possible de présenter les réponses de l'étape (a-ii) simultanément à la consigne de l'étape (a-i). Dans ce cas, les étapes (a-i) et (a-ii) sont réalisées de manière simultanée.

Les réponses de l'étape (a-ii) sont présentées au poisson modèle pendant une durée déterminée Dᵣₐ. Ladite durée déterminée Dᵣₐ peut par exemple aller de 1 seconde à 10 minutes, de préférence de 5 secondes à 9 minutes, de préférence de 10 secondes à 8 minutes, de préférence de 15 secondes à 7 minutes, de préférence de 20 secondes à 6 minutes, de préférence de 25 secondes à 5 minutes, de préférence de 30 à secondes à 4 minutes, de préférence de 35 secondes à 3 minutes, de préférence de 40 secondes à 2 minutes, de préférence de 45 secondes à 60 secondes.

Selon un mode de réalisation préféré, ladite durée déterminée Dᵣₐ va de 1 seconde à 300 secondes, de préférence de 3 secondes à 240 secondes, de préférence de 5 secondes à 200 secondes, de préférence de 10 secondes à 180 secondes, de préférence encore de 15 secondes à 120 secondes, de préférence de 20 secondes à 100 secondes, de préférence de 25 secondes à 90 secondes, de préférence de 30 secondes à 60 secondes, de préférence encore de 25 secondes à 35 secondes, de préférence encore de 5 secondes à 90 secondes, de préférence encore de 10 secondes à 80 secondes, de préférence encore de 15 secondes à 60 secondes, de préférence encore de 20 secondes à 50 secondes, de préférence encore de 25 secondes à 45 secondes, de préférence encore de 30 secondes à 35 secondes. De manière particulièrement préférée, ladite durée déterminée Dᵣₐ est choisie parmi 5, 6, 7, 8, 9, 10, 11, 12, 13, 14, 15, 20, 25, 30, 35, 40, 45, 50, 55, 60, 70, 80, 90, 100, 110, 120, 130, 150, 180, 200, 240 et 300 secondes. De préférence, ladite durée déterminée Dᵣₐ est d'environ 30 secondes ou de 30 secondes. Pour un essai donné, lors de l'étape de détection (a-iii), la réponse choisie par le poisson modèle de l'étape (a-ii) parmi lesdites au moins deux réponses distinctes, est dite correcte lorsque que le choix d'au moins une de ladite ou desdites réponses identiques à la consigne de l'étape (a-i) par le poisson modèle est détecté à l'expiration de la durée Dᵣₐ. Autrement dit, la réponse est dite correcte lorsque ledit poisson modèle choisit au moins une de ladite ou desdites réponses identiques à la consigne de l'étape (a-i), pendant la durée de présentation des réponses Dᵣₐ.

Pour un essai donné, lors de l'étape de détection (a-iii), la réponse choisie par le poisson modèle de l'étape (a-ii) parmi lesdites au moins deux réponses distinctes, est dite incorrecte lorsque que le choix d'au moins une de ladite ou desdites réponses différentes de la consigne de l'étape (a-i) par le poisson modèle est détecté à l'expiration de la durée Dᵣₐ. Autrement dit, la réponse est dite incorrecte lorsque ledit poisson modèle choisit au moins une de ladite ou desdites réponses différentes de la consigne de l'étape (a-i), pendant la durée de présentation des réponses Dᵣₐ.

Selon un mode de réalisation, pour un essai donné, la réponse est également dite incorrecte lorsque qu'aucun choix de réponse par le poisson modèle n'est détecté à l'expiration de la durée Dᵣₐ, lors de l'étape de détection (a-iii). Autrement dit, selon ce mode de réalisation, la réponse est également dite incorrecte lorsque le poisson modèle n'a fait aucun choix durant ladite durée Dᵣₐ, lors de l'étape de détection (a-iii). Selon ce mode de réalisation, la réponse est donc dite incorrecte lorsque ledit poisson modèle choisit au moins une de ladite ou desdites réponses différentes de la consigne de l'étape (a-i), ou lorsque ledit poisson n'a fait aucun choix, pendant ladite durée de présentation des réponses Dᵣₐ. Selon ce mode de réalisation, ladite durée Dᵣₐ représente le temps imparti au poisson modèle pour choisir une réponse, lors d'un essai. Ladite durée Dᵣₐ est telle que définie ci-dessus. Avantageusement, chaque essai de la phase de conditionnement a) comprend l'étape additionnelle (a-iii'), réalisée en aval de l'étape (a-iii). Ladite étape (a-iii') consiste à conclure que la réponse choisie par le poisson modèle de l'étape (a-iii), est la réponse choisie par le poisson modèle de l'étape (a-iii) (ou l'absence de réponse, selon le mode de réalisation décrit ci-dessus) à l'expiration de la durée Dᵣₐ.

Selon un mode de réalisation, chaque session de la phase de conditionnement a) comprend de, ou consiste en, 2 à 20 essais, de préférence de 3 à 18 essais, de préférence de 4 à 16 essais, de préférence de 5 à 15 essais, de préférence de 6 à 14 essais, de préférence encore de 7 à 13 essais, de préférence encore de 8 à 12 essais, de préférence encore de 9 à 11 essais, de préférence encore 10 essais. Pour une session donnée, les essais peuvent être enchaînés les uns après les autres.

Lorsque les essais sont réalisés de manière enchaînée, une pause, d'une durée déterminée, peut être observée entre chaque essai d'une session de la phase de conditionnement a). Durant cette pause, aucune consigne n'est présentée au poisson et aucun essai n'est réalisé. De préférence, ladite durée déterminée va de 0,5 à 60 secondes, de préférence encore de 1 à 30 secondes, de préférence encore de 2 à 25 secondes, de préférence encore de 3 à 20 secondes, de préférence encore de 4 à 15 secondes, de préférence encore de 5 à 14 secondes, de manière encore plus préférée de 6 à 13 secondes, de manière toujours plus préférée de 7 à 12 secondes, de manière plus préférentielle encore de 8 à 11 secondes, de manière encore plus préférentielle de 9 à 10 secondes. De préférence, ladite durée déterminée de ladite pause est d'environ 10 secondes, de préférence encore de 10 secondes. La pause entre deux essais est également appelée « intervalle entre essai » ou « **ITI** » (pour « intertrial interval »).

Il est alternativement possible d'introduire une interruption entre chaque essai, ou tous les 2 essais ou plus. Durant cette interruption, aucune consigne n'est présentée au poisson et aucun essai n'est réalisé. La durée d'une telle interruption pourra par exemple aller de plusieurs minutes (par exemple 3, 4, 5, 10, 15, 20, 25, 30, 35, 40, 45, 50 ou 55 minutes) à une heure, ou d'une ou plusieurs heures (par exemple de 1 à 7 heures, ou encore 8 à 23h) à un jour (soit 24h), ou quelques jours (par exemple 2 à 15 jours).

L'interruption est généralement plus longue que la pause.

Selon un mode de réalisation préféré, chaque session de la phase de conditionnement a) comprend, ou consiste en, une répétition d'essais consécutifs dont le nombre est choisi parmi 5 essais, 6 essais, 7 essais, 8 essais, 9 essais, 10 essais, 11 essais, 12 essais, 13 essais, 14 essais et 15 essais. Les Inventeurs ont montré que le nombre de réponses correctes choisies par les poissons modèles est particulièrement optimal lorsqu'une session comporte 10 essais consécutifs. Aussi, avantageusement, chaque session de la phase de conditionnement a) consiste en une répétition de 10 essais consécutifs. Avantageusement, chaque session de la phase de conditionnement a) consiste en une répétition de 10 essais consécutifs réalisés de manière enchaînée. Avantageusement, chaque session de la phase de conditionnement a) consiste en une répétition de 10 essais consécutifs réalisés de manière enchaînée, avec l'observation d'une pause (ITI) telle que définie ci-dessus (de préférence une pause allant de 6 à 13 secondes et de manière particulièrement préférée une pause allant de 8 à 11 secondes, par exemple une pause de 10 secondes), entre chaque essai de la session.

Selon un mode de réalisation, la phase de conditionnement a) comprend de, ou consiste en, 2 à 30 sessions, de préférence de 3 à 25 sessions, de préférence de 4 à 22 sessions, de préférence de 5 à 20 sessions, de préférence de 6 à 19 sessions, de préférence encore de 7 à 18 sessions, de préférence encore de 8 à 17 sessions, de préférence encore de 9 à 16 sessions, de préférence encore de 10 à 15 sessions. Les sessions peuvent être réalisées de manière consécutive (le terme « consécutif » étant défini en relation avec les sessions comme indiqué précédemment en relation avec les essais, c'est-à-dire que les sessions réalisées de manière consécutive sont soit des sessions enchaînées, avec l'observation d'une pause entre chaque session ou non (le terme « pause » étant défini en relation avec les sessions comme indiqué précédemment en relation avec les essais), soit des sessions séparées par une interruption (le terme « interruption » étant défini en relation avec les sessions comme indiqué précédemment en relation avec les essais)). Le temps de pause observé entre deux sessions consécutives est généralement (mais pas systématiquement) plus long que le temps de pause observé entre deux essais consécutifs.

Ainsi, pour une phase de conditionnement a) donnée, les sessions consécutives peuvent être réalisées de manière enchaînée les unes après les autres.

Lorsque les sessions sont réalisées de manière enchaînée, une pause, d'une durée déterminée, peut être observée entre chaque session de la phase de conditionnement a). Durant cette pause, aucune consigne n'est présentée au poisson et aucun essai n'est réalisé. De préférence, ladite durée déterminée va de 15 à 300 secondes, de préférence encore de 20 à 240 secondes, de préférence encore de 25 à 180 secondes, de préférence encore de 30 à 150 secondes, de préférence encore de 40 à 120 secondes, de préférence encore de 45 à 100 secondes, de préférence encore de 50 à 90 secondes, de préférence encore de 60 à 80 secondes, de préférence encore de 65 à 75 secondes. De préférence, ladite durée déterminée est de 15, 30, 45, 60, 90, 120, 240 ou 300 secondes.

Il est alternativement possible d'introduire des interruptions entre chaque session consécutive, ou toutes les 2 sessions consécutives ou plus. Durant cette interruption, aucune consigne n'est présentée au poisson et aucun essai n'est réalisé. La durée d'une telle interruption pourra par exemple aller de plusieurs minutes (par exemple 10, 15, 20, 25, 30, 35, 40, 45, 50 ou 55 minutes) à une heure, ou d'une à quelques heures (par exemple de 1 à 7 heures, ou encore 8 à 23h) à un jour (soit 24h) ou quelques jours (par exemple 2 à 15 jours).

Comme indiqué précédemment pour les essais, l'interruption est généralement plus longue que la pause.

Selon un mode de réalisation préféré, la phase de conditionnement a) comprend, ou consiste en, une répétition de sessions consécutives, dont le nombre est choisi parmi 5 sessions, 6 sessions, 7 sessions, 8 sessions, 9 sessions, 10 sessions, 11 sessions, 12 sessions, 13 sessions, 14 sessions et 15 sessions. Avantageusement, chaque session de la phase de conditionnement a) consiste en une répétition de 10 essais consécutifs.

Selon un mode de réalisation, on réalise de 1 à 10 sessions par jour, de préférence de 1 à 9 sessions par jour, de préférence encore de 2 à 8 sessions par jour, de préférence encore de 3 à 6 sessions par jour, de préférence encore de 4 à 5 sessions par jour.

Selon un mode de réalisation préféré, on réalise 1 session de 10 essais consécutifs par jour. Selon un mode de réalisation préféré, on réalise 1 session de 10 essais consécutifs par jour, avec une interruption allant d'une nuit, à un ou plusieurs jours, entre chaque session (par exemple une interruption de 10h à 18h, d'1 jour, de 2 jours, de 3 jours, de 4 jours, de 5 jours, de 6 jours, de 7 jours, de 8 jours, de 9 jours, de 10 jours, de 11 jours, de 12 jours, de 13 jours, de 14 jours, ou encore de 15 à 60 jours), sur une période allant de 1 à 90 jours, de préférence de 3 à 60 jours, de préférence encore de 5 à 50 jours, de préférence encore de 5 à 40 jours, de préférence encore de 5 à 30 jours, de préférence encore de 5 à 25 jours, de préférence encore de 5 à 20 jours, de préférence encore de 5 à 15 jours, de préférence encore de 5 à 10 jours.

Par « **taux de réussite T1 prédéterminé** », on entend le taux de réussite minimum obtenu par un poisson modèle donné, par session de la phase de conditionnement a), à partir duquel le poisson modèle est conditionné.

Selon un mode de réalisation, ledit taux de réussite T1 est d'au moins 70%, de préférence d'au moins 75%, de préférence encore d'au moins 80%, de préférence encore d'au moins 85%, de préférence encore d'au moins 90%, de préférence encore d'au moins 95%, de préférence encore de 100%.

Selon un mode de réalisation, le poisson modèle est conditionné lorsqu'il obtient un taux de réussite T1 par session d'au moins 70%, de préférence d'au moins 75%, de préférence encore d'au moins 80%, de préférence encore d'au moins 85%, de préférence encore d'au moins 90%, de préférence encore d'au moins 95%, de préférence encore de 100%.

Selon un mode de réalisation préféré, le poisson modèle est conditionné lorsqu'il obtient un taux de réussite T1 par session d'au moins 70%, de préférence d'au moins 75%, de préférence encore d'au moins 80%, de préférence encore d'au moins 85%, de préférence encore d'au moins 90%, de préférence encore d'au moins 95%, de préférence encore de 100%, pour au moins deux sessions consécutives (enchainées ou non). Selon un mode de réalisation préféré, le poisson modèle est conditionné lorsqu'il obtient un taux de réussite T1 par session d'au moins 70% pour au moins deux sessions consécutives (enchainées ou non), de préférence un taux de réussite T1 par session d'au moins 70% pour au moins trois sessions consécutives (enchainées ou non), de préférence encore d'au moins 75%, de préférence encore d'au moins 80%, de préférence encore un taux de réussite T1 par session d'au moins 85%, de préférence encore d'au moins 90%, de préférence encore d'au moins 95%, de préférence encore de 100%, pour au moins trois sessions consécutives (enchainées ou non).

Selon un mode de réalisation préféré, le poisson modèle est conditionné lorsqu'il obtient un taux de réussite T1 par session d'au moins 70% (de préférence encore d'au moins 75%, de préférence encore d'au moins 80%, de préférence encore un taux de réussite T1 par session d'au moins 85%, de préférence encore d'au moins 90%, de préférence encore d'au moins 95% de préférence encore d'au moins 100%) pour au moins trois sessions consécutives, chaque session consistant en ou comprenant au moins une répétition de 10 essais (réalisés de manière enchaînée ou non). Selon un mode de réalisation particulièrement préféré, le poisson modèle est conditionné lorsqu'il obtient un taux de réussite T1 par session d'au moins 70% (de préférence encore d'au moins 75%, de préférence encore d'au moins 80%, de préférence encore un taux de réussite T1 par session d'au moins 85%, de préférence encore d'au moins 90%, de préférence encore d'au moins 95% de préférence encore d'au moins 100%) pour au moins trois sessions consécutives, chaque session consistant en une répétition de 10 essais réalisés de manière enchaînée.

Selon un mode de réalisation, le poisson modèle est conditionné lorsqu'il obtient un taux de réussite T1 par session d'au moins 70% (de préférence encore d'au moins 75%, de préférence encore d'au moins 80%, de préférence encore un taux de réussite T1 par session d'au moins 85%, de préférence encore d'au moins 90%) pour au moins deux sessions consécutives (enchainées ou non), ledit taux de réussite T1 d'au moins 70% pour au moins deux sessions consécutives étant de préférence atteint dès la quinzième session réalisée (enchaînées ou non), de préférence encore dès la douzième session réalisée (enchaînées ou non), de préférence encore dès la onzième session réalisée (enchaînées ou non), de préférence encore dès la dixième session réalisée (enchaînées ou non), de préférence encore dès la neuvième session réalisée (enchainées ou non), de préférence encore dès la huitième session réalisée (enchainées ou non). Dans ce cas, chaque session consiste de préférence en ou comprend de préférence au moins une répétition de 10 essais (réalisés de manière enchaînée ou non).

Selon un mode de réalisation préféré, le poisson modèle est conditionné lorsqu'il obtient un taux de réussite T1 par session d'au moins 70% (de préférence encore d'au moins 75%, de préférence encore d'au moins 80%, de préférence encore un taux de réussite T1 par session d'au moins 85%, de préférence encore d'au moins 90%) pour au moins trois sessions consécutives (enchainées ou non), ledit taux de réussite T1 d'au moins 70% pour au moins trois sessions consécutives étant de préférence atteint dès la quinzième session réalisée (enchainées ou non), de préférence encore dès la douzième session réalisée (enchainées ou non), de préférence encore dès la onzième session réalisée (enchainées ou non), de préférence encore dès la dixième session réalisée (enchainées ou non), de préférence encore dès la neuvième session réalisée (enchainées ou non). Dans ce cas, chaque session consiste de préférence en, ou comprend de préférence, au moins une répétition de 10 essais (réalisés de manière enchaînée ou non).

Selon un mode de réalisation particulièrement préféré, le poisson modèle est conditionné lorsqu'il obtient un taux de réussite T1 par session d'au moins 70%(de préférence encore d'au moins 75%, de préférence encore d'au moins 80%, de préférence encore un taux de réussite T1 par session d'au moins 85%, de préférence encore d'au moins 90%) pour au moins trois sessions consécutives (enchainées ou non), ledit taux de réussite T1 d'au moins 70% pour au moins trois sessions consécutives étant de préférence atteint dès la quinzième session réalisée (enchainées ou non), de préférence encore dès la douzième session réalisée (enchainées ou non), de préférence encore dès la onzième session réalisée (enchainées ou non), de préférence encore dès la dixième session réalisée (enchainées ou non), de préférence encore dès la neuvième session réalisée (enchainées ou non), chaque session consistant en une répétition de 10 essais réalisés de manière enchaînée.

La méthode d'obtention de poissons modèles conditionnés peut être mise en oeuvre simultanément/en parallèle sur un groupe d'individus, lesdits individus appartenant au même genre de poisson modèle ou non, à la même espèce de poisson modèle ou non. Ledit groupe est constitué d'un nombre prédéterminé de poissons modèles, comprenant au minimum trois individus. Par exemple, ledit groupe est constitué de 3, 4, 5, 6, 7, 8, 9, 10, 12, 15 poissons modèles. Ledit nombre prédéterminé d'individus va par exemple d'une dizaine à plusieurs centaines de poissons modèles. De préférence, le groupe d'individus est évalué de manière globale.

Ainsi, selon un premier mode de réalisation, le groupe de poissons modèles est conditionné lorsqu'un nombre prédéterminé d'individus obtient un taux de réussite T1 prédéterminé tel que défini ci-dessus. Le groupe de poissons modèles est conditionné lorsqu'au moins la moitié (50%) des individus du groupe obtient un taux de réussite T1 prédéterminé tel que défini ci-dessus. De préférence, le groupe de poissons modèles est conditionné lorsqu'au moins 55% des individus du groupe obtient un taux de réussite T1 prédéterminé tel que défini ci-dessus, de préférence encore lorsqu'au moins 60% des individus du groupe, de préférence encore lorsqu'au moins 65% des individus du groupe, de préférence encore lorsqu'au moins 70% des individus du groupe, de préférence encore lorsqu'au moins 75% des individus du groupe, de préférence encore lorsqu'au moins 80% des individus du groupe, de préférence encore lorsqu'au moins 85% des individus du groupe, de préférence encore lorsqu'au moins 90% des individus du groupe, de préférence encore lorsqu'au moins 95% des individus du groupe, de préférence encore lorsque 100% des individus du groupe, obtient un taux de réussite T1 prédéterminé tel que défini ci-dessus.

Selon un mode de réalisation, ladite consigne et/ou ladite au moins une réponse est un stimulus visuel. Par exemple, le stimulus visuel est choisi parmi une couleur, une forme géométrique (en 2 ou 3 dimensions), une forme non géométrique (en 2 ou 3 dimensions), une image (en 2 ou 3 dimensions), un objet (en 2 ou 3 dimensions), un signal lumineux ou une combinaison quelconque de l'un de ceux-ci (tel qu'un signal lumineux sous forme de couleur, de forme géométrique (en 2 ou 3 dimensions), de forme non géométrique (en 2 ou 3 dimensions), d'image (en 2 ou 3 dimensions), ou encore d'objet (en 2 ou 3 dimensions)). Lorsque le stimulus visuel consiste en ou comprend un signal lumineux, ledit signal lumineux est émis par une source de lumière, par exemple une source primaire de lumière, telle qu'une lampe (électrique), une diode électroluminescente (ou LED), un laser, ou encore un écran. Le stimulus visuel peut comprendre toute matière naturelle ou synthétique, par exemple du papier, du carton, du verre, du métal, du tissu, ou encore un mélange quelconque de l'une de ces matières. Le stimulus visuel peut par exemple être représenté sur une feuille, une carte, une photographie, un dessin. Le stimulus visuel peut être affiché ou projeté sur un écran. Selon un mode de réalisation, ladite consigne et/ou ladite au moins une réponse est une couleur.

De préférence, le stimulus visuel se situe dans le spectre visible (ayant donc une longueur d'onde dans le vide allant d'environ 380 à environ 780 nanomètres (nm)), ou dans l'ultraviolet (ayant donc une longueur d'onde dans le vide allant d'environ 400 à 100 nm).

Selon un mode de réalisation, l'étape de détection (a-iii) de la réponse choisie par le poisson modèle comprend la détection du passage et/ou de la présence dudit poisson modèle dans une zone de réponse (ou zone de choix). Ladite zone de réponse (ou zone de choix) correspond à un espace ou à un secteur dans lequel est présentée une réponse. Dans le cas d'un stimulus visuel, ladite zone de réponse correspond à un espace ou à un secteur dans lequel est présenté ledit stimulus visuel. La détection de la présence du poisson modèle dans une zone de réponse (ou zone de choix) comprend, ou consiste en, par exemple, la détection du passage (ou franchissement) dudit poisson modèle à travers une ligne (ou encore frontière, limite, ou borne) de démarcation de ladite zone de réponse, ou encore une ligne (ou encore frontière, limite, ou borne) située à l'intérieur de la zone de réponse. Ainsi, ladite ligne est une ligne dédiée à la détection de la réponse choisie par le poisson. Ladite ligne est donc dite « ligne de réponse ». Une telle ligne permet de discriminer la réponse choisie par le poisson. Ainsi, le poisson choisit une réponse lorsqu'il passe à travers ladite ligne de réponse. En d'autres termes, lorsque le poisson passe à travers ladite ligne de réponse, le choix du poisson est validé (valablement détecté). En d'autres termes encore, la détection du passage du poisson à travers ladite ligne de réponse correspond à la détection de la réponse choisie par le poisson. L'homme du métier comprendra aisément qu'il peut être suffisant que le poisson passe une partie de son corps à travers ladite ligne de réponse pour que son passage à travers la ligne de réponse soit détecté. Par exemple, il peut être suffisant que le poisson passe sa tête à travers la ligne de réponse pour que son passage à travers la ligne soit détecté. De préférence, le passage du poisson à travers la ligne de réponse est détecté lorsque le poisson passe entièrement (c'est-à-dire la totalité de son corps) à travers la ligne.

Ladite ligne de réponse peut être virtuelle ou non. La ligne de réponse peut être marquée ou tracée par un trait visible ou un objet longiligne (mobile ou non), ou encore marquée à l'aide d'un signal lumineux. Alternativement, ladite ligne de réponse peut comprendre, ou consister, en une cloison ou une paroi (mobile ou non). Ladite ligne de réponse peut par exemple comprendre ou consister en une cloison ou une paroi séparant une zone de départ, dans laquelle la consigne est présentée, d'une zone de réponse (ou zone de choix), dans laquelle est présentée au moins une réponse.

Alternativement ou en combinaison, ladite ligne de réponse (et/ou ladite zone de réponse ou zone de choix) consiste en, ou comprend, un orifice (ou une ouverture, un trou ou encore une embrasure, une cavité ou une niche). Dans ce cas, l'orifice est un orifice dédié à la détection de la réponse choisie par le poisson. Ledit orifice est donc dit « orifice de réponse ». Un tel orifice permet de discriminer la réponse choisie par le poisson. Ainsi, le poisson choisit une réponse lorsqu'il passe dans ledit orifice de réponse. En d'autres termes, lorsque le poisson passe dans ledit orifice de réponse, le choix du poisson est validé (valablement détecté). En d'autres termes encore, la détection du passage du poisson dans ledit orifice de réponse correspond à la détection de la réponse choisie par le poisson. Ledit orifice de réponse est par exemple un orifice d'un distributeur de récompense. Ledit orifice de réponse peut être un orifice dans une paroi, tel qu'une fenêtre, une ouverture, un trou ou encore une embrasure, une cavité ou une niche. Dans ce cas, l'orifice de réponse peut par exemple être un orifice dans une paroi séparant une zone de départ, dans laquelle la consigne est présentée, et une zone de choix, dans laquelle est présentée au moins une réponse. L'homme du métier comprendra aisément qu'il peut être suffisant que le poisson passe une partie de son corps dans l'orifice de réponse pour que son passage dans l'orifice soit détecté. Par exemple, dans le cas d'un orifice d'un distributeur de récompense, il peut être suffisant que le poisson passe sa tête dans ledit orifice, de manière à saisir sa récompense, pour que son passage dans l'orifice de réponse soit détecté.

Avantageusement, l'étape de détection (a-iii) de la réponse choisie par le poisson modèle est effectuée au moyen de capteurs. Lorsque la détection de la réponse choisie par le poisson modèle comprend la détection du passage dudit poisson modèle dans une zone de réponse telle que définie ci-dessus, par exemple la détection de son passage à travers une ligne de réponse telle que définie ci-dessus (par exemple un orifice tel que défini ci-dessus), au moins un capteur est positionné de manière à détecter le passage du poisson dans ladite zone de réponse, par exemple à travers la dite ligne de réponse (par exemple dans ledit orifice de réponse). Alternativement ou en combinaison, la détection de la réponse choisie par le poisson modèle (par exemple la détection du passage dudit poisson modèle dans une zone de réponse) est visuelle, par exemple une détection directement par l'expérimentateur.

Il peut être avantageux de détecter le passage du poisson à travers une ligne qui n'est pas dédiée à la détection de la réponse choisie par le poisson (par exemple le passage du poisson dans un orifice qui n'est pas dédié à la détection de la réponse choisie par le poisson). Une telle détection permet notamment le suivi du poisson durant le déroulement d'un essai et/ou d'une session et ou d'un test. Ladite ligne qui n'est pas dédiée à la détection de la réponse choisie par le poisson est donc dite « ligne de suivi » (ou encore ligne de traçage ou « tracking », ou encore ligne de « monitoring »). Ladite ligne de suivi est définie telle que la ligne de réponse. Ladite ligne de suivi comprend par exemple, ou consiste en, un orifice, dit « orifice de suivi » (ou encore orifice de traçage ou « tracking », ou encore orifice de « monitoring »). Ledit orifice de suivi est par exemple un orifice dans une paroi, tel qu'une fenêtre, une ouverture, un trou ou encore une embrasure, une cavité ou une niche. Dans ce cas, l'orifice de suivi peut par exemple être un orifice dans une paroi séparant une zone de départ, dans laquelle la consigne est présentée, et une zone de choix, dans laquelle sont présentées lesdites au moins deux réponses. Dans ce cas, la détection du passage du poisson dans ledit orifice de suivi indique que le poisson sort de la zone de départ et se prépare à choisir (ou a déjà choisi) une réponse. Avantageusement, la détection du passage du poisson dans un orifice de suivi est effectuée au moyen de capteurs. Alternativement ou en combinaison, la détection du passage du poisson dans un orifice de suivi est visuelle, par exemple une détection directement par l'expérimentateur.

Alternativement ou en combinaison, il peut être avantageux de détecter les mouvements et/ou la position du poisson modèle durant tout ou partie d'un essai et/ou d'une session et/ou de la phase de conditionnement a). Une telle détection permet notamment le suivi (ou encore le traçage ou « tracking », ou encore le « monitoring ») du poisson durant le déroulement de tout ou partie d'un essai et/ou d'une session et/ou de la procédure de conditionnement a). La détection suivie du poisson est par exemple effectuée au moyen de capteurs (par exemple au moyen de plusieurs capteurs positionnés dans différentes zones (zone de départ, zone de réponse), de caméra(s) de suivi de mouvement, et/ou d'un système de « tracking » ou de « monitoring ». Alternativement ou en combinaison, la détection suivie peut être visuelle, par exemple une détection directement par l'expérimentateur.

Selon un mode de réalisation, la phase de conditionnement a) comprend en outre une étape additionnelle effectuée en aval de l'étape de détection (a-iii). Ladite étape additionnelle comprend de préférence :
- la distribution d'une récompense audit poisson modèle lorsqu'il a choisi au moins une de ladite ou desdites réponses identiques à ladite consigne pendant ladite durée Dᵣₐ, ladite récompense comprenant de préférence de la nourriture adaptée audit poisson modèle; ou
- la non distribution de récompense audit poisson modèle lorsqu'il a choisi au moins une de ladite ou desdites réponses différentes de ladite consigne pendant ladite durée Dᵣₐ et, optionnellement, l'isolement et/ou la mise en obscurité dudit poisson modèle dudit poisson modèle, lorsqu'il a choisi au moins une de ladite ou desdites réponses différentes de ladite consigne (ou optionnellement, lorsqu'il n'a choisi aucune réponse) pendant ladite durée Dᵣₐ,

La présente invention concerne donc une méthode d'obtention de poissons modèles conditionnés, comprenant une phase de conditionnement a) ; ladite phase de conditionnement étant caractérisée en ce qu'elle consiste en au moins une session d'au moins deux essais consécutifs ; chaque essai comprenant les étapes consistant à :
(a-i) présenter une consigne audit poisson modèle ;
(a-ii) présenter simultanément au moins deux réponses distinctes au poisson modèle de l'étape (a-i) pendant une durée Dᵣₐ, lesdites au moins deux réponses distinctes comprenant au moins une réponse identique à ladite consigne et au moins une réponse différente de ladite consigne ;
(a-iii) détecter la réponse choisie par le poisson modèle de l'étape (a-ii) parmi lesdites au moins deux réponses distinctes, pendant ladite durée Dᵣₐ ; et (a-iv) distribuer une récompense audit poisson modèle lorsqu'il a choisi au moins une de ladite ou desdites réponses identiques à ladite consigne pendant ladite durée Dᵣₐ, ladite récompense comprenant de préférence de la nourriture adaptée audit poisson modèle; ou
ne pas distribuer de récompense audit poisson modèle lorsqu'il a choisi au moins une de ladite ou desdites réponses différentes de ladite consigne pendant ladite durée Dᵣₐ et, optionnellement, isoler et/ou mettre en obscurité ledit poisson modèle lorsqu'il a choisi au moins une de ladite ou desdites réponses différentes de ladite consigne (ou optionnellement, lorsqu'il n'a choisi aucune réponse) pendant ladite durée Dᵣₐ.
ledit poisson modèle étant conditionné lorsqu'il choisit au moins une de ladite ou desdites réponses identiques à ladite consigne avec un taux de réussite T1 prédéterminé par session.

Avantageusement, ladite récompense est distribuée par un distributeur de récompense. Par exemple, la distribution de ladite récompense est déclenchée par le passage et/ou la détection de la présence dudit poisson modèle dans une zone de réponse, par exemple à travers une ligne de réponse (comprenant ou consistant en un orifice de réponse). Avantageusement, ladite zone de réponse, ladite ligne de réponse et/ou ledit orifice de réponse est tel(le) que défini(e) ci-dessus. Notamment, ladite ligne de réponse et/ou ledit orifice de réponse est dédié(e) à la détection de la réponse choisie par le poisson. Notamment, ledit orifice de réponse peut être un orifice d'un distributeur de récompense ou encore un orifice dans une paroi, tel qu'une fenêtre, une ouverture, un trou ou encore une embrasure, une cavité ou une niche.

Avantageusement, ladite récompense n'est distribuée au poisson modèle que lorsque celui-ci a choisi au moins une réponse correcte pendant ladite durée Dᵣₐ. Dans ce cas, le poisson modèle ne reçoit pas de récompense lorsque celui-ci a choisi au moins une réponse incorrecte pendant ladite durée Dᵣₐ. La réponse correcte et la réponse incorrecte sont telles que définies ci-dessus. Notamment, selon un mode de réalisation, pour un essai donné, la réponse est également dite incorrecte lorsque qu'aucun choix de réponse par le poisson modèle n'est détecté à l'expiration de la durée Dᵣₐ, lors de l'étape de détection (a-iii). Autrement dit, selon ce mode de réalisation, la réponse est également dite incorrecte lorsque le poisson modèle n'a fait aucun choix durant ladite durée Dᵣₐ, lors de l'étape de détection (a-iii). Selon ce mode de réalisation, la réponse est donc dite incorrecte lorsque ledit poisson modèle choisit au moins une de ladite ou desdites réponses différentes de la consigne de l'étape (a-i), ou lorsque ledit poisson n'a fait aucun choix, pendant ladite durée de présentation des réponses Dᵣₐ. Selon ce mode de réalisation, ladite durée Dᵣₐ représente le temps imparti au poisson modèle pour choisir une réponse, lors d'un essai. Ladite durée Dᵣₐ est telle que définie ci-dessus.

Selon un mode de réalisation, le poisson modèle est isolé lorsque celui-ci a choisi au moins une réponse incorrecte pendant ladite durée Dᵣₐ (et/ou lorsque le poisson n'a fait aucun choix pendant ladite durée Dᵣₐ, selon le mode de réalisation). Selon cette étape d'isolement, ledit poisson modèle peut par exemple être isolé (ou encore enfermé, ou encore confiné) dans une zone définie, pour une durée déterminée Dᵢ. Avantageusement, ladite durée déterminée Dᵢ durant laquelle le poisson est isolé va de 5 secondes à 60 secondes, de préférence de 10 secondes à 50 secondes, de préférence encore de 20 secondes à 40 secondes, de préférence encore de 25 secondes à 35 secondes, de préférence encore, ladite durée est choisie parmi 10 secondes, 15 secondes, 20 secondes, 25 secondes, 30 secondes, 35 secondes, 40 secondes et 45 secondes, et de manière particulièrement préférée d'environ 30 secondes ou de 30 secondes. Le poisson modèle est par exemple isolé dans la zone de départ telle que définie ci-dessus. Alternativement ou en combinaison, le poisson modèle est placé dans l'obscurité lorsque celui-ci a choisi au moins une réponse incorrecte pendant ladite durée Dᵣₐ (et/ou lorsque le poisson n'a fait aucun choix pendant ladite durée Dᵣₐ, selon le mode de réalisation), pour ladite durée déterminée Dᵢ.

Selon un mode de réalisation, ladite récompense comprend ou consiste en de la nourriture (ou un aliment) adaptée audit poisson modèle. Par exemple, la récompense comprend ou consiste en un granulé ou une pastille ou un flocon de nourriture (ou d'un aliment) séchée et compactée, tel que les granulés, pastilles ou flocons disponibles dans le commerce (ou encore « pellet », par exemple de type Gemma^{®}, commercialisé par SKRETTING, tel que le Gemma Micro^{®} (notamment le Gemma Micro^{®} 600)). Par exemple, la récompense comprend ou consiste en de la larve d'insecte, telle que de la larve de moustique, de mouche ou de moucheron, ladite larve étant par exemple fraiche, séchée, lyophilisée, congelée ou surgelée. La récompense peut également comprendre ou consister en un mélange de granulé, de pastille ou de flocon de nourriture et de larve d'insecte (moustique, mouche, moucheron etc.). L'homme du métier saura définir la nourriture adaptée au poisson modèle utilisé.

Les Inventeurs ont montré, de manière avantageuse, que le nombre de réponses correctes choisies par les poissons modèles était particulièrement optimal lorsque la récompense est de la nourriture appétente (ou un aliment appétent). Avantageusement, la récompense comprend, ou consiste en, de la nourriture appétente (ou un aliment appétent).

Par « **nourriture appétente** » ou « **aliment appétent** », on entend ici de la nourriture ou un aliment qui va attirer un poisson modèle pour la ou le consommer. Ainsi, un poisson modèle va être attiré de manière préférentielle vers de la nourriture appétente ou un aliment appétent, pour la ou le consommer. Autrement dit, de la nourriture ou un aliment est appétent(e) pour un poisson modèle si le poisson modèle montre une préférence pour ladite nourriture ou ledit aliment. Par exemple, en présence de plusieurs types de nourriture ou de plusieurs aliments différents, un poisson modèle sélectionnera (préférera) la nourriture la plus appétente ou l'aliment le plus appétent. Cette attirance (ou préférence) peut provenir de l'un quelconque des sens du poisson modèle, mais est habituellement liée, entre autres, à l'aspect, à l'odeur, au goût, à l'arôme, à la saveur, la texture, et/ou la sensation en bouche. Par exemple, de la nourriture appétente ou un aliment appétent pour un poisson modèle est de la nourriture ou un aliment qui ressemble visuellement et/ou olfactivement et/ou gustativement à la nourriture consommée (ou à un aliment consommé) par le poisson modèle considéré dans son milieu naturel.

Les Inventeurs ont également montré que, de manière avantageuse, le nombre de réponses correctes choisies par les poissons modèles était particulièrement optimal lorsque la récompense est de la nourriture ou un aliment nutritionnellement pauvre. Avantageusement, la récompense comprend, ou consiste en, de la nourriture ou un aliment nutritionnellement pauvre. Avantageusement encore, la récompense comprend, ou consiste en, de la nourriture appétente et nutritionnellement pauvre ou en un aliment appétent et nutritionnellement pauvre.

Par « **nourriture nutritionnellement pauvre** » ou « **nourriture appauvrie** » ou « **nourriture pauvre ou appauvrie sur le plan nutritionnel** », « **aliment nutritionnellement pauvre** » ou **«aliment appauvri** » ou « **aliment pauvre ou appauvri sur le plan nutritionnel** », on entend une dose (ou ration) d'une nourriture ou d'un aliment adapté(e) au poisson modèle utilisé, apportant moins de 20%, de préférence moins de 15%, de préférence encore moins de 10%, de préférence encore moins de 5%, de préférence encore moins de 3%, de la quantité journalière moyenne en matière grasse, et/ou protéique, et/ou glucidique, nécessaire audit poisson modèle adulte.

Des exemples de nourriture ou d'aliment appétent(e) et/ou nutritionnellement pauvre, pour poisson modèle comprennent la larve d'insecte lyophilisée, telle que de la larve lyophilisée de moustique, de mouche ou de moucheron. Des larves d'insecte lyophilisées sont disponibles dans le commerce, par exemple les larves d'insecte lyophilisées commercialisées par la société JBL, par exemple les larves de la gamme NovoFil^{®}. Des exemples de nourriture ou d'aliment nutritionnellement pauvre pour poisson modèle comprennent des granulés, pastilles ou flocons de nourriture séchée et compactée dont la teneur en matière grasse, et/ou protéique, et/ou glucidique est pauvre ou appauvrie (par exemple une pastille qui apporte moins de 20%, de préférence moins de 15%, de préférence encore moins de 10%, de préférence encore moins de 5%, de préférence encore moins de 3%, de la quantité journalière moyenne en matière grasse, et/ou protéique, et/ou glucidique, nécessaire à un poisson modèle adulte). Les Inventeurs ont notamment montré que le taux de réussite T1 était particulièrement optimal lorsque la récompense comprend de la larve d'insecte lyophilisée. Les Inventeurs ont également montré qu'un taux de réussite T1 d'au moins 70% était atteint plus rapidement (c'est-à-dire qu'un taux de réussite T1 d'au moins 70% était atteint après avoir réalisé un nombre plus faible de sessions consécutives), lorsque la récompense comprend de la larve d'insecte. Avantageusement, la récompense comprend ou consiste en de la larve d'insecte lyophilisée, telle que de la larve lyophilisée de moustique, de mouche ou de moucheron, ou un mélange de larve lyophilisée de moustique et/ou de mouche et/ou de moucheron. De préférence, la récompense comprend ou consiste en de la larve lyophilisée de moustique.

Selon un mode de réalisation, ledit poisson modèle est choisi parmi les poissons modèles appartenant aux genres *Danio* sp., *Pseudotropheus* sp., *Gnathonemus* sp., *Pomacentrus* sp., *Phoxinus* sp., *Xenotoca* sp., *Astronotus* sp., *Oryzias sp., Nothobranchius sp., Astyanax sp.* et *Carassius* sp., de préférence parmi le *Danio rerio, Metriaclima zébra, Gnathonemus petersii, Pomacentrus amboinensis, Phoxinus phoxinus, Xenotoca eiseni, Astronotus ocellatus, Oryzias latipes, Nothobranchius furzeri, Astyanax mexicanus* et *Carassius* auratus.Selon un mode de réalisation, la méthode de l'invention comprend en outre une phase de pré-conditionnement (ou de préapprentissage, ou de pré-éducation, ou de pré-entrainement, ou encore de « pré-training ») pré-a), effectuée en amont de la phase de conditionnement a). Avantageusement, ladite phase de pré-conditionnement comprend une étape d'adaptation du poisson modèle à l'environnement dans lequel la phase de conditionnement a) et/ou la phase de conditionnement approfondie b) se dérouleront. L'homme du métier sait déterminer les étapes comprises dans ladite phase de pré-conditionnement.

Par exemple, ladite phase de pré-conditionnement pré-a) comprend une ou des étapes d'adaptation du poisson modèle au dispositif tel que défini ci-dessous. Par exemple, dans ce cas, ladite étape d'adaptation consiste en, ou comprend, au moins une session d'au moins deux essais ;
chaque essai, comprenant ou consistant à récompenser ledit poisson modèle s'il approche de la zone de réponse dudit dispositif à une distance maximum déterminée, en l'absence de toute consigne ou de toute réponse, dans laquelle ladite distance maximum déterminée diminue à chaque nouvel essai.

Avantageusement, ledit poisson modèle est préconditionné lorsque le poisson modèle est familiarisé avec son environnement. Le poisson est alors apte à générer une réponse comportementale claire. En d'autres termes, le poisson est préconditionné/préparé à la mise en oeuvre de la méthode de conditionnement/d'apprentissage décrite ci-dessus, dans un environnement donné.

Ladite phase de pré-conditionnement pré-a) comprend par exemple une étape d'adaptation du poisson modèle à un distributeur de nourriture qui sera utilisé durant la phase de conditionnement a) et/ou la phase de conditionnement approfondie b) (voir la section suivante).

Par exemple, dans ce cas, la phase de pré-conditionnement (ou de préapprentissage, ou de pré-éducation, ou de pré-entrainement, ou encore de « pré-training ») pré-a), consiste en, ou comprend, au moins une session d'au moins deux tests consécutifs ;

chaque test comprenant les étapes consistant à :
(pré-a-i) présenter une récompense dans un distributeur de récompense audit poisson modèle ;
(pré-a-ii) détecter la prise de la récompense de l'étape (pré-a-i) par ledit poisson dans le distributeur de récompense, par exemple au moyen de capteurs ;
ledit poisson modèle étant préconditionné lorsqu'il choisit au moins une de ladite ou desdites réponses identiques à ladite consigne avec un taux de réussite T3 prédéterminé par session.

Un essai est réussi lorsque le poisson modèle saisi la récompense dans le distributeur de récompense.

Par « **taux de réussite T3 prédéterminé** », on entend le taux de réussite minimum obtenu par un poisson modèle donné, par session de la phase de pré-conditionnement pré-a), à partir duquel le poisson modèle est préconditionné. Avantageusement, le taux de réussite T3 est défini en relation avec de la phase de pré-conditionnement pré-a) comme indiqué précédemment en relation avec la phase de conditionnement a). Ainsi, selon un mode de réalisation, ledit taux de réussite T3 est d'au moins 70%, de préférence d'au moins 75%, de préférence encore d'au moins 80%, de préférence encore d'au moins 85%, de préférence encore d'au moins 90%, de préférence encore d'au moins 95%, de préférence encore de 100%. Selon un mode de réalisation préféré, le poisson modèle est préconditionné lorsqu'il obtient un taux de réussite T3 par session d'au moins 70%, de préférence d'au moins 75%, de préférence encore d'au moins 80%, de préférence encore d'au moins 85%, de préférence encore d'au moins 90%, de préférence encore d'au moins 95%, de préférence encore de 100%.

La consigne, la réponse, l'essai, le nombre d'essais, la session, le nombre de sessions, la récompense, les différentes durées (par exemple la durée de la pause et la durée de l'interruption), l'étape de détection et la détection, et le poisson modèle de la phase de pre-conditionnement pré-a), sont tels que définis ci-dessus pour la phase de conditionnement a). En particulier, les essais de la phase de pré-conditionnement pré-a) peuvent être réalisés de manière enchainée ou non ; les sessions de la phase de pré-conditionnement pré-a) peuvent être réalisées de manière consécutive, de manière enchainée ou non ; tel que défini ci-dessus en relation avec la phase de conditionnement a).

La présente invention concerne également un poisson modèle conditionné, susceptible d'être obtenu par la méthode selon l'invention telle que décrite ci-dessus.

### Méthode d'obtention de poissons modèles conditionnés de manière approfondie

Les Inventeurs ont pu montrer de manière surprenante que les poissons modèles conditionnés selon la phase de conditionnement a) de la méthode de l'invention, sont également capables de mémoriser une consigne pendant plusieurs secondes. Les Inventeurs ont notamment montré que ces poissons modèles conditionnés sont capables d'associer une consigne à une réponse correspondante, même après leur avoir imposé un délai suivant le retrait de la consigne. De telles capacités n'avaient encore jamais été démontrées chez le poisson modèle. Les Inventeurs ont ainsi mis au point, de manière tout à fait surprenante, la première méthode permettant d'obtenir des poissons modèles conditionnés de manière approfondie. Une telle méthode, permettant d'obtenir de telles capacités chez le poisson modèle, n'avait encore jamais été décrite.

Ainsi, l'invention concerne en outre une méthode d'obtention de poisson modèles conditionnés de manière approfondie, comprenant une phase de conditionnement approfondi b) caractérisée en ce qu'elle consiste en au moins une session d'au moins deux essais consécutifs ; chaque essai comprenant les étapes consistant à :
(b-i) présenter une consigne audit poisson modèle conditionné durant une durée déterminée ;
(b-ii) retirer ladite consigne puis observer un délai d'une durée D1 prédéterminée, durant laquelle aucune consigne n'est présentée au poisson modèle de l'étape (b-i) ;
(b-iii) présenter simultanément au moins deux réponses distinctes au poisson modèle de l'étape (b-ii) pendant une durée D_{rb}, lesdites au moins deux réponses distinctes comprenant au moins une réponse identique à ladite consigne et au moins une réponse différente de ladite consigne ; et
(b-iv) détecter la réponse choisie par le poisson modèle de l'étape (b-iii) parmi lesdites au moins deux réponses distinctes, pendant ladite durée D_{rb} ;
ledit poisson modèle étant conditionné de manière approfondie lorsqu'il choisit au moins une de ladite ou desdites réponses identiques à ladite consigne avec un taux de réussite T2 prédéterminé par session.

En d'autres termes, le poisson modèle conditionné de manière approfondie choisit au moins une de ladite ou desdites réponses identiques à ladite consigne avec un taux de réussite T2 prédéterminé par session.

La méthode selon l'invention est donc une méthode d'apprentissage approfondi, d'entrainement cognitif approfondi, ou encore d'éducation approfondie, d'un poisson modèle.

Ainsi, selon un mode de réalisation, la méthode d'obtention d'un poisson modèle conditionné selon l'invention comprend en outre une phase de conditionnement approfondi b) caractérisée en ce qu'elle consiste en au moins une session d'au moins deux essais consécutifs ; chaque essai comprenant les étapes consistant à :
(b-i) présenter une consigne audit poisson modèle conditionné durant une durée déterminée ;
(b-ii) retirer ladite consigne puis observer un délai d'une durée D1 prédéterminée, durant laquelle aucune consigne n'est présentée au poisson modèle de l'étape (b-i) ;
(b-iii) présenter simultanément au moins deux réponses distinctes au poisson modèle de l'étape (b-ii) pendant une durée D_{rb}, lesdites au moins deux réponses distinctes comprenant au moins une réponse identique à ladite consigne et au moins une réponse différente de ladite consigne ; et
(b-iv) détecter la réponse choisie par le poisson modèle de l'étape (b-iii) parmi lesdites au moins deux réponses distinctes, pendant ladite durée D_{rb} ;
ledit poisson modèle étant conditionné de manière approfondie lorsqu'il choisit au moins une de ladite ou desdites réponses identiques à ladite consigne avec un taux de réussite T2 prédéterminé par session.

La présente invention concerne donc une méthode d'obtention de poissons modèles conditionnés, comprenant une phase de conditionnement a) ; ladite phase de conditionnement étant caractérisée en ce qu'elle consiste en au moins une session d'au moins deux essais consécutifs ; chaque essai comprenant les étapes consistant à :
(a-i) présenter une consigne audit poisson modèle ;
(a-ii) présenter simultanément au moins deux réponses distinctes au poisson modèle de l'étape (a-i) pendant une durée Dᵣₐ, lesdites au moins deux réponses distinctes comprenant au moins une réponse identique à ladite consigne et au moins une réponse différente de ladite consigne ; et
(a-iii) détecter la réponse choisie par le poisson modèle de l'étape (a-ii) parmi lesdites au moins deux réponses distinctes, pendant ladite durée Dᵣₐ,
   ledit poisson modèle étant conditionné lorsqu'il choisit au moins une de ladite ou desdites réponses identiques à ladite consigne avec un taux de réussite T1 prédéterminé par session ;
   ladite phase de conditionnement a) étant suivie d'une phase de conditionnement approfondi b) caractérisée en ce qu'elle consiste en au moins une session d'au moins deux essais consécutifs ; chaque essai comprenant les étapes consistant à :
(b-i) présenter une consigne audit poisson modèle conditionné durant une durée déterminée ;
(b-ii) retirer ladite consigne puis observer un délai d'une durée D1 prédéterminée, durant laquelle aucune consigne n'est présentée au poisson modèle de l'étape (b-i) ;
(b-iii) présenter simultanément au moins deux réponses distinctes au poisson modèle de l'étape (b-ii) pendant une durée D_{rb}, lesdites au moins deux réponses distinctes comprenant au moins une réponse identique à ladite consigne et au moins une réponse différente de ladite consigne ; et
(b-iv) détecter la réponse choisie par le poisson modèle de l'étape (b-iii) parmi lesdites au moins deux réponses distinctes, pendant ladite durée D_{rb} ;
ledit poisson modèle étant conditionné de manière approfondie lorsqu'il choisit au moins une de ladite ou desdites réponses identiques à ladite consigne avec un taux de réussite T2 prédéterminé par session.

Selon un mode de réalisation, la consigne de l'étape (b-i) de ladite phase de conditionnement approfondi b) est présentée durant une durée déterminée D_{cb}, avant d'être retirée lors de l'étape (b-ii). Ladite durée déterminée D_{cb} peut par exemple aller de 1 seconde à 5 minutes, de préférence de 5 secondes à 4 minutes, de préférence de 10 secondes à 3 minutes, de préférence de 15 secondes à 2 minutes, de préférence de 20 secondes à 1 minute.

Selon un mode de réalisation préféré, ladite durée déterminée D_{cb} va de 1 seconde à 300 secondes, de préférence de 3 seconde à 240 secondes, de préférence de 5 secondes à 240 secondes, de préférence de 10 secondes à 180 secondes, de préférence encore de 15 secondes à 120 secondes, de préférence de 20 secondes à 100 secondes, de préférence de 25 secondes à 90 secondes, de préférence de 30 secondes à 60 secondes, de préférence encore de 6 secondes à 35 secondes, de préférence encore de 7 secondes à 30 secondes, de préférence encore de 8 secondes à 25 secondes, de préférence encore de 9 secondes à 20 secondes, de préférence encore de 10 secondes à 15 secondes. De manière particulièrement préférée, ladite durée déterminée D_{cb} est choisie parmi 5, 6, 7, 8, 9, 10, 11, 12, 13, 14, 15, 20, 25, 30, 35, 40, 45, 50, 55, 60, 70, 80, 90, 100, 110, 120, 130, 150, 180, 200, 240 et 300 secondes. De manière particulièrement préférée, ladite durée déterminée D_{cb} est choisie parmi environ 30 secondes, 30 secondes, environ 45 secondes, 45 secondes, environ 60 secondes, 60 secondes, environ 90 secondes, 90 secondes, environ 120 secondes, et 120 secondes.

Selon un mode de réalisation, la durée D_{cb} de la phase de conditionnement approfondie b) est égale à, ou environ égale à, la durée D_{ca} de la phase de conditionnement a). Selon un mode de réalisation alternatif, la durée D_{cb} de la phase de conditionnement approfondie b) est différente de la durée D_{ca} de la phase de conditionnement a).

Selon un mode de réalisation, la durée D1 prédéterminée du délai (ou « delay ») de l'étape (b-ii) de la phase de conditionnement approfondi b) va de de 0,5 à 10 secondes, de préférence de 0,7 à 9 secondes, de préférence encore de 0,9 à 8 secondes, de préférence encore de 1 à 7 secondes, de préférence encore de 1,5 à 6 secondes, de préférence encore de 2 à 5 secondes, de préférence encore de 2,5 à 4,5 secondes, de préférence encore de 3 à 4 secondes. De manière particulièrement préférée, ladite durée D1 prédéterminée est choisie parmi 3 secondes, environ 3 secondes, 3,5 secondes, environ 3,5 secondes, 4 secondes, environ 4 secondes, 4,5 secondes, environ 4,5 secondes, 5 secondes et environ 5 secondes, 6 secondes et environ 6 secondes. Avantageusement, la durée D1 prédéterminée représente la capacité de mémoire de travail du poisson modèle.

Les réponses de l'étape (b-iii) sont présentées pendant une durée déterminée D_{rb}. Ladite durée déterminée D_{rb} peut par exemple aller de 1 seconde à 10 minutes, de préférence de 5 secondes à 9 minutes, de préférence de 10 secondes à 8 minutes, de préférence de 15 secondes à 7 minutes, de préférence de 20 secondes à 6 minutes, de préférence de 25 secondes à 5 minutes, de préférence de 30 à secondes à 4 minutes, de préférence de 35 secondes à 3 minutes, de préférence de 40 secondes à 2 minutes, de préférence de 45 secondes à 60 secondes.

Selon un mode de réalisation préféré, ladite durée déterminée D_{rb} va de 1 seconde à 300 secondes, de préférence de 3 seconde à 240 secondes, de préférence de 5 secondes à 240 secondes, de préférence de 10 secondes à 180 secondes, de préférence encore de 15 secondes à 120 secondes, de préférence de 20 secondes à 100 secondes, de préférence de 25 secondes à 90 secondes, de préférence de 30 secondes à 60 secondes, de préférence encore de 5 secondes à 90 secondes, de préférence encore de 10 secondes à 80 secondes, de préférence encore de 15 secondes à 60 secondes, de préférence encore de 20 secondes à 50 secondes, de préférence encore de 25 secondes à 45 secondes, de préférence encore de 30 secondes à 35 secondes. De manière particulièrement préférée, ladite durée déterminée D_{rb} est choisie parmi 5, 6, 7, 8, 9, 10, 11, 12, 13, 14, 15, 20, 25, 30, 35, 40, 45, 50, 55, 60, 70, 80, 90, 100, 110, 120, 130, 150, 180, 200, 240 et 300 secondes. De préférence, ladite durée déterminée D_{rb} est d'environ 30 secondes ou de 30 secondes.

Pour un essai donné, lors de l'étape de détection (b-iv), la réponse choisie par le poisson modèle de l'étape (b-iii) parmi lesdites au moins deux réponses distinctes, est dite correcte lorsque que le choix d'au moins une de ladite ou desdites réponses identiques à la consigne de l'étape (b-i) par le poisson modèle est détecté à l'expiration de la durée D_{rb}. Autrement dit, la réponse est dite correcte lorsque ledit poisson modèle choisit au moins une de ladite ou desdites réponses identiques à la consigne de l'étape (b-i), pendant la durée de présentation des réponses D_{rb}.

Pour un essai donné, lors de l'étape de détection (b-iv), la réponse choisie par le poisson modèle de l'étape (b-iii) parmi lesdites au moins deux réponses distinctes, est dite incorrecte lorsque que le choix d'au moins une de ladite ou desdites réponses différentes de la consigne de l'étape (b-i) par le poisson modèle est détecté à l'expiration de la durée D_{rb}. Autrement dit, la réponse est dite incorrecte lorsque ledit poisson modèle choisit au moins une de ladite ou desdites réponses différentes de la consigne de l'étape (b-i), pendant la durée de présentation des réponses D_{rb}.

Selon un mode de réalisation, pour un essai donné, la réponse est également dite incorrecte lorsque qu'aucun choix de réponse par le poisson modèle n'est détecté à l'expiration de la durée D_{rb}, lors de l'étape de détection (b-iv). Autrement dit, selon ce mode de réalisation, la réponse est également dite incorrecte lorsque le poisson modèle n'a fait aucun choix durant ladite durée D_{rb}, lors de l'étape de détection (b-iv). Selon ce mode de réalisation, la réponse est donc dite incorrecte lorsque ledit poisson modèle choisit au moins une de ladite ou desdites réponses différentes de la consigne de l'étape (b-i), ou lorsque ledit poisson n'a fait aucun choix, pendant ladite durée de présentation des réponses D_{rb}. Selon ce mode de réalisation, ladite durée D_{rb} représente le temps imparti au poisson modèle pour choisir une réponse, lors d'un essai. Ladite durée D_{rb} est telle que définie ci-dessus.

Avantageusement, chaque essai de la phase de conditionnement approfondie b) comprend l'étape additionnelle (b-iv'), réalisée en aval de l'étape (b-iv). Ladite étape (b-iv') consiste à conclure que la réponse choisie par le poisson modèle de l'étape (b-iv), est la réponse choisie par le poisson modèle de l'étape (b-iv) (ou l'absence de réponse, selon le mode de réalisation décrit ci-dessus) à l'expiration de la durée D_{rb}.

Les étapes b-i), b-iii) et b-iv) de la phase de conditionnement approfondi b) sont conformes aux étapes a-i), a-ii) et a-iii) de la phase de conditionnement a), respectivement. Ainsi, la consigne, la réponse, l'essai, le nombre d'essais, la session, le nombre de sessions, la récompense, les différentes durées (par exemple la durée de la pause et la durée de l'interruption), l'étape de détection et la détection, et le poisson modèle de la phase de conditionnement approfondi b), sont tels que définis ci-dessus pour la phase de conditionnement a). En particulier, les essais de la phase de conditionnement approfondi b) peuvent être réalisés de manière enchaînée ou non ; les sessions de la phase de conditionnement approfondi b) peuvent être réalisés de manière consécutive, de manière enchaînée ou non ; tel que défini ci-dessus en relation avec la phase de conditionnement a). Dans le cas de la phase de conditionnement approfondi b), une seule session peut être réalisée. Ainsi, selon un mode de réalisation, la phase de conditionnement approfondi b) comprend, ou consiste en, une seule session (comprenant, ou consistant en, une répétition d'un nombre d'essais, réalisés de manière enchaînée ou non, telle que définie ci-dessus en relation avec la phase de conditionnement a)).

Selon un mode de réalisation, la consigne utilisée dans l'étape b-i) de la phase de conditionnement approfondi b) est identique à la consigne utilisée dans l'étape a-i) de la phase de conditionnement a) ; et/ou la ou les réponses utilisée(s) dans l'étape b-iii) de la phase de conditionnement approfondi b) est (sont) identique(s) à la ou les réponses utilisée(s) dans l'étape a-ii) de la phase de conditionnement a).

Selon un mode de réalisation alternatif, la consigne utilisée dans l'étape b-i) de la phase de conditionnement approfondi b) est différente de la consigne utilisée dans l'étape a-i) de la phase de conditionnement a) ; et/ou la ou les réponses utilisée(s) dans l'étape b-iii) de la phase de conditionnement approfondi b) est (sont) différente(s) de la ou les réponses utilisée(s) dans l'étape a-ii) de la phase de conditionnement a).

Par « **taux de réussite T2 prédéterminé** », on entend le taux de réussite minimum obtenu par un poisson modèle donné, par session de la phase de conditionnement approfondi b), à partir duquel le poisson modèle est conditionné de manière approfondie.

Selon un mode de réalisation, ledit taux de réussite T2 est d'au moins 70%, de préférence d'au moins 75%, de préférence encore d'au moins 80%, de préférence encore d'au moins 85%, de préférence encore d'au moins 90%, de préférence encore d'au moins 95%, de préférence encore de 100%.

Selon un mode de réalisation, le poisson modèle est conditionné de manière approfondie, lorsqu'il obtient un taux de réussite T2 par session d'au moins 70%, de préférence d'au moins 75%, de préférence encore d'au moins 80%, de préférence encore d'au moins 85%, de préférence encore d'au moins 90%, de préférence encore d'au moins 95%, de préférence encore de 100%.

Selon un mode de réalisation préféré, le poisson modèle est conditionné de manière approfondie lorsqu'il obtient un taux de réussite T2 par session d'au moins 70%, de préférence d'au moins 75%, de préférence encore d'au moins 80%, de préférence encore d'au moins 85%, de préférence encore d'au moins 90%, de préférence encore d'au moins 95%, de préférence encore de 100%, pour au moins deux sessions consécutives (enchainées ou non). Selon un mode de réalisation préféré, le poisson modèle est conditionné de manière approfondie lorsqu'il obtient un taux de réussite T2 par session d'au moins 70% pour au moins deux sessions consécutives (enchainées ou non). Selon un mode de réalisation préféré, le poisson modèle est conditionné de manière approfondie lorsqu'il obtient un taux de réussite T2 par session d'au moins 70% (de préférence encore d'au moins 75%, de préférence encore d'au moins 80%, de préférence encore d'au moins 85%, de préférence encore d'au moins 90%, de préférence encore d'au moins 95% de préférence encore d'au moins 100%) pour au moins trois sessions consécutives (enchainées ou non). Selon un mode de réalisation préféré, le poisson modèle est conditionné de manière approfondie lorsqu'il obtient un taux de réussite T2 par session d'au moins 70% pour au moins trois sessions consécutives (enchainées ou non).

Selon un mode de réalisation préféré, le poisson modèle est conditionné de manière approfondie lorsqu'il obtient un taux de réussite T2 par session d'au moins 70% pour une session, de préférence pour au moins deux sessions consécutives (enchainées ou non), de préférence pour au moins trois sessions consécutives (enchainées ou non) ; chaque session consistant en, ou comprenant, au moins une répétition de 10 essais (réalisés de manière enchaînée ou non). Selon un mode de réalisation particulièrement préféré, le poisson modèle est conditionné de manière approfondie lorsqu'il obtient un taux de réussite T2 par session d'au moins 70% pour au moins trois sessions consécutives (enchaînées ou non), chaque session consistant en une répétition de 10 essais réalisés de manière enchaînée.

La méthode de conditionnement approfondie peut être mise en oeuvre simultanément sur un groupe d'individus (appartenant au même genre de poisson modèle ou non, à la même espèce de poisson modèle ou non). Ledit groupe est défini en relation avec la phase de conditionnement approfondie b) comme indiqué précédemment en relation avec la phase de conditionnement a).

Ainsi, selon un premier mode de réalisation, le groupe de poissons modèles est conditionné de manière approfondie lorsqu'un nombre prédéterminé d'individus obtient un taux de réussite T2 prédéterminé tel que défini ci-dessus. Le groupe de poissons modèles est conditionné de manière approfondie lorsqu'au moins la moitié (50%) des individus du groupe obtient un taux de réussite T2 prédéterminé tel que défini ci-dessus. De préférence, le groupe de poissons modèles est conditionné de manière approfondie lorsqu'au moins 55% des individus du groupe obtient un taux de réussite T2 prédéterminé tel que défini ci-dessus, de préférence encore lorsqu'au moins 60% des individus du groupe, de préférence encore lorsqu'au moins 65% des individus du groupe ,de préférence encore lorsqu'au moins 70% des individus du groupe, de préférence encore lorsqu'au moins 75% des individus du groupe, de préférence encore lorsqu'au moins 80% des individus du groupe, de préférence encore lorsqu'au moins 85% des individus du groupe, de préférence encore lorsqu'au moins 90% des individus du groupe, de préférence encore lorsqu'au moins 95% des individus du groupe, de préférence encore lorsque 100% des individus du groupe obtient un taux de réussite T2 prédéterminé tel que défini ci-dessus.

Selon un mode de réalisation, l'étape (b-iv) de détection de la réponse choisie par le poisson modèle comprend la détection du passage et/ou de la présence dudit poisson modèle dans une zone de réponse (ou zone de choix). La détection de la présence du poisson modèle dans une zone de réponse (ou zone de choix), ainsi que ladite zone de réponse (ou zone de choix) sont définies en relation avec la phase de conditionnement approfondie b) comme indiqué précédemment en relation avec la phase de conditionnement a). Notamment, la détection de la présence du poisson modèle dans une zone de réponse (ou zone de choix), comprend par exemple la détection du passage (ou franchissement) dudit poisson modèle à travers une ligne de réponse (ou encore frontière, limite, ou borne), ladite ligne de réponse en relation avec la phase de conditionnement approfondie b) étant définie comme indiqué précédemment en relation avec la phase de conditionnement a). Notamment, ladite ligne de réponse (et/ou ladite zone de réponse ou zone de choix) peut consister en, ou comprendre, un orifice de réponse (ou une ouverture, un trou ou encore une embrasure, une cavité ou une niche), ledit orifice de réponse en relation avec la phase de conditionnement approfondie b) étant défini comme indiqué précédemment en relation avec la phase de conditionnement a).

Avantageusement, l'étape de détection (b-iv) de la réponse choisie par le poisson modèle est effectuée au moyen de capteurs, lesdits capteurs en relation avec la phase de conditionnement approfondie b) étant défini comme indiqué précédemment en relation avec la phase de conditionnement a). Alternativement ou en combinaison, la détection de la réponse choisie par le poisson modèle (par exemple la détection du passage dudit poisson modèle dans une zone de réponse) est visuelle, par exemple une détection directement par l'expérimentateur.

Il peut être avantageux de détecter le passage du poisson à travers une ligne qui n'est pas dédiée à la détection de la réponse choisie par le poisson (par exemple le passage du poisson dans un orifice qui n'est pas dédié à la détection de la réponse choisie par le poisson). Ladite ligne (par exemple comprenant ou consistant en un orifice) qui n'est pas dédiée à la détection de la réponse choisie par le poisson est donc dite « ligne de suivi » (ou encore ligne de traçage ou « tracking », ou encore ligne de « monitoring »). La ligne de suivi et l'orifice de suivi en relation avec la phase de conditionnement approfondie b), sont définis comme indiqué précédemment en relation avec la phase de conditionnement a).

Alternativement ou en combinaison, il peut être avantageux de détecter les mouvements et/ou la position du poisson modèle durant tout ou partie d'un essai et/ou d'une session et/ou de la phase de conditionnement approfondie b). Une telle détection permet notamment le suivi (ou encore le traçage ou « tracking », ou encore le « monitoring ») du poisson durant le déroulement de tout ou partie d'un essai et/ou d'une session et/ou de la procédure de conditionnement approfondie b). La détection suivie du poisson est par exemple effectuée au moyen de capteurs (par exemple au moyen de plusieurs capteurs positionnés dans différentes zones (zone de départ, zone de réponse), de caméra(s) de suivi de mouvement, et/ou d'un système de « tracking » ou de « monitoring ». Alternativement ou en combinaison, la détection suivie peut être visuelle, par exemple une détection directement par l'expérimentateur.

Selon un mode de réalisation, la phase de conditionnement approfondie b) comprend en outre une étape additionnelle effectuée en aval de l'étape de détection (b-v). Ladite étape additionnelle comprend de préférence :
- la distribution d'une récompense audit poisson modèle lorsqu'il a choisi au moins une de ladite ou desdites réponses identiques à ladite consigne pendant ladite durée D_{rb}, ladite récompense comprenant de préférence de la nourriture adaptée audit poisson modèle; ou
- la non distribution de récompense audit poisson modèle lorsqu'il a choisi au moins une de ladite ou desdites réponses différentes de ladite consigne pendant ladite durée D_{rb} et, optionnellement, l'isolement et/ou la mise en obscurité dudit poisson modèle, lorsqu'il a choisi au moins une de ladite ou desdites réponses différentes de ladite consigne (ou optionnellement, lorsqu'il n'a choisi aucune réponse) pendant ladite durée D_{rb}.

La présente invention concerne donc une méthode d'obtention de poissons modèles conditionnés de manière approfondie, comprenant une phase de conditionnement approfondi b) ; ladite phase de conditionnement approfondi étant caractérisée en ce qu'elle consiste en au moins une session d'au moins deux essais consécutifs ; chaque essai comprenant les étapes consistant à :
(b-i) présenter une consigne audit poisson modèle conditionné durant une durée déterminée ;
(b-ii) retirer ladite consigne puis observer un délai d'une durée D1 prédéterminée, durant laquelle aucune consigne n'est présentée au poisson modèle de l'étape (b-i) ;
(b-iii) présenter simultanément au moins deux réponses distinctes au poisson modèle de l'étape (b-ii) pendant une durée D_{rb}, lesdites au moins deux réponses distinctes comprenant au moins une réponse identique à ladite consigne et au moins une réponse différente de ladite consigne ;
(b-iv) détecter la réponse choisie par le poisson modèle de l'étape (b-iii) parmi lesdites au moins deux réponses distinctes, pendant ladite durée D_{rb} ; et
(b-v) distribuer une récompense audit poisson modèle lorsqu'il a choisi au moins une de ladite ou desdites réponses identiques à ladite consigne pendant ladite durée D_{rb}, ladite récompense comprenant de préférence de la nourriture adaptée audit poisson modèle; ou
ne pas distribuer de récompense audit poisson modèle lorsqu'il a choisi au moins une de ladite ou desdites réponses différentes de ladite consigne pendant ladite durée D_{rb} et, optionnellement, isoler et/ou mettre en obscurité ledit poisson modèle lorsqu'il a choisi au moins une de ladite ou desdites réponses différentes de ladite consigne (ou optionnellement, lorsqu'il n'a choisi aucune réponse) pendant ladite durée D_{rb}.
ledit poisson modèle étant conditionné de manière approfondie lorsqu'il choisit au moins une de ladite ou desdites réponses identiques à ladite consigne avec un taux de réussite T2 prédéterminé par session.

Ainsi, selon un mode de réalisation, la méthode d'obtention d'un poisson modèle conditionné selon l'invention comprend en outre une phase de conditionnement approfondi b) caractérisée en ce qu'elle consiste en au moins une session d'au moins deux essais consécutifs ; chaque essai comprenant les étapes consistant à :
(b-i) présenter une consigne audit poisson modèle conditionné durant une durée déterminée ;
(b-ii) retirer ladite consigne puis observer un délai d'une durée D1 prédéterminée, durant laquelle aucune consigne n'est présentée au poisson modèle de l'étape (b-i) ;
(b-iii) présenter simultanément au moins deux réponses distinctes au poisson modèle de l'étape (b-ii) pendant une durée D_{rb}, lesdites au moins deux réponses distinctes comprenant au moins une réponse identique à ladite consigne et au moins une réponse différente de ladite consigne ;
(b-iv) détecter la réponse choisie par le poisson modèle de l'étape (b-iii) parmi lesdites au moins deux réponses distinctes, pendant ladite durée D_{rb} ; et
(b-v) distribuer une récompense audit poisson modèle lorsqu'il a choisi au moins une de ladite ou desdites réponses identiques à ladite consigne pendant ladite durée D_{rb}, ladite récompense comprenant de préférence de la nourriture adaptée audit poisson modèle; ou
ne pas distribuer de récompense audit poisson modèle lorsqu'il a choisi au moins une de ladite ou desdites réponses différentes de ladite consigne pendant ladite durée D_{rb} et, optionnellement, isoler et/ou mettre en obscurité ledit poisson modèle lorsqu'il a choisi au moins une de ladite ou desdites réponses différentes de ladite consigne (ou optionnellement, lorsqu'il n'a choisi aucune réponse) pendant ladite durée D_{rb}.
ledit poisson modèle étant conditionné de manière approfondie lorsqu'il choisit au moins une de ladite ou desdites réponses identiques à ladite consigne avec un taux de réussite T2 prédéterminé par session.

La présente invention concerne donc une méthode d'obtention de poissons modèles conditionnés, comprenant une phase de conditionnement a) ; ladite phase de conditionnement étant caractérisée en ce qu'elle consiste en au moins une session d'au moins deux essais consécutifs ; chaque essai comprenant les étapes consistant à :
(a-i) présenter une consigne audit poisson modèle ;
(a-ii) présenter simultanément au moins deux réponses distinctes au poisson modèle de l'étape (a-i) pendant une durée Dᵣₐ, lesdites au moins deux réponses distinctes comprenant au moins une réponse identique à ladite consigne et au moins une réponse différente de ladite consigne ;
(a-iii) détecter la réponse choisie par le poisson modèle de l'étape (a-ii) parmi lesdites au moins deux réponses distinctes pendant ladite durée Dᵣₐ ; et, optionnellement,
(a-iv) distribuer une récompense audit poisson modèle lorsqu'il a choisi au moins une de ladite ou desdites réponses identiques à ladite consigne pendant ladite durée Dᵣₐ, ladite récompense comprenant de préférence de la nourriture adaptée audit poisson modèle; ou
ne pas distribuer de récompense audit poisson modèle lorsqu'il a choisi au moins une de ladite ou desdites réponses différentes de ladite consigne pendant ladite durée Dᵣₐ et, optionnellement, isoler et/ou mettre en obscurité ledit poisson modèle lorsqu'il a choisi au moins une de ladite ou desdites réponses différentes de ladite consigne (ou optionnellement, lorsqu'il n'a choisi aucune réponse) pendant ladite durée Dᵣₐ
   ledit poisson modèle étant conditionné lorsqu'il choisit au moins une de ladite ou desdites réponses identiques à ladite consigne avec un taux de réussite T1 prédéterminé par session ;
   ladite phase de conditionnement a) étant suivie d'une phase de conditionnement approfondi b) caractérisée en ce qu'elle consiste en au moins une session d'au moins deux essais consécutifs ; chaque essai comprenant les étapes consistant à :
(b-i) présenter une consigne audit poisson modèle conditionné durant une durée déterminée ;
(b-ii) retirer ladite consigne puis observer un délai d'une durée D1 prédéterminée, durant laquelle aucune consigne n'est présentée au poisson modèle de l'étape (b-i) ;
(b-iii) présenter simultanément au moins deux réponses distinctes au poisson modèle de l'étape (b-ii) pendant une durée D_{rb}, lesdites au moins deux réponses distinctes comprenant au moins une réponse identique à ladite consigne et au moins une réponse différente de ladite consigne ; et
(b-iv) détecter la réponse choisie par le poisson modèle de l'étape (b-iii) parmi lesdites au moins deux réponses distinctes pendant ladite durée D_{rb} ; et optionnellement,
(b-v) distribuer une récompense audit poisson modèle lorsqu'il a choisi au moins une de ladite ou desdites réponses identiques à ladite consigne pendant ladite durée D_{rb}, ladite récompense comprenant de préférence de la nourriture adaptée audit poisson modèle; ou
ne pas distribuer de récompense audit poisson modèle lorsqu'il a choisi au moins une de ladite ou desdites réponses différentes de ladite consigne pendant ladite durée D_{rb} et, optionnellement, isoler et/ou mettre en obscurité ledit poisson modèle lorsqu'il a choisi au moins une de ladite ou desdites réponses différentes de ladite consigne (ou optionnellement, lorsqu'il n'a choisi aucune réponse) pendant ladite durée D_{rb}.
ledit poisson modèle étant conditionné de manière approfondie lorsqu'il choisit au moins une de ladite ou desdites réponses identiques à ladite consigne avec un taux de réussite T2 prédéterminé par session.

Avantageusement, ladite récompense de la phase de conditionnement approfondie b) est telle que la récompense définie en relation avec la phase de conditionnement a). Par exemple, la récompense de la phase de conditionnement approfondie b) est distribuée par un distributeur de récompense. Par exemple, la distribution de ladite récompense est déclenchée par le passage et/ou la détection de la présence dudit poisson modèle dans un zone de réponse, par exemple à travers une ligne de réponse (comprenant ou consistant en un orifice de réponse). Avantageusement, ladite zone de réponse, ladite ligne de réponse et/ou ledit orifice de réponse est tel(le) que défini(e) ci-dessus. Notamment, ladite ligne de réponse et/ou ledit orifice de réponse est dédié(e) à la détection de la réponse choisie par le poisson. Notamment, ledit orifice de réponse peut être un orifice d'un distributeur de récompense ou encore un orifice dans une paroi, tel qu'une fenêtre, une ouverture, un trou ou encore une embrasure, une cavité ou une niche.

Avantageusement, ladite récompense n'est distribuée au poisson modèle que lorsque celui-ci a choisi au moins une réponse correcte pendant ladite durée D_{rb}. Dans ce cas, le poisson modèle ne reçoit pas de récompense lorsque celui-ci a choisi au moins une réponse incorrecte pendant ladite durée D_{rb}. La réponse correcte et la réponse incorrecte en relation avec la phase de conditionnement approfondie b) sont telles que définies ci-dessus en relation avec la phase de conditionnement a). Notamment, selon un mode de réalisation, pour un essai donné, la réponse est également dite incorrecte lorsque qu'aucun choix de réponse par le poisson modèle n'est détecté à l'expiration de la durée D_{rb}, lors de l'étape de détection (b-iv). Autrement dit, selon ce mode de réalisation, la réponse est également dite incorrecte lorsque le poisson modèle n'a fait aucun choix durant ladite durée D_{rb}, lors de l'étape de détection (b-iv). Selon ce mode de réalisation, la réponse est donc dite incorrecte lorsque ledit poisson modèle choisit au moins une de ladite ou desdites réponses différentes de la consigne de l'étape (b-i), ou lorsque ledit poisson n'a fait aucun choix, pendant ladite durée de présentation des réponses D_{rb}. Selon ce mode de réalisation, ladite durée D_{rb} représente le temps imparti au poisson modèle pour choisir une réponse, lors d'un essai. Ladite durée D_{rb} est telle que définie ci-dessus.

Selon un mode de réalisation, le poisson modèle est isolé lorsque celui-ci a choisi au moins une réponse incorrecte pendant ladite durée D_{rb} (et/ou le poisson n'a fait aucun choix pendant ladite durée D_{rb}, selon le mode de réalisation), durant un essai de la phase de conditionnement approfondie b). Selon cette étape d'isolement, ledit poisson modèle peut par exemple être isolé (ou encore enfermé, ou encore confiné) dans une zone définie, pour une durée déterminée Dᵢ. Avantageusement, ladite durée déterminée Dᵢ durant laquelle le poisson est isolé va de 5 secondes à 60 secondes, de préférence de 10 secondes à 50 secondes, de préférence encore de 20 secondes à 40 secondes, de préférence encore de 25 secondes à 35 secondes, de préférence encore, ladite durée est choisie parmi 10 secondes, 15 secondes, 20 secondes, 25 secondes, 30 secondes, 35 secondes, 40 secondes et 45 secondes, et de manière particulièrement préférée d'environ 30 secondes ou de 30 secondes. Le poisson modèle est par exemple isolé dans la zone de départ telle que définie ci-dessus. Alternativement ou en combinaison, le poisson modèle est placé dans l'obscurité lorsque celui-ci a choisi au moins une réponse incorrecte pendant ladite durée D_{rb} (et/ou lorsque le poisson n'a fait aucun choix pendant ladite durée D_{rb}, selon le mode de réalisation), pour ladite durée déterminée Dᵢ.

Selon un mode de réalisation, ladite récompense comprend ou consiste en de la nourriture adaptée audit poisson modèle, telle que les granulés, pastilles ou flocons de nourriture (ou d'un aliment) séchée et compactée disponibles dans le commerce (ou encore « pellet », par exemple de type Gemma^{®}, commercialisé par SKRETTING, tel que le Gemma Micro^{®} (notamment le Gemma Micro^{®} 600). Avantageusement, la récompense comprend, ou consiste en, de la nourriture (ou un aliment) nutritionnellement pauvre. Avantageusement encore, la récompense comprend, ou consiste en, de la nourriture appétente ou en un aliment appétent. Avantageusement encore, la récompense comprend, ou consiste en, de la nourriture (ou un aliment) appétente et nutritionnellement pauvre. Avantageusement, la récompense comprend ou consiste en de la larve d'insecte lyophilisée, telle que de la larve lyophilisée de moustique, de mouche ou de moucheron, ou un mélange de larve lyophilisée de moustique et/ou de mouche et/ou de moucheron (par exemple les larves d'insecte lyophilisées commercialisées par la société JBL, notamment la gamme NovoFil^{®}), ou des granulés, pastilles ou flocons de nourriture séchée et compactée dont la teneur en matière grasse, et/ou protéique, et/ou glucidique est pauvre ou appauvrie (par exemple une pastille qui apporte moins de 20%, de préférence moins de 15%, de préférence encore moins de 10%, de préférence encore moins de 5%, de préférence encore moins de 3%, de la quantité journalière moyenne en matière grasse, et/ou protéique, et/ou glucidique, nécessaire à un poisson modèle adulte). Les Inventeurs ont notamment montré que le taux de réussite T2 était particulièrement optimal lorsque la récompense comprend de la larve d'insecte lyophilisée. De préférence, la récompense comprend ou consiste en de la larve lyophilisée de moustique.

Selon un mode de réalisation, la méthode de l'invention comprend en outre une phase de pré-conditionnement (ou de préapprentissage, ou de pré-éducation, ou de pré-entrainement, ou encore de « pré-training ») pré-a), effectuée en amont de la phase de conditionnement a). Avantageusement, ladite phase de pré-conditionnement comprend une étape d'adaptation du poisson modèle à l'environnement dans lequel la phase de conditionnement a) et/ou la phase de conditionnement approfondie b) se dérouleront. L'homme du métier sait déterminer les étapes comprises dans ladite phase de pré-conditionnement. Avantageusement, ladite phase de pré-conditionnement pré-a) en relation avec la phase de conditionnement approfondie b) est telle que définie ci-dessus en relation avec la phase de conditionnement a).

Les étapes, la consigne, la réponse, l'essai, le nombre d'essais, la session, le nombre de sessions, la récompense, les différentes durées (par exemple la durée de la pause et la durée de l'interruption), l'étape de détection et la détection, et le poisson modèle, le taux de réussite T3 prédéterminé, de la phase de pré-conditionnement pré-a), en relation avec la phase de conditionnement approfondie b), sont définis comme indiqué précédemment en relation avec la phase de conditionnement a).

La présente invention concerne également un poisson modèle conditionné de manière approfondie, susceptible d'être obtenu par la méthode selon l'invention telle que décrite ci-dessus.

La présente invention concerne également un poisson modèle conditionné de manière approfondie obtenu par la méthode selon l'invention telle que décrite ci-dessus. La présente invention concerne également un poisson modèle conditionné de manière approfondie directement obtenu par la méthode selon l'invention telle que décrite ci-dessus.

### Dispositif

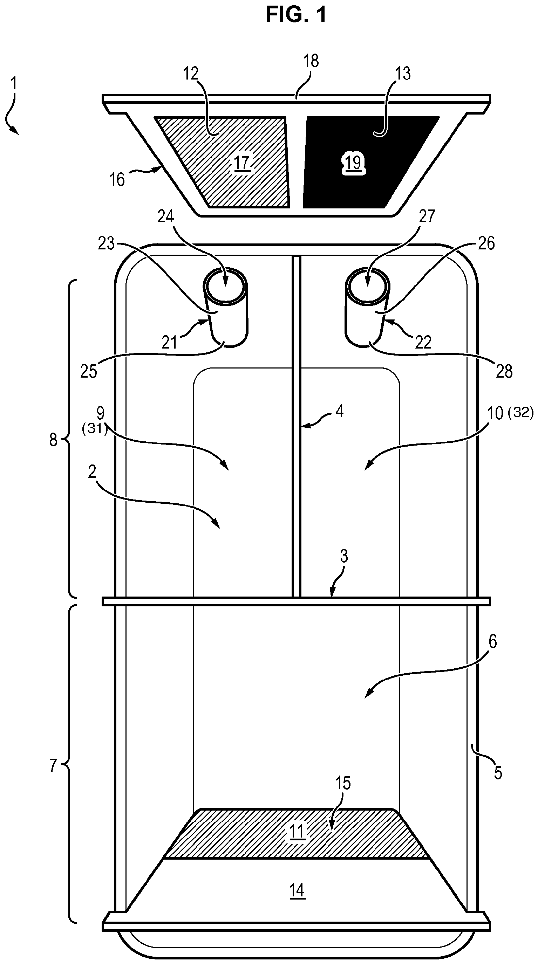

Le dispositif 1 illustré sur la figure 1 comprend un bac 2, une première cloison 3 et une deuxième cloison 4.

Le bac 2 comprend une paroi latérale 5 délimitant une cavité 6 propre à contenir de l'eau et dans laquelle peut être disposé le poisson. La cavité 6 présente une forme sensiblement rectangulaire. La paroi latérale 5 peut être formée en un matériau transparent, c'est-à-dire un matériau qui laisse passer les rayons lumineux dans une gamme de longueurs d'ondes dans le vide comprises entre 380 nanomètres (nm) et 780 nm.

La cavité 6 comprend une zone de départ 7 et une zone de choix 8 (ou zone de réponse 8). La zone de départ 7 et la zone de choix 8 sont séparées par la première cloison 3. La première cloison 3 s'étend parallèlement à un plan transversal de la cavité 6. La deuxième cloison 4 est disposée dans la zone de choix 8. La deuxième cloison 4 sépare la zone de choix 8 en un premier compartiment 9 et un deuxième compartiment 10. La deuxième cloison 4 s'étend perpendiculairement à la première cloison 3. La deuxième cloison 4 s'étend parallèlement à un plan longitudinal de la cavité 6.

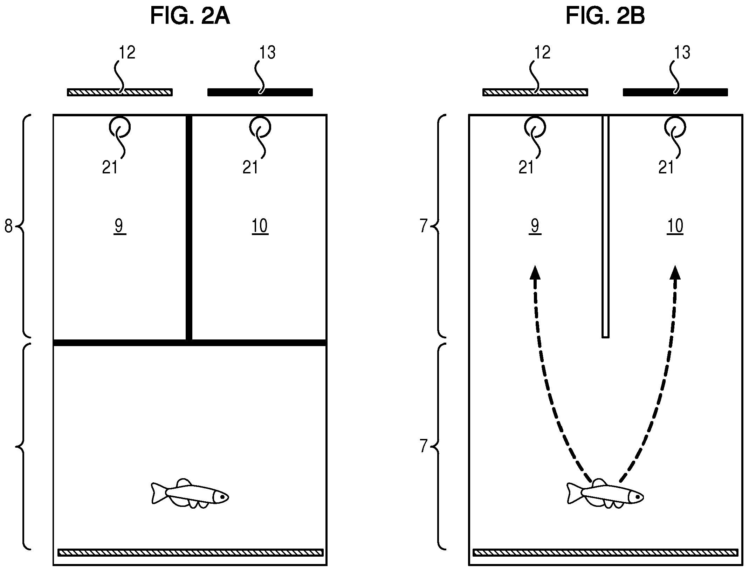

Dans le mode de réalisation illustré sur la figure 1, la première cloison 3 est montée mobile par rapport au bac 2, entre une position fermée (illustrée schématiquement sur la figure 2A) dans laquelle la première cloison 3 empêche une circulation du poisson entre la zone de départ 7 et la zone de choix 8, et une position ouverte (illustrée schématiquement sur la figure 2B) dans laquelle le poisson peut circuler entre la zone de départ 7 et la zone de choix 8. La première cloison 3 peut être fabriquée en un matériau opaque, c'est-à-dire un matériau qui ne laisse pas passer les rayons lumineux dans une gamme de longueurs d'ondes dans le vide comprises entre 380 nanomètres (nm) et 780 nm. De cette manière, lorsque la première cloison 3 est en position fermée, le poisson situé dans la zone de départ 7 ne voit pas la zone de choix 8.

La deuxième cloison 4 peut être montée fixe ou mobile par rapport au bac 2. La deuxième cloison 4 peut également être fabriquée en un matériau opaque, c'est-à-dire un matériau qui ne laisse pas passer les rayons lumineux dans une gamme de longueurs d'ondes dans le vide comprises entre 380 nanomètres (nm) et 780 nm. De cette manière, lorsque le poisson est situé dans l'un des compartiments 9 et 10, le poisson ne voit pas l'autre des compartiments.

Le dispositif 1 comprend en outre un premier élément de présentation 11 pour présenter une consigne dans la zone de départ 7, un deuxième élément de présentation 12 pour présenter une première réponse dans le premier compartiment 9 et un troisième élément de présentation 13 pour présenter une deuxième réponse dans le deuxième compartiment 10.

Dans l'exemple illustré sur la figure 1, la consigne comprend une couleur. Le premier élément de présentation 11 comprend une première plaque de support 14 et une première couche colorée 15 s'étendant sur l'une des faces de la plaque de support 14. Le premier élément de présentation 11 peut être disposé dans la cavité 6 du bac 2, dans la zone de départ 7. Alternativement, le premier élément de présentation 11 peut être disposé à l'extérieur du bac 2, contre la paroi latérale 5, de sorte que la première couche colorée 15 soit visible par le poisson lorsque le poisson se trouve dans la zone de départ 7.

La première réponse comprend une première couleur et la deuxième réponse comprend une deuxième couleur, différente de la première couleur. L'une de la première couleur et de la deuxième couleur est identique à la couleur de la consigne et l'autre de la première couleur et de la deuxième couleur est différente de la couleur de la consigne. Le deuxième élément de présentation 12 comprend une deuxième plaque de support 16 et une deuxième couche colorée 17 de la première couleur s'étendant sur l'une des faces de la deuxième plaque de support 16. De même, le troisième élément de présentation 13 comprend une troisième plaque de support 18 et une troisième couche colorée 19 de la deuxième couleur s'étendant sur l'une des faces de la troisième plaque de support 18. Le deuxième élément de présentation 12 et le troisième élément de présentation 13 peuvent être disposés à l'intérieur du bac 2, respectivement dans le premier compartiment 9 et dans le deuxième compartiment 10 de la zone de choix 8. Alternativement, le deuxième élément de présentation 12 peut être disposé à l'extérieur du bac 2, contre la paroi latérale 5, de sorte que la deuxième couche colorée 17 soit visible par le poisson lorsque le poisson se trouve dans la zone de départ 7, avec la première cloison 3 en position ouverte, et lorsque le poisson se trouve dans le premier compartiment 9. De même, le troisième élément de présentation 13 peut être disposé à l'extérieur du bac 2, contre la paroi latérale 5, de sorte que la troisième couche colorée 19 soit visible par le poisson lorsque le poisson se trouve dans la zone de départ 7, avec la première cloison 3 en position ouverte, et dans le deuxième compartiment 10.

Les éléments de présentation 11, 12 et 13 sont des panneaux d'affichage permettant d'afficher la consigne, la première réponse et la deuxième réponse de manière visible pour le poisson. La consigne, la première réponse et la deuxième réponse peuvent être affichées manuellement, sur des écrans ou projetées (une couleur, une forme, une image, un objet, un signal lumineux ou une combinaison quelconque de l'un de ceux-ci). Le dispositif 1 comprend en outre un premier distributeur de récompense 21 disposé dans le premier compartiment 9 de la zone de choix 8, et un deuxième distributeur de récompense 22 disposé dans le deuxième compartiment 10 de la zone de choix 8.

Le premier distributeur de récompense 21 comprend un premier tube 23. Le premier tube 23 présente une première extrémité ayant une première ouverture 24 et une deuxième extrémité ayant une deuxième ouverture 25. Le premier tube 23 est disposé par rapport au bac 2 de sorte que la deuxième extrémité affleure la surface de l'eau contenue dans le bac 2 dans le premier compartiment 9.

Le deuxième distributeur de récompense 22 est identique au premier distributeur de récompense 21. Le deuxième distributeur de récompense 22 comprend un deuxième tube 26. Le deuxième tube 26 présente une première extrémité ayant une première ouverture 27 et une deuxième extrémité ayant une deuxième ouverture 28. Le deuxième tube 26 est disposé par rapport au bac 2 de sorte que la deuxième extrémité affleure la surface de l'eau contenue dans le bac 2 dans le deuxième compartiment 10.

De préférence, le premier tube 21 et le deuxième tube 22 sont agencés à proximité respectivement du deuxième élément de présentation 12 et du troisième élément de présentation 13. Le premier tube 23 et le deuxième tube 26 sont agencés de sorte qu'une portion de la paroi latérale 5 du bac 2 s'étend entre le premier tube 23 et le deuxième élément de présentation 12, et une autre portion de la paroi latérale 5 du bac 2 s'étend entre le deuxième tube 26 et le troisième élément de présentation 13. De cette manière, le poisson passe à proximité de la deuxième ouverture du premier tube 23 lorsqu'il se dirige vers le deuxième élément de présentation 12. De même, le poisson passe à proximité de la deuxième ouverture du deuxième tube 26 lorsqu'il se dirige vers le troisième élément de présentation 13.

En utilisation, le poisson est initialement positionné dans la zone de départ 7, la première cloison 3 étant en position fermée. La consigne (i.e. la couleur de consigne) est présentée dans la zone de départ 7 grâce au premier élément de présentation 12. Puis, au bout d'un temps prédéterminé, la première cloison 3 est déplacée de la position fermée vers la position ouverte, de sorte que le poisson puisse circuler vers la zone de choix 8. La première réponse (i.e. la première couleur de réponse) est présentée dans le premier compartiment 9 grâce au deuxième élément de présentation 12. La deuxième réponse (i.e. la deuxième couleur de réponse) est présentée dans le deuxième compartiment 10 grâce au troisième élément de présentation 13. Une fois, la première cloison 3 en position ouverte, le poisson peut pénétrer au choix dans le premier compartiment 9 ou dans le deuxième compartiment 10. Si le poisson pénètre dans le premier compartiment 9, il est considéré comme ayant choisi la première réponse. Si le poisson pénètre dans le deuxième compartiment 10, il est considéré comme ayant choisi la deuxième réponse. Alternativement, un autre mode de détection peut être utilisé : la réponse comportementale est considérée comme correcte lorsque le poisson pénètre dans une zone de réponse, cette zone de réponse pouvant adopter différentes formes et tailles, par exemple un orifice.

Le dispositif 1 peut comprendre un ou plusieurs capteurs 31, 32 propre à détecter la réponse choisie par le poisson. Par exemple, le dispositif 1 peut comprendre un premier capteur 31 disposé dans le premier compartiment 9 et propre à détecter la présence du poisson dans le premier compartiment 9, et un deuxième capteur 32 disposé dans le deuxième compartiment 10 et propre à détecter la présence du poisson dans le deuxième compartiment 10. Les capteurs 31, 32 peuvent par exemple comprendre des capteurs de mouvement, tels que des capteurs propres à détecter un rayonnement infrarouge, ou une caméra.

Lorsque le poisson a choisi la bonne réponse (i.e. la réponse qui est identique à la consigne), une récompense peut être distribuée. La récompense est distribuée via le distributeur de récompense 21 ou 22 situé dans le compartiment 9 ou 10 dans lequel se trouve le poisson. A cet effet, de la nourriture est introduite dans le tube 21 ou 26 via la première ouverture 24 ou 27. La nourriture descend dans le tube 21 ou 26 jusqu'à la surface de l'eau. Le poisson peut passer sa tête dans la deuxième ouverture 25 ou 28 afin d'attraper la nourriture.

Le même test peut être renouvelé en modifiant les éléments de présentation.

Dans l'exemple dans lequel viennent d'être décrits les éléments de présentation 11, 12 et 13, d'autres éléments de présentation peuvent être utilisés, tel que des écrans d'affichage sur lesquels la consigne et les réponses sont projetées. La consigne peut consister en une couleur, une forme, une image, un objet, un signal lumineux ou une combinaison de ces éléments.

### Méthodes d'application

Les poissons modèles conditionnés ou conditionnés de manière approfondie sont particulièrement adaptés à la réalisation d'études de caractérisation d'une atteinte ou d'une déficience cognitive, résultant par exemple d'une maladie neuropsychiatrique ou neurologique (telle qu'une maladie neurodégénérative, une addiction ou une lésion au cerveau) ; incluant notamment l'hyperactivité, l'autisme, la schizophrénie, la maladie de Parkinson, la maladie d'Alzheimer, et une lésion au cerveau ; s'agissant notamment des effets sur la mémoire de travail et/ou les capacités cognitives et/ou les capacités d'apprentissage chez un poisson modèle ; ou d'études de la capacité de régénération du cerveau chez un poisson modèle ; ou encore d'études d'identification de molécules, de composés, de compositions ou de formulations modifiant la mémoire de travail et/ou les capacités cognitives et/ou les capacités d'apprentissage chez un poisson modèle .

Ainsi, la présente invention concerne une méthode de caractérisation d'une atteinte ou d'une déficience cognitive, résultant par exemple d'une maladie neuropsychiatrique ou neurologique (telle qu'une maladie neurodégénérative, une addiction ou une lésion au cerveau), chez un poisson modèle, ou encore une méthode d'étude de l'effet d'une atteinte ou d'une déficience cognitive, résultant par exemple d'une maladie neuropsychiatrique ou neurologique (telle qu'une maladie neurodégénérative, une addiction ou une lésion au cerveau), sur la mémoire de travail et/ou les capacités cognitives et/ou les capacités d'apprentissage chez un poisson modèle, comprenant les étapes consistant en :
a) l'obtention de poissons modèles conditionnés, ou conditionnés de manière approfondie, par la méthode de l'invention telle que décrite ci-dessus, lesdits poissons modèles conditionnés, ou conditionnés de manière approfondie, étant des poissons modèles de ladite atteinte ou ladite déficience cognitive (par exemple des poissons modèles de ladite maladie neuropsychiatrique ou neurologique, tels que des poissons modèles de ladite maladie neurodégénérative, de ladite addiction ou de ladite lésion au cerveau);
b) la comparaison du temps moyen nécessaire pour obtenir les poissons modèles conditionnés, ou conditionnés de manière approfondie, de l'étape a) avec un temps moyen de référence et/ou la comparaison du taux de réussite moyen obtenu par les poissons modèles conditionnés de l'étape a) avec un taux de réussite moyen de référence.

Le temps moyen nécessaire pour obtenir les poissons modèles conditionnés, ou conditionnés de manière approfondie, peut par exemple consister en la somme des temps (en jours, semaines ou mois) nécessaires pour que chaque individu (i.e. pour chaque poisson modèle de l'étape a)) au sein d'un groupe d'individus conditionnés simultanément/parallèlement ou séparément/indépendamment, ou conditionnés de manière approfondie simultanément/parallèlement ou séparément/indépendamment, atteigne le taux de réussite T1 ou T2 prédéterminé tel que défini ci-dessus, divisée par le nombre d'individus considérés. Alternativement, le temps moyen nécessaire pour obtenir les poissons modèles conditionnés peut par exemple consister en la somme des temps (en jours, semaines ou mois) nécessaires pour qu'un groupe d'individus conditionnés simultanément/parallèlement ou séparément/indépendamment, ou conditionnés de manière approfondie simultanément/parallèlement ou séparément/indépendamment, atteigne le taux de réussite T1 ou T2 moyen prédéterminé tel que défini ci-dessus, divisée par le nombre de groupe d'individus considérés. Le temps moyen de référence peut par exemple consister en un temps moyen nécessaire pour obtenir un poisson modèle de référence, ou un groupe de poissons modèles de référence, conditionné, ou conditionné de manière approfondie, selon la méthode de l'invention.

Le taux de réussite moyen est tel que défini ci-dessus en relation avec la méthode d'obtention de poissons modèles conditionnés, ou conditionnés de manière approfondie, de l'invention. Le taux de réussite moyen de référence peut par exemple consister en un taux de réussite moyen obtenu par un poisson modèle de référence, ou un groupe de poissons modèles de référence, soumis à la méthode d'obtention de poissons modèles conditionnés, ou conditionnés de manière approfondie, selon l'invention.

Avantageusement, le poisson modèle de référence appartient au même genre et/ou à la même espèce que le poisson modèle de l'étape a). Ledit poisson modèle de référence est par exemple un poisson modèle sain. Ledit poisson modèle de référence est par exemple un poisson modèle qui n'est pas un poisson modèle de ladite atteinte ou déficience cognitive, résultant par exemple d'une maladie neuropsychiatrique ou neurologique (telle qu'une maladie neurodégénérative, une addiction ou une lésion au cerveau). Dans ce cas, ladite atteinte ou ladite déficience cognitive, résultant par exemple d'une maladie neuropsychiatrique ou neurologique (telle qu'une maladie neurodégénérative, une addiction ou une lésion au cerveau) affecte ou inhibe la mémoire de travail et/ou les capacités cognitives et/ou les capacités d'apprentissage chez un poisson modèle si, suite à la comparaison de l'étape b), le temps moyen nécessaire pour obtenir les poissons modèles conditionnés, ou conditionnés de manière approfondie, de l'étape a) est supérieur au temps moyen de référence et/ou le taux de réussite moyen obtenu par les poissons modèles conditionnés, ou conditionnés de manière approfondie, de l'étape a) est inférieur au taux de réussite moyen de référence. A l'inverse, ladite atteinte ou ladite déficience cognitive, résultant par exemple d'une maladie neuropsychiatrique ou neurologique (telle qu'une maladie neurodégénérative, une addiction ou une lésion au cerveau) n'affecte pas ou n'inhibe pas la mémoire de travail et/ou les capacités cognitives et/ou les capacités d'apprentissage chez un poisson modèle si, suite à la comparaison de l'étape b), le temps moyen nécessaire pour obtenir les poissons modèles conditionnés, ou conditionnés de manière approfondie, de l'étape a) est environ égal au temps moyen de référence et/ou le taux de réussite moyen obtenu par les poissons modèles conditionnés, ou conditionnés de manière approfondie, de l'étape a) est environ égal au taux de réussite moyen de référence.

Selon un mode de réalisation, le poisson modèle de l'étape a) est choisi parmi un poisson modèle de l'hyperactivité, de l'autisme, de la schizophrénie, de la maladie de Parkinson, de la maladie d'Alzheimer, et d'une lésion au cerveau.

La présente invention concerne également, une méthode d'étude de la capacité de régénération du cerveau chez un poisson modèle, comprenant les étapes consistant en :
a) l'obtention de poissons modèles conditionnés, ou conditionnés de manière approfondie, par la méthode de l'invention telle que décrite ci-dessus, lesdits poissons modèles conditionnés, ou conditionnés de manière approfondie, étant des poissons modèles d'une atteinte ou d'une déficience cognitive, résultant par exemple d'une maladie neuropsychiatrique ou neurologique (telle qu'une maladie neurodégénérative, une addiction ou une lésion au cerveau);
b) la mise en oeuvre de la méthode d'obtention de poissons modèles conditionnés, ou conditionnés de manière approfondie, telle que décrite ci-dessus sur les poissons modèles conditionnés, ou conditionnés de manière approfondie, de l'étape a), après un intervalle d'une durée minimale déterminée, allant de préférence de 0, 5 à 6 mois, de préférence de 1 à 5 mois, de préférence encore de 2 à 4 mois, de préférence encore de 3 mois ;
c) la comparaison du taux de réussite moyen obtenus par les poissons modèles conditionnés, ou conditionnés de manière approfondie, de l'étape a) avec le taux de réussite moyen obtenu par les poissons modèles conditionnés, ou conditionnés de manière approfondie, de l'étape b).

Selon un mode de réalisation, ledit poisson modèle présente une capacité de régénération du cerveau si, suite à la comparaison de l'étape c), le taux de réussite moyen obtenu par les poissons modèles conditionnés, ou conditionnés de manière approfondie, de l'étape b) est supérieur au taux de réussite moyen obtenu par les poissons modèles conditionnés, ou conditionnés de manière approfondie, de l'étape a). A l'inverse, ledit poisson modèle ne présente pas de capacité de régénération du cerveau si, suite à la comparaison de l'étape c), le taux de réussite moyen obtenu par les poissons modèles conditionnés, ou conditionnés de manière approfondie, de l'étape b) est inférieur ou égal au taux de réussite moyen obtenu par les poissons modèles conditionnés, ou conditionnés de manière approfondie, de l'étape a).

L'invention concerne également une méthode de caractérisation de la mémoire de travail et/ou des capacités cognitives et/ou les capacités d'apprentissage chez un poisson modèle, comprenant les étapes consistant en :
a) l'obtention de poissons modèles conditionnés, ou conditionnés de manière approfondie, par la méthode de l'invention telle que décrite ci-dessus;
b) la détermination du temps moyen nécessaire pour obtenir les poissons modèles conditionnés, ou conditionnés de manière approfondie, de l'étape a) et/ou la détermination du taux de réussite moyen obtenu par les poissons modèles conditionnés, ou conditionnés de manière approfondie, de l'étape a) et/ou la détermination de la durée D1 maximale applicable aux poissons modèles conditionnés de manière approfondie de l'étape a) ; et optionnellement
c) la comparaison du temps moyen de l'étape b) avec un temps moyen de référence et/ou la comparaison du taux de réussite moyen de l'étape b) avec un taux de réussite moyen de référence et/ou la comparaison de la durée D1 maximale de l'étape b) à une durée D1 maximale de référence.

Le temps moyen nécessaire pour obtenir les poissons modèles conditionnés, ou conditionnés de manière approfondie, peut par exemple consister en la somme des temps (en jours, semaines ou mois) nécessaires pour que chaque individu (i.e. pour chaque poisson modèle de l'étape a)) au sein d'un groupe d'individus conditionnés simultanément/parallèlement ou séparément/indépendamment, ou conditionnés de manière approfondie simultanément/parallèlement ou séparément/indépendamment, atteigne le taux de réussite T1 ou T2 prédéterminé tel que défini ci-dessus, divisée par le nombre d'individus considérés. Alternativement, le temps moyen nécessaire pour obtenir les poissons modèles conditionnés, ou conditionnés de manière approfondie, peut par exemple consister en la somme des temps (en jours, semaines ou mois) nécessaires pour qu'un groupe d'individus conditionnés simultanément/parallèlement ou séparément/indépendamment, ou conditionnés de manière approfondie simultanément/parallèlement ou séparément/indépendamment, atteigne le taux de réussite T1 ou T2 moyen prédéterminé tel que défini ci-dessus, divisée par le nombre de groupe d'individus considérés. Le temps moyen de référence peut par exemple consister en un temps moyen nécessaire pour obtenir un poisson modèle de référence, ou un groupe de poissons modèles de référence, conditionné, ou conditionné de manière approfondie, selon la méthode de l'invention.

Le taux de réussite moyen est tel que défini ci-dessus en relation avec la méthode d'obtention de poissons modèles conditionnés, ou conditionnés de manière approfondie, de l'invention. Le taux de réussite moyen de référence peut par exemple consister en un taux de réussite moyen obtenu par un poisson modèle de référence, ou un groupe de poissons modèles de référence, soumis à la méthode d'obtention de poissons modèles conditionnés, ou conditionnés de manière approfondie, selon l'invention.

La durée D1 maximale applicable aux poissons modèles conditionnés de l'étape a) est la durée D1 maximale pour laquelle le poisson modèle conditionné de l'étape a), soumis à la méthode d'obtention de poisson modèle conditionné de manière approfondie selon l'invention, obtient un taux de réussite T2 prédéterminé tel que défini ci-dessus. Avantageusement, la durée D1 maximale (ou limite) représente la capacité de mémoire de travail maximale (ou limite) du poisson modèle. Ainsi, il est possible d'augmenter progressivement la durée D1 au cours de différents essais consécutifs, par exemple afin de déterminer la durée D1 maximale (ou durée D1 limite) du poisson modèle considéré dans les conditions considérées. La durée D1 maximale de référence peut par exemple consister en une durée D1 maximale obtenue par un poisson modèle de référence, ou un groupe de poissons modèles de référence, soumis à la méthode d'obtention de poisson modèle conditionné de manière approfondie selon l'invention.

Le poisson modèle de référence est par exemple un poisson modèle sain. Avantageusement, le poisson modèle de référence appartient au même genre et/ou à la même espèce que le poisson modèle de l'étape a). Selon un mode de réalisation, le poisson modèle de l'étape a) est un poisson modèle sain. Selon un mode de réalisation alternatif, le poisson modèle de l'étape a) est choisi parmi des poissons modèles d'une atteinte ou d'une déficience cognitive, résultant par exemple d'une maladie neuropsychiatrique ou neurologique (telle qu'une maladie neurodégénérative, une addiction ou une lésion au cerveau), notamment choisi parmi des poissons modèles de l'hyperactivité, de l'autisme, de la schizophrénie, de la maladie de Parkinson, de la maladie d'Alzheimer, et d'une lésion au cerveau. Selon ce mode de réalisation alternatif, le poisson modèle de référence est de préférence un poisson modèle sain.

Selon un mode de réalisation, le poisson modèle de l'étape a) est un poisson modèle conditionné de manière approfondie. Selon ce mode de réalisation, le poisson modèle de référence peut être un poisson modèle conditionné selon la méthode de l'invention, qui n'a pas été conditionné de manière approfondie. Avantageusement, le poisson modèle de référence appartient au même genre et/ou à la même espèce que le poisson modèle de l'étape a). Avantageusement, le poisson modèle de référence est un poisson modèle sain si le poisson modèle de l'étape a) est un poisson modèle sain. Alternativement, si le poisson modèle de l'étape a) est choisi parmi des poissons modèles d'une atteinte ou d'une déficience cognitive, résultant par exemple d'une maladie neuropsychiatrique ou neurologique (telle qu'une maladie neurodégénérative, une addiction ou une lésion au cerveau), notamment choisi parmi des poissons modèles de l'hyperactivité, de l'autisme, de la schizophrénie, de la maladie de Parkinson, de la maladie d'Alzheimer, et d'une lésion au cerveau, alors le poisson modèle de référence est un poisson modèle de ladite atteinte ou de ladite déficience cognitive.

L'invention concerne également une méthode d'identification de molécules, de composés, de compositions ou de formulations modifiant la mémoire de travail et/ou les capacités cognitives et/ou les capacités d'apprentissage chez un poisson modèle, ou encore une méthode de criblage de molécules, de composés, de compositions ou de formulations modifiant la mémoire de travail et/ou les capacités cognitives et/ou les capacités d'apprentissage chez un poisson modèle.

Dans un mode de réalisation, ladite méthode d'identification de molécules, de composés, de compositions ou de formulations modifiant la mémoire de travail et/ou les capacités cognitives et/ou les capacités d'apprentissage chez un poisson modèle, comprend les étapes consistant en :
a) la mise en contact de poissons modèles avec ladite molécule, ledit composé, ladite composition ou ladite formulation ;
b) la mise en oeuvre de la méthode d'obtention de poissons modèles conditionnés, ou conditionnés de manière approfondie, telle que décrite ci-dessus, sur le poisson modèle de l'étape a) ;
c) la comparaison du temps moyen nécessaire pour obtenir les poissons modèles conditionnés, ou conditionnés de manière approfondie, de l'étape a) avec un temps moyen de référence et/ou la comparaison du taux de réussite moyen obtenu par les poissons modèles conditionnés, ou conditionnés de manière approfondie, de l'étape a) avec un taux de réussite moyen de référence.

Selon ce mode de réalisation, ladite molécule, ledit composé, ladite composition ou ladite formulation restaure et/ou améliore et/ou affecte positivement, et/ou modifie positivement la mémoire de travail et/ou les capacités cognitives et/ou les capacités d'apprentissage dudit poisson modèle si, suite à la comparaison de l'étape c), le temps moyen nécessaire pour obtenir les poissons modèles conditionnés, ou conditionnés de manière approfondie, de l'étape b) est inférieur au temps moyen de référence et/ou le taux de réussite moyen obtenu par les poissons modèles conditionnés, ou conditionnés de manière approfondie, de l'étape b) est supérieur au taux de réussite moyen de référence. A l'inverse, ladite molécule, ledit composé, ladite composition ou ladite formulation perturbe et/ou inhibe et/ou affecte négativement et/ou altère et/ou modifie négativement la mémoire de travail et/ou les capacités cognitives et/ou les capacités d'apprentissage dudit poisson modèle si, suite à la comparaison de l'étape c), le temps moyen nécessaire pour obtenir les poissons modèles conditionnés, ou conditionnés de manière approfondie, de l'étape b) est supérieur au temps moyen de référence et/ou le taux de réussite moyen obtenu par les poissons modèles conditionnés, ou conditionnés de manière approfondie, de l'étape b) est inférieur au taux de réussite moyen de référence.

Dans un mode de réalisation alternatif, ladite méthode d'identification de molécules, de composés, de compositions ou de formulations modifiant la mémoire de travail et/ou les capacités cognitives et/ou les capacités d'apprentissage chez un poisson modèle, comprend les étapes consistant en :
a) l'obtention de poissons modèles conditionnés, ou conditionnés de manière approfondie, par la méthode de l'invention que décrite ci-dessus, en présence de ladite molécule, dudit composé, de ladite composition ou de ladite formulation;
b) la comparaison du temps moyen nécessaire pour obtenir les poissons modèles conditionnés, ou conditionnés de manière approfondie, de l'étape a) avec un temps moyen de référence et/ou la comparaison du taux de réussite moyen obtenu par les poissons modèles conditionnés, ou conditionnés de manière approfondie, de l'étape a) avec un taux de réussite moyen de référence.

Selon ce mode de réalisation alternatif, ladite molécule, ledit composé, ladite composition ou ladite formulation restaure et/ou améliore et/ou affecte positivement, et/ou modifie positivement la mémoire de travail et/ou les capacités cognitives et/ou les capacités d'apprentissage dudit poisson modèle si, suite à la comparaison de l'étape b), le temps moyen nécessaire pour obtenir les poissons modèles conditionnés, ou conditionnés de manière approfondie, de l'étape a) est inférieur au temps moyen de référence et/ou le taux de réussite moyen obtenu par les poissons modèles conditionnés, ou conditionnés de manière approfondie, de l'étape a) est supérieur au taux de réussite moyen de référence. A l'inverse, ladite molécule, ledit composé, ladite composition ou ladite formulation perturbe et/ou inhibe et/ou affecte négativement et/ou altère et/ou modifie négativement la mémoire de travail et/ou les capacités cognitives et/ou les capacités d'apprentissage dudit poisson modèle si, suite à la comparaison de l'étape b), le temps moyen nécessaire pour obtenir les poissons modèles conditionnés, ou conditionnés de manière approfondie, de l'étape a) est supérieur au temps moyen de référence et/ou le taux de réussite moyen obtenu par les poissons modèles conditionnés, ou conditionnés de manière approfondie, de l'étape a) est inférieur au taux de réussite moyen de référence.

Quels que soient ces modes de réalisation, cette méthode permet également d'étudier l'effet de molécules, de composés, de compositions ou de formulations sur la mémoire de travail et/ou les capacités cognitives et/ou les capacités d'apprentissage chez un poisson modèle.

Le temps moyen nécessaire pour obtenir les poissons modèles conditionnés, ou conditionnés de manière approfondie, peut par exemple consister en la somme des temps (en jours, semaines ou mois) nécessaires pour que chaque individu (i.e. pour chaque poisson modèle de l'étape a)) au sein d'un groupe d'individus conditionnés simultanément/parallèlement ou séparément/indépendamment, ou conditionnés de manière approfondie simultanément/parallèlement ou séparément/indépendamment, atteigne le taux de réussite T1 ou T2 prédéterminé tel que défini ci-dessus, divisée par le nombre d'individus considérés. Alternativement, le temps moyen nécessaire pour obtenir les poissons modèles conditionnés, ou conditionnés de manière approfondie, peut par exemple consister en la somme des temps (en jours, semaines ou mois) nécessaires pour qu'un groupe d'individus conditionnés simultanément/parallèlement ou séparément/indépendamment, ou conditionnés de manière approfondie simultanément/parallèlement ou séparément/indépendamment, atteigne le taux de réussite T1 ou T2 moyen prédéterminé tel que défini ci-dessus, divisée par le nombre de groupe d'individus considérés. Le temps moyen de référence peut par exemple consister en un temps moyen nécessaire pour obtenir un poisson modèle de référence, ou un groupe de poissons modèles de référence, conditionné, ou conditionné de manière approfondie, selon la méthode de l'invention.

Le taux de réussite moyen est tel que défini ci-dessus en relation avec la méthode d'obtention de poissons modèles conditionnés, ou conditionnés de manière approfondie, de l'invention. Le taux de réussite moyen de référence peut par exemple consister en un taux de réussite moyen obtenu par un poisson modèle de référence, ou un groupe de poissons modèles de référence, soumis à la méthode d'obtention de poissons modèles conditionnés, ou conditionnés de manière approfondie, selon l'invention.

Ledit poisson modèle de référence est par exemple un poisson modèle sain. Avantageusement, le poisson modèle de référence appartenant au même genre et/ou à la même espèce que le poisson modèle de l'étape a). Avantageusement, le poisson modèle de référence n'a pas été mis en contact avec ladite molécule, ledit composé, ladite composition ou ladite formulation. Un poisson modèle de référence qui n'a pas été mis en contact avec ladite molécule, ledit composé, ladite composition ou ladite formulation est un poisson « naïf ».

Selon un mode de réalisation, le poisson modèle de l'étape a) est choisi parmi des poissons modèle d'une atteinte ou d'une déficience cognitive, résultant par exemple d'une maladie neuropsychiatrique ou neurologique (telle qu'une maladie neurodégénérative, une addiction ou une lésion au cerveau), notamment parmi les poissons modèles de l'hyperactivité, de l'autisme, de la schizophrénie, de la maladie de Parkinson, de la maladie d'Alzheimer, et d'une lésion au cerveau. Dans ce cas, le poisson modèle de référence peut être un poisson modèle sain ou un poisson modèle de ladite atteinte ou déficience cognitive. Avantageusement, le poisson modèle de référence appartient au même genre et/ou à la même espèce que le poisson modèle de l'étape a). Avantageusement, le poisson modèle de référence n'a pas été mis en contact avec ladite molécule, ledit composé, ladite composition ou ladite formulation (poisson modèle de référence « naïf »).

L'invention concerne également une méthode d'identification de molécules, de composés, de compositions ou de formulations, ou encore une méthode de criblage de molécules, de composés, de compositions ou de formulations modifiant la mémoire de travail et/ou les capacités cognitives et/ou les capacités d'apprentissage chez un poisson modèle conditionné, ou conditionné de manière approfondie, selon l'invention, comprenant les étapes consistant en :
a) la fourniture de poissons modèles conditionnés, ou conditionnés de manière approfondie, par la méthode d'obtention de poissons modèles conditionnés, ou conditionnés de manière approfondie, de l'invention, telle que décrite ci-dessus ;
b) la mise en contact des poissons modèles conditionnés, ou conditionnés de manière approfondie, de l'étape a) avec ladite molécule, ledit composé, ladite composition ou ladite formulation ;
c) la mise en oeuvre de la méthode d'obtention de poisson modèles conditionnés, ou conditionnés de manière approfondie, telle que décrite ci-dessus sur le poisson modèle de l'étape b) ;
d) la comparaison du taux de réussite moyen obtenu par les poissons modèles conditionnés, ou conditionnés de manière approfondie, de l'étape a) avec le taux de réussite moyen obtenu par les poissons modèles conditionnés, ou conditionnés de manière approfondie, de l'étape c).

Cette méthode permet également d'étudier l'effet de molécules, de composés, de compositions ou de formulations sur la mémoire de travail et/ou les capacités cognitives et/ou les capacités d'apprentissage chez un poisson modèle.

Selon un mode de réalisation, ladite molécule, ledit composé, ladite composition ou ladite formulation restaure et/ou améliore et/ou affecte positivement, et/ou modifie positivement la mémoire de travail et/ou les capacités cognitives et/ou les capacités d'apprentissage dudit poisson modèle si, suite à la comparaison de l'étape d), le taux de réussite moyen obtenu par les poissons modèles conditionnés, ou conditionnés de manière approfondie, de l'étape c) est supérieur au taux de réussite moyen obtenu par les poissons modèles conditionnés, ou conditionnés de manière approfondie, de l'étape a). A l'inverse, ladite molécule, ledit composé, ladite composition ou ladite formulation perturbe et/ou inhibe et/ou affecte négativement et/ou altère et/ou modifie négativement la mémoire de travail et/ou les capacités cognitives et/ou les capacités d'apprentissage dudit poisson modèle si, suite à la comparaison de l'étape d), le taux de réussite moyen obtenu par les poissons modèles conditionnés, ou conditionnés de manière approfondie, de l'étape c) est inférieur au taux de réussite moyen obtenu par les poissons modèles conditionnés, ou conditionnés de manière approfondie, de l'étape a).

Selon un mode de réalisation, le poisson modèle de l'étape a) est choisi parmi des poissons modèles d'une atteinte ou d'une déficience cognitive, résultant par exemple d'une maladie neuropsychiatrique ou neurologique (telle qu'une maladie neurodégénérative, une addiction ou une lésion au cerveau), notamment parmi les poissons modèles de l'hyperactivité, de l'autisme, de la schizophrénie, de la maladie de Parkinson, de la maladie d'Alzheimer, et d'une lésion au cerveau.

### Utilisation

Les poissons modèles conditionnés ou conditionnés de manière approfondie sont particulièrement adaptés à la réalisation d'études de caractérisation d'une atteinte ou d'une déficience cognitive, résultant par exemple d'une maladie neuropsychiatrique ou neurologique (telle qu'une maladie neurodégénérative, une addiction ou une lésion au cerveau) ; incluant notamment l'hyperactivité, l'autisme, la schizophrénie, la maladie de Parkinson, la maladie d'Alzheimer, et une lésion au cerveau ; s'agissant notamment des effets sur la mémoire de travail et/ou les capacités cognitives et/ou les capacités d'apprentissage chez un poisson modèle ; ou d'études de la capacité de régénération du cerveau chez un poisson modèle ; ou encore d'études d'identification de molécules, de composés, de compositions ou de formulations modifiant la mémoire de travail et/ou les capacités cognitives et/ou les capacités d'apprentissage chez un poisson modèle .

Ainsi, la présente invention concerne l'utilisation d'un poisson modèle conditionné, ou conditionné de manière approfondie, susceptible d'être obtenu par la méthode de l'invention telle que décrite ci-dessus, obtenu par la méthode de l'invention telle que décrite ci-dessus, et/ou directement obtenu par la méthode de l'invention telle que décrite ci-dessus, pour :
- étudier et/ou caractériser d'une atteinte ou d'une déficience cognitive, résultant par exemple d'une maladie neuropsychiatrique ou neurologique (telle qu'une maladie neurodégénérative, une addiction ou une lésion au cerveau) ; incluant notamment l'hyperactivité, l'autisme, la schizophrénie, la maladie de Parkinson, la maladie d'Alzheimer, et une lésion au cerveau ; s'agissant notamment des effets sur la mémoire de travail et/ou sur les capacités cognitives et/ou sur les capacités d'apprentissage chez ledit poisson modèle ;
- étudier la capacité de régénération du cerveau chez ledit poisson modèle ;
- caractériser la mémoire de travail et/ou les capacités cognitives et/ou les capacités d'apprentissage chez un poisson modèle ; ou
- identifier ou encore cribler des molécules, des composés, des compositions ou des formulations modifiant la mémoire de travail et/ou les capacités cognitives et/ou les capacités d'apprentissage chez un poisson modèle.

La présente invention concerne en outre l'utilisation / l'application de la méthode d'obtention d'un poisson modèle conditionné, ou conditionné de manière approfondie, selon l'invention, pour
- étudier et/ou caractériser une atteinte ou une déficience cognitive, résultant par exemple d'une maladie neuropsychiatrique ou neurologique (telle qu'une maladie neurodégénérative, une addiction ou une lésion au cerveau) ; incluant notamment l'hyperactivité, l'autisme, la schizophrénie, la maladie de Parkinson, la maladie d'Alzheimer, et une lésion au cerveau ; s'agissant notamment des effets sur la mémoire de travail et/ou sur les capacités cognitives et/ou sur les capacités d'apprentissage chez ledit poisson modèle ;
- étudier la capacité de régénération du cerveau chez ledit poisson modèle ;
- caractériser la mémoire de travail et/ou les capacités cognitives et/ou les capacités d'apprentissage chez un poisson modèle ; ou
- identifier ou encore cribler des molécules, des composés, des compositions ou des formulations modifiant la mémoire de travail et/ou les capacités cognitives et/ou les capacités d'apprentissage chez un poisson modèle.

Selon un mode de réalisation de l'une quelconque des utilisations décrites ci-dessus, ledit poisson modèle est choisi parmi des poissons modèles d'une atteinte ou d'une déficience cognitive, résultant par exemple d'une maladie neuropsychiatrique ou neurologique (telle qu'une maladie neurodégénérative, une addiction ou une lésion au cerveau), notamment parmi les poissons modèles de l'hyperactivité, de l'autisme, de la schizophrénie, de la maladie de Parkinson, de la maladie d'Alzheimer, et d'une lésion au cerveau.

Avantageusement, les utilisations décrites ci-dessus sont conformes aux méthodes d'application telles que définie dans la section précédente. Ainsi, les utilisations décrites ci-dessus sont mises en oeuvre selon les méthodes d'application telles que définie dans la section précédente.

### EXEMPLES

### PARTIE A: MATERIELS ET METHODES

### A.1. Elevage des poissons

Les poissons sont élevés dans une animalerie aquatique en conditions contrôlées. Ils sont nourris 2 à 3 fois par jour avec du gemma micro 300, la température est de 28 °C, le cycle jour nuit est de 14h de jour pour 10h de nuit, avec un changement d'eau continu.

### A.2. Préparation des poissons

Un lot de 10 poissons adultes *Danio rerio* (âgés de 3 à 12 mois) est placé en pièce comportement. Chaque poisson est isolé dans un aquarium test de 6L, il n'y a donc pas de contact olfactif ou chimique mais le contact visuel persiste. La température et le cycle jour nuit sont les mêmes que ci-dessus. Le changement d'eau est effectué manuellement une fois par semaine, et le nourrissage est fait une fois par jour avec du gemma micro 600. Pendant 1 semaine les poissons sont mis en habituation pour se familiariser avec leur nouvel environnement. Durant le weekend les poissons sont privés de nourriture. Cette diminution globale de la quantité de nourriture par rapport à celle fournie dans l'animalerie sera importante par la suite pour la motivation des poissons à effectuer la tâche.

### A.3. Phase de préapprentissage (pré-conditionnement)

La phase de préapprentissage (pré-conditionnement) consiste à conditionner un poisson à aller chercher une récompense dans une zone prédéfinie (zone de choix, zone de réponse ou zone de récompense, Figures 1 et 2). La récompense est positionnée dans un distributeur de nourriture (par exemple une pipette). Pour saisir la récompense, l'animal doit passer la tête dans le distributeur (Figures 1 et 2). Dans les expériences qui suivent, la récompense est un morceau de larve de moustique, nourriture très appétente mais peu protéique afin que le poisson ne soit pas rassasié.

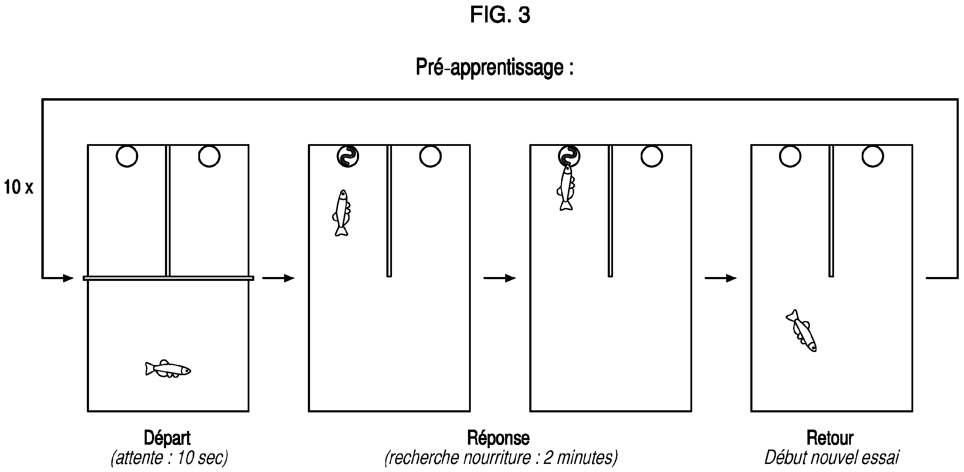

Jour 1 et 2 : Au début d'un essai, le poisson est placé dans la zone de départ et la barrière entre la zone de départ et la zone de choix est fermée durant 10s (Figure 3). La barrière est ensuite levée. Le poisson peut donc aller dans la zone de récompense gauche ou droite, il a jusqu'à 2min pour le faire (Figure 3). Si le poisson ne se positionne pas dans une zone de récompense dans le temps imparti, il est alors replacé dans la zone de départ (par exemple à l'aide d'une épuisette) et un nouvel essai commence. A l'inverse, dès qu'il s'approche de la zone de récompense, une récompense lui est fournie (Figure 3) puis on le laisse retourner dans la zone de départ. La barrière est refermée et un nouvel essai commence. Chaque session (série) comporte 10 essais consécutifs, réalisés selon la procédure décrite ci-dessus. On réalise une session (série) par jour. On réalise donc 10 essais par jour.

Cette phase de préapprentissage permet à l'individu de s'habituer à la zone de récompense afin de ne plus avoir peur de celle-ci. Il apprend également à approcher du distributeur de nourriture (pipette) : à chaque nouvel essai, la récompense est déposée de plus en plus proche du distributeur, jusqu'à la déposer à l'intérieur de celui-ci. Cette étape permet de fixer la réponse comportementale correcte : le poisson pénètre dans une zone de réponse, cette zone de réponse pouvant adopter différentes formes et tailles, par exemple comprenant, ou consistant en un orifice, à laquelle s'ajouteront par la suite des conditions plus complexes dans le cas des tests utilisés (SMTS, DMTS).

Jours 3, 4 et 5 : La phase de préapprentissage peut optionnellement se prolonger sur les jours 3, 4 ou 5 si et seulement si certains individus n'ont pas appris à récupérer la récompense dans de distributeur de récompense. Dès que tous les poissons ont appris à aller chercher la récompense dans le distributeur, on passe à l'étape suivante du protocole.

On considère qu'un poisson est préconditionné lorsqu'il obtient 70% de réussite par session (un essai étant considéré comme réussi lorsque le poisson saisi la récompense dans le distributeur de récompense).

A la fin du 5^{e} jour, un nettoyage de l'aquarium est effectué et les poissons sont nourris avec du gemma 600 afin d'avoir un apport protéique. Durant le weekend les poissons sont privés de nourriture.

### A.4. Phase de conditionnement

Commence alors la phase d'apprentissage de la tâche (phase de conditionnement). En termes scientifiques précis, le paradigme utilisé est le conditionnement opérant (réponse comportementale active à un stimulus présenté). La tâche peut consister en un « matching to sample » (MTS, ou encore « simultaneous matching to sample » (SMTS)), selon la règle explicitée ci-après. Le poisson doit choisir d'aller dans la zone de récompense qui montre une réponse identique à la consigne (ou indice) présentée dans la zone de départ. Dans le cas où la consigne est une couleur, le poisson doit choisir d'aller dans la zone de récompense qui montre la même couleur que l'indice présenté dans la zone de départ. Il sera récompensé si et seulement s'il choisit la même couleur que l'indice présenté (« sample »). Autrement dit le poisson apprend la règle : « .la zone à choisir est celle qui montre la même couleur que l'indice (« matching ») ».

### Description d'un essai d'une session dans la phase de conditionnement :

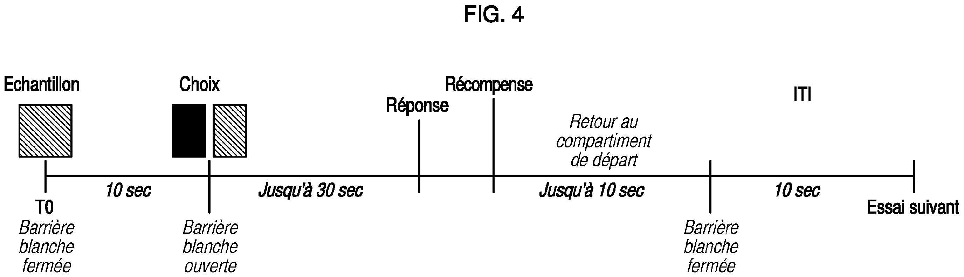

Le déroulement d'un essai dans la phase de conditionnement est résumé sur la Figure 4.

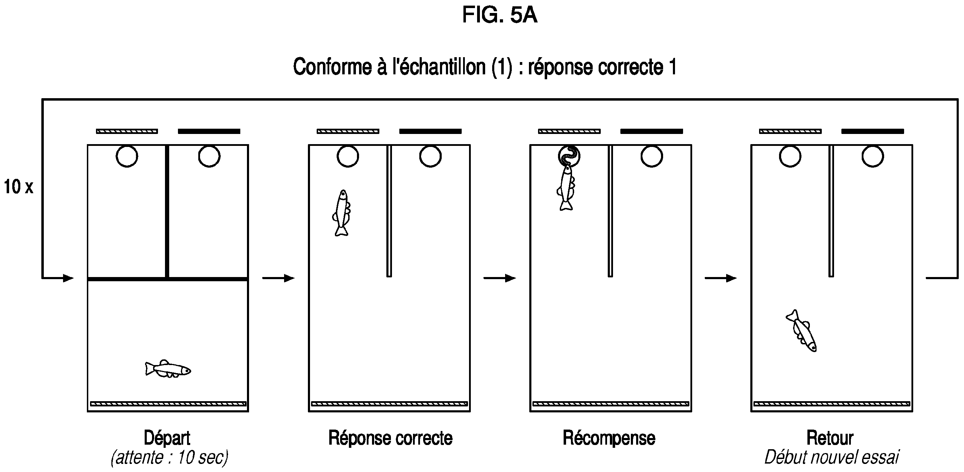

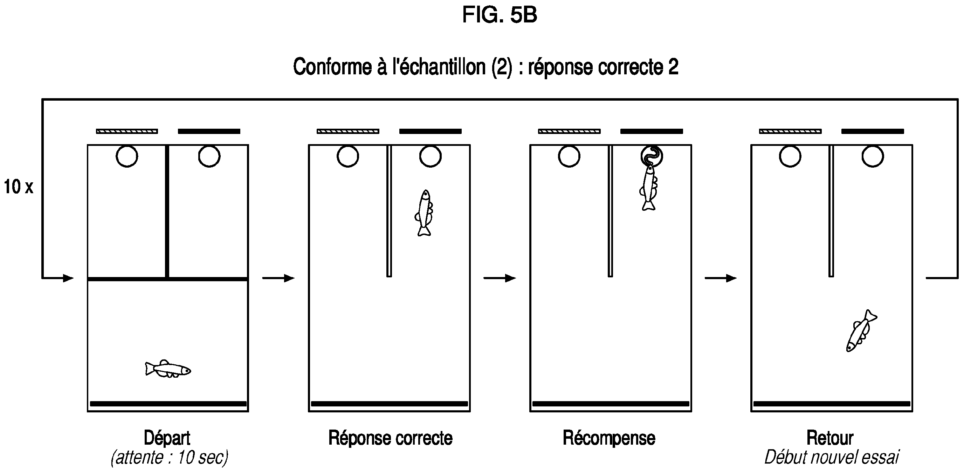

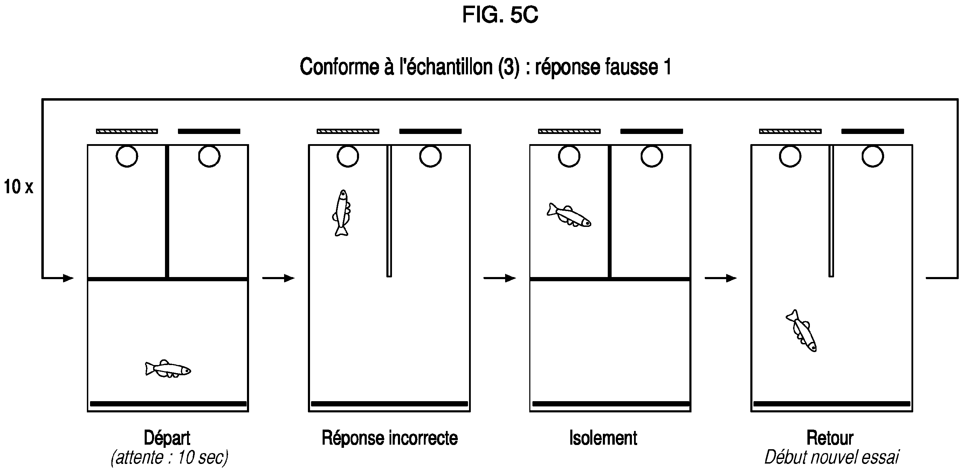

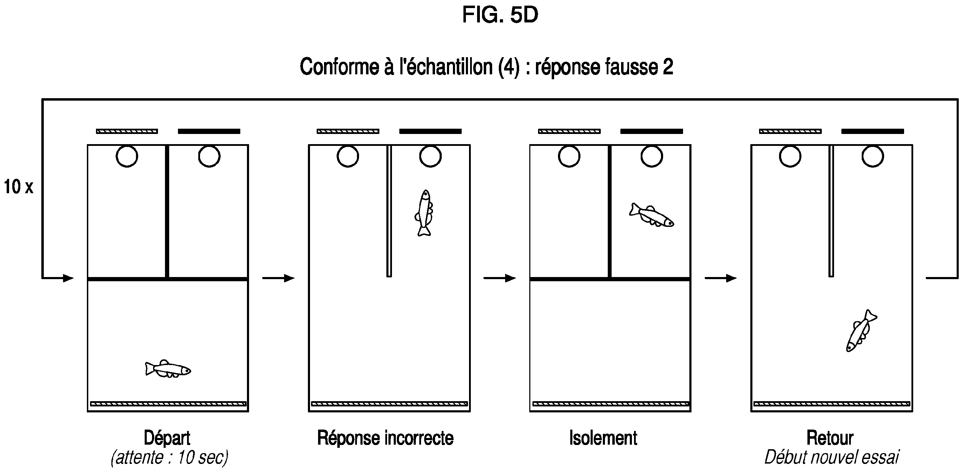

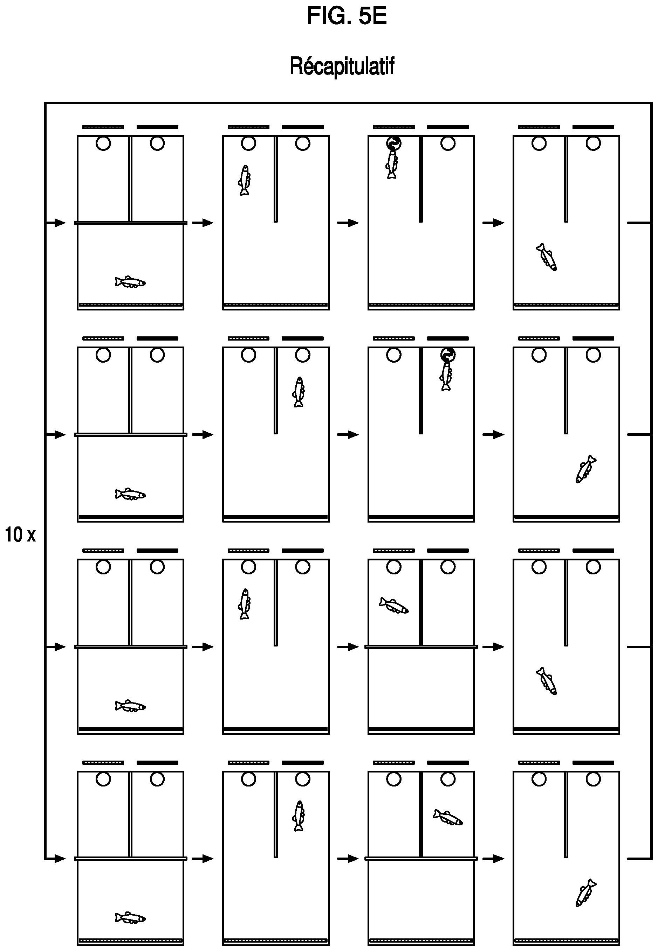

Au cours d'un essai, le poisson est placé dans la zone de départ pendant 10s sans indice (Figure 4). Cette période est appelée « intertrial interval » (ITI) ou intervalle entre les essais. Un indice (ou consigne) coloré sous forme de carte est ensuite présenté dans la zone de départ pendant 10s (Figure 5 A-D). Après ce délai la barrière est ouverte et le poisson a 30s pour choisir une zone de récompense (Figure 5 A-D). Si la zone choisie est la réponse correcte (couleur identique à celle montrée en zone de départ) alors le poisson obtient une récompense (Figure 5 A-B). Si la zone choisie correspond à la réponse « fausse » (couleur différente de celle montrée en zone de départ) alors le poisson est enfermé dans la zone de choix sans récompense pendant 30s (il s'agit d'une punition légère ; Figure 5 C-D). L'indice coloré est alors enlevé. Le poisson retourne ensuite dans la zone de départ et un nouvel essai peut commencer.

Chaque session (série) comporte 10 essais consécutifs, réalisés selon la procédure décrite ci-dessus (Figure 5 A-E). On réalise une session (série) par jour, qui peuvent être consécutifs ou non.

Le nombre de sessions (ou jours de conditionnement) nécessaires est fonction de l'individu (le nombre de jours de conditionnement de 15 jours à 1 mois). On considère que le poisson a acquis le conditionnement lorsqu'il obtient au moins 70% de bonnes réponses par session pour 3 sessions consécutives, réalisées sur 3 jours consécutifs.

### A.5. Phase de conditionnement approfondi

Une fois la phase de conditionnement acquise, la capacité de rétention d'information est testée avec une phase de conditionnement approfondi (« delayed matching to sample » ou DMTS). Durant cette phase, le poisson doit se souvenir de l'indice coloré montré et choisir la bonne zone de récompense.

### Description d'un essai d'une session dans la phase de conditionnement approfondi :

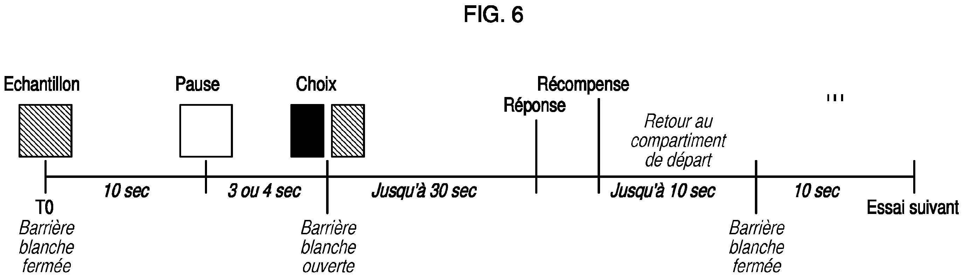

Le déroulement d'un essai est résumé sur la Figure 6.

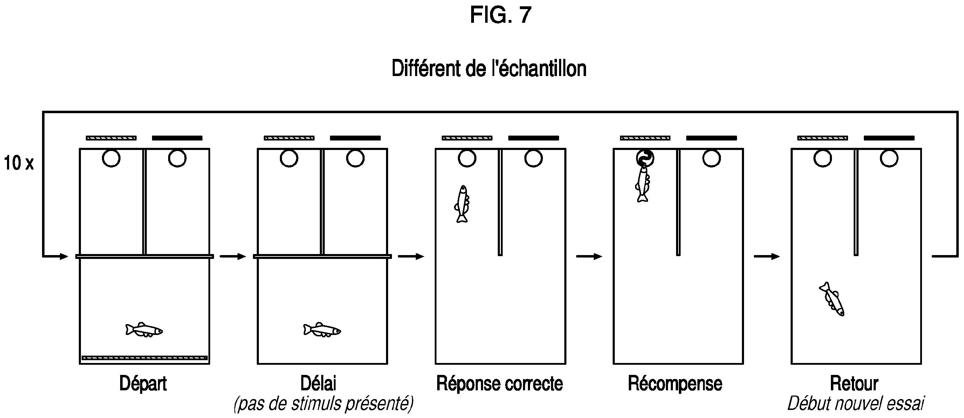

Au cours d'un essai, le poisson est dans la zone de départ pendant 10s sans indice (ITI, Figure 6) un indice coloré sous forme de carte est présenté dans la zone de départ pendant 10s (Figure 7). L'indice est ensuite retiré. La barrière est ouverte 3 sec après. Le poisson a 30s pour choisir une zone de récompense (Figure 7). Si la zone choisie est « fausse » (couleur différente de celle montrée en zone de départ) alors le poisson est enfermé dans la zone de choix sans récompense pendant 30s (il s'agit d'une punition légère). Si la zone choisie est la bonne (couleur identique à celle montrée en zone de départ) alors le poisson obtient une récompense. Le poisson revient ensuite dans la zone de départ. Et un nouvel essai peut commencer.

Chaque session (session) contient 10 essais et peut être répétée sur 3 jours consécutifs (une session par jour) pour des résultats plus précis. Si le poisson obtient 70% de bonnes réponses par session, on considère qu'il est capable de retenir l'information et que la phase de conditionnement approfondie est donc acquise.

### PARTIE B: RESULTATS

### Exemple B.1 : Expérience 1

Dans cette expérience, le Gemma Micro 600 a été utilisé comme récompense. Ces granulés sont très nourrissants et sont couramment utilisés en animalerie poisson zèbre.

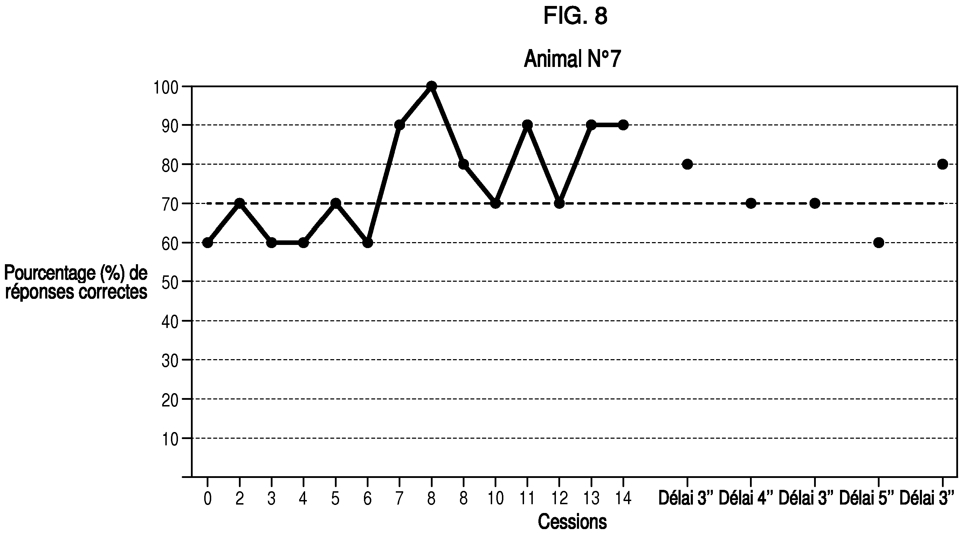

Le Tableau 1 ci-dessous montre le pourcentage (%) de réponses correctes dans chaque session (série) pour chaque animal. La Figure 8 montre, sous forme de graphe, ce pourcentage pour un poisson type (le poisson n°7 dans cette expérience).

### 8.1.1. Phase de Conditionnement ou SMTS (« simultaneous matchins-to-sample »)

Un lot de 10 poissons zèbres adultes *(Danio rerio)* a été soumis à la phase de conditionnement SMTS telle que décrite ci-dessus (paragraphe A.4).

Durant les tests, l'animal n°6 a eu un comportement de "freezing" (immobilité) et a donc été exclu de l'analyse. Les 9 autres animaux ont atteint le critère d'apprentissage stable (plus de 70% de réponses correctes par session sur trois sessions (sessions) consécutives), au moins une fois (Tableau 1 et Figure 8).

Dans la première session (série), le taux de réponses correctes était aléatoire (30-60%). Deux animaux (n°3 et 7) ont atteint 70% de réponses correctes sur la seconde session (Tableau 1 et Figure 8). Quatre poissons (n°1, 8, 9 et 10) ont atteint le critère d'apprentissage stable en 7 sessions (séries) ou moins (Tableau 1).

Pour des raisons d'ordre pratique, les 14 sessions (séries) ne correspondent pas à 14 jours consécutifs. La 2^{e} colonne "jours" dans le Tableau 1 indique le nombre de jours depuis le premier jour de l'expérience. Par exemple, il y a eu une pause de 11 jours sans essai entre la 8^{e} et la 9^{e} session (série). Il est intéressant de noter que 5 animaux (n°3, 4, 5, 7 et 9) ont obtenu plus de 70% de réussite même après 11 jours sans essai entre 2 sessions (Tableau 1 et Figure 8).

Ces résultats montrent que tous les individus ont atteint 70% de réponses correctes rapidement. Certains individus ont néanmoins eu des difficultés à maintenir ce niveau de performance de manière continue. Il a été considéré que cela pouvait être dû à un manque de motivation. Afin de tester cette hypothèse, la fréquence des expériences a été réduite : après la 6^{e} session, les sessions suivantes ont été espacées d'au moins deux jours. Cela a permis d'améliorer globalement les performances des animaux (Tableau 1 et Figure 8).

En conclusion, ces résultats montrent pour la première fois que le poisson zèbre est capable d'apprendre le SMTS. De plus cet animal est capable d'apprendre le SMTS malgré des jours d'entraînement irréguliers. Enfin, ces résultats montrent pour la première fois que le poisson zèbre est capable de conserver un taux de réponse élevé après plusieurs jours sans entrainement.

### 8.1.2. Phase Conditionnement approfondi ou DMTS (delayed matching-to-sample)

Dans cette expérience, le test DMTS a été réalisé après les 14 sessions de SMTS, sur le lot de poisson conditionné obtenu après l'expérience B.1.1 décrite ci-dessus. La durée du délai était de 3 secondes pour le premier essai. 7 poissons sur 9 ont pu atteindre plus de 70% de réponses correctes dès la première session (Tableau 1, Figure 8). La durée du délai a alors été augmenté à 4 secondes pour la session suivante. A nouveau 7 poissons sur 9 ont obtenu un taux de réponses correctes de plus de 70%, ce qui a permis de tester un délai de 5 secondes pour la session suivante. 2 sujets ont pu atteindre un taux de réponses correctes d'au moins 70%. Le délai a été réduit lors de la session suivante (3 secondes) : tous les sujets ont pu atteindre plus de 70% de taux de réponses correctes (Tableau 1, Figure 8).

Ces données montrent pour la première fois que le poisson zèbre peut réaliser le Conditionnement approfondi DMTS avec un délai pouvant aller jusqu'à 4-5 secondes.

**Tableau 1. Pourcentage de réponses correctes pour chaque animal en fonction des sessions dans l'Expérience 1.**

| | | Pourcentage (%) de réponses correctes | | | | | | | | | |
|---|---|---|---|---|---|---|---|---|---|---|---|
| sessions | jours | n°1 | n°2 | n°3 | n°4 | n°5 | n°7 | n°8 | n°9 | n°10 | moyenne |
| 1 | 1 | 40 | 30 | 50 | 40 | 40 | 60 | 40 | 50 | 40 | 43,3 |
| 2 | 2 | 60 | 40 | 70 | 30 | 60 | 70 | 50 | 60 | 60 | 55,6 |
| 3 | 3 | 50 | 60 | 80 | 60 | 60 | 60 | 70 | 40 | 60 | 60,0 |
| 4 | 4 | 70 | 80 | 60 | 80 | 80 | 60 | 80 | 70 | 60 | 71,1 |
| 5 | 5 | 80 | 60 | 60 | 60 | 60 | 70 | 80 | 70 | 70 | 67,8 |
| 6 | 8 | 80 | 40 | 70 | 60 | 80 | 60 | 70 | 100 | 100 | 73,3 |
| 7 | 10 | 60 | 60 | 90 | 70 | 40 | 90 | 90 | 50 | 100 | 72,2 |
| 8 | 12 | 80 | 70 | 60 | 50 | 60 | 100 | 70 | 90 | 60 | 71,1 |
| 9 | 23 | 60 | 60 | 80 | 80 | 80 | 80 | 60 | 70 | 60 | 70,0 |
| 10 | 25 | 70 | 90 | 60 | 50 | 80 | 70 | 80 | 100 | 80 | 75,6 |
| 11 | 29 | 60 | 80 | 90 | 80 | 70 | 90 | 90 | 80 | 90 | 81,1 |
| 12 | 32 | 80 | 60 | 70 | 80 | 70 | 70 | 60 | 70 | 60 | 68,9 |
| 13 | 36 | 60 | 90 | 70 | 80 | 100 | 90 | 90 | 80 | 70 | 81,1 |
| 14 | 39 | 90 | 80 | 100 | 70 | 70 | 90 | 90 | 90 | 80 | 84,4 |
| | | | | | | | | | | | |
| délai 3" | 43 | 50 | 80 | 60 | 90 | 80 | 80 | 90 | 70 | 70 | 74,4 |
| délai 4" | 46 | 50 | 70 | 70 | 80 | 90 | 70 | 80 | 80 | 60 | 72,2 |
| délai 3" | 52 | 80 | 80 | 80 | 60 | 90 | 70 | 70 | 50 | 60 | 71,1 |
| délai 5" | 59 | 30 | 60 | 70 | 40 | 100 | 60 | 30 | 30 | 40 | 51,1 |
| délai 3" | 61 | 90 | 80 | 90 | 80 | 80 | 80 | 80 | 90 | 80 | 83,3 |

### Exemple B.2 : Expérience 2

Une baisse de motivation, probablement liée à la satiété, ayant été observée dans l'Expérience 1, la récompense alimentaire a été changée pour l'Expérience 2. Les larves de moustique séchées ont été coupées en petits morceaux, et un morceau à la fois a été donné comme récompense.

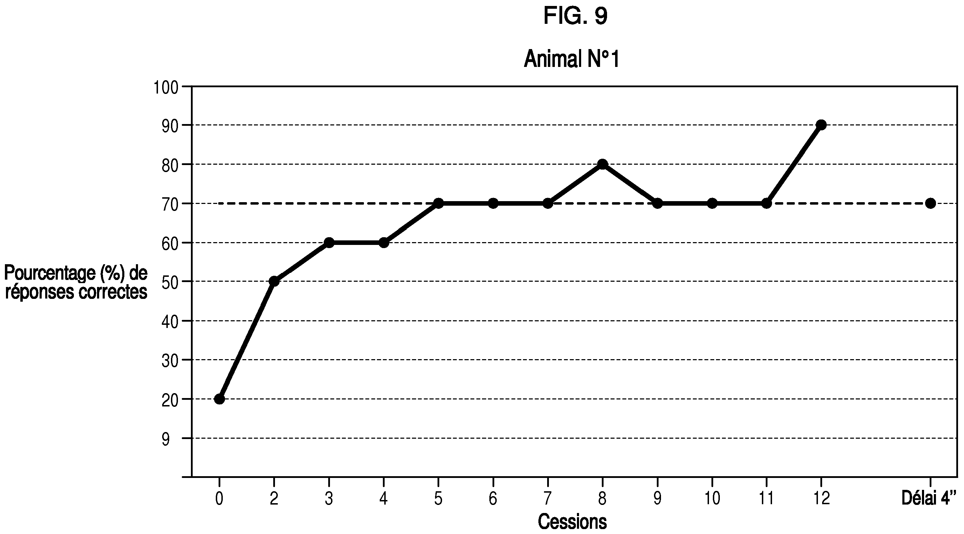

Le Tableau 2 montre le pourcentage (%) de réponses correctes dans chaque session pour chaque animal. La Figure 9 montre, sous forme de graphe, ce pourcentage pour un poisson type, le poisson n°1.

### 8.2.1. SMTS (simultaneous matchinç-to-sample)

Deux individus (n°6 et 8) ont obtenu un taux de réponses correctes de 70% dès la première session et ont gardé ce niveau de performance tout au long de l'expérience (Tableau 2). Les autres sujets ont commencé avec une performance proche du hasard, mais tous les animaux ont atteint un taux de réponses correctes de 70% au moins une fois durant les 6 premières sessions (Tableau 2 et Figure 9). Parmi les 9 animaux, 8 (à l'exception du n°10) ont atteint le critère d'apprentissage stable (70% de réussite par session sur 3 sessions consécutives) au moins une fois dès la fin de la 12^{e} session. Le niveau de performance a été bien conservé : certains individus (n°1) ont maintenu un niveau de réponse correcte de plus de 70% pendant 8 sessions continues (Tableau 2 et Figure 9).

Ainsi, le morceau de larve de moustique comme récompense a permis d'obtenir de meilleurs résultats que le Gemma Micro 600, dans les conditions testées.

### 8.2.2. DMTS (delayed matching-to-sample)

Le test de DMTS avec 4 secondes de délai a été réalisé après que la majorité des animaux a atteint le critère d'apprentissage stable en phase de conditionnement MTS.

Les résultats montrent qu'au moins la moitié des individus est capable de réaliser le DMTS avec un taux de réussite d'au moins 70%, dès la première session (Tableau 2 et Figure 9).

**Tableau 2. Pourcentage de réponses correctes pour chaque animal en fonction des sessions dans l'Expérience 2.**

| | | Pourcentage (%) de réponses correctes | | | | | | | | | |
|---|---|---|---|---|---|---|---|---|---|---|---|
| sessions | jours | n°1 | n°2 | n°3 | n°4 | n°5 | n°6 | n°7 | n°8 | n°10 | moyenne |
| 1 | 1 | 20 | 50 | 50 | 40 | 60 | 70 | 40 | 70 | 50 | 50,0 |
| 2 | 2 | 50 | 40 | 50 | 80 | 60 | 70 | 60 | 50 | 60 | 57,8 |
| 3 | 3 | 60 | 40 | 50 | 50 | 60 | 70 | 50 | 90 | 50 | 57,8 |
| 4 | 4 | 60 | 60 | 80 | 90 | 70 | 60 | 80 | 80 | 60 | 71,1 |
| 5 | 5 | 70 | 70 | 50 | 60 | 70 | 70 | 70 | 30 | 50 | 60,0 |
| 6 | 8 | 70 | 50 | 60 | 70 | 80 | 80 | 70 | 30 | 70 | 64,4 |
| 7 | 9 | 70 | 70 | 70 | 80 | 50 | 60 | 70 | 70 | 90 | 70,0 |
| 8 | 11 | 80 | 90 | 80 | 70 | 40 | 70 | 50 | 90 | 50 | 68,9 |
| 9 | 12 | 70 | 80 | 60 | 70 | 50 | 90 | 70 | 80 | 50 | 68,9 |
| 10 | 16 | 70 | 70 | 80 | 70 | 60 | 70 | 70 | 70 | 60 | 68,9 |
| 11 | 17 | 70 | 70 | 100 | 80 | 80 | 80 | 90 | 100 | 80 | 83,3 |
| 12 | 23 | 90 | 70 | 90 | 80 | 100 | 90 | 70 | 90 | 60 | 82,2 |
| | | | | | | | | | | | |
| délai 4" | 24 | 70 | 50 | 90 | 50 | 60 | 90 | 80 | 80 | 60 | 70,0 |

### REFERENCES BIBLIOGRAPHIQUES

Arthur and Levin, 2001, Anim. Cogn., 4:125-131;
Bloch et al., 2019, Behavioural Brain Research, 370;
Colwill et al., 2005, Behav Processes, Aug 31;70(1):19-31 ;
Gierszewski et al., 2013, PLoS One 8(2):e57363 ;
Goldman and Shapiro, 1979, J. of the Exp. Analysis of Behaviour, Mar;31(2):259-266;
Schluessel et al., 2012, Anim. Cogn., Jul;15(4):525-37.
Schuster and Amtsfeld, 2002, J Exp Biol., Feb;205(Pt 4):549-57;
Sovrano and Bisazza, 2009, Anim Cogn., 2008 Jan;11(1):161-6;
Wyzisk and Neumeyer, 2007, Vis Neurosci., May-Jun;24(3):291-8.

## Revendications

1. Méthode d'obtention de poissons modèles conditionnés comprenant une phase de conditionnement a), ladite phase de conditionnement consistant en au moins une session d'au moins deux essais consécutifs ;
chaque essai comprenant les étapes consistant à :
(a-i) présenter une consigne audit poisson modèle ;
(a-ii) présenter simultanément au moins deux réponses distinctes au poisson modèle de l'étape (a-i) pendant une durée Dᵣₐ, lesdites au moins deux réponses distinctes comprenant au moins une réponse identique à ladite consigne et au moins une réponse différente de ladite consigne ; et
(a-iii) détecter la réponse choisie par le poisson modèle de l'étape (a-ii) parmi lesdites au moins deux réponses distinctes, pendant ladite durée Dᵣₐ, par exemple au moyen de capteurs ;
ledit poisson modèle étant conditionné lorsqu'il choisit au moins une de ladite ou desdites réponses identiques à ladite consigne avec un taux de réussite T1 prédéterminé par session.

2. Méthode selon la revendication 1, comprenant en outre une phase de conditionnement approfondi b) **caractérisée en ce qu'**elle consiste en au moins une session d'au moins deux essais consécutifs ;
chaque essai comprenant les étapes consistant à :
(b-i) présenter une consigne audit poisson modèle conditionné durant une durée déterminée ;
(b-ii) retirer ladite consigne puis observer un délai d'une durée D1 prédéterminée, durant laquelle aucune consigne n'est présentée au poisson modèle de l'étape (b-i) ;
(b-iii) présenter simultanément au moins deux réponses distinctes au poisson modèle de l'étape (b-ii) pendant une durée D_{rb}, lesdites au moins deux réponses distinctes comprenant au moins une réponse identique à ladite consigne et au moins une réponse différente de ladite consigne ; et
(b-iv) détecter la réponse choisie par le poisson modèle de l'étape (b-iii) parmi lesdites au moins deux réponses distinctes, pendant ladite durée D_{rb}, par exemple au moyen de capteurs ;
ledit poisson modèle étant conditionné de manière approfondie lorsqu'il choisit au moins une de ladite ou desdites réponses identiques à ladite consigne avec un taux de réussite T2 prédéterminé par session.

3. Méthode selon la revendication 2, dans laquelle la durée D1 dudit délai va de 0,5 à 10 secondes, de préférence de 0,7 à 9 secondes, de préférence encore de 0,9 à 8 secondes, de préférence encore de 1 à 7 secondes, de préférence encore de 1,5 à 6 secondes, de préférence encore de 2 à 5 secondes, de préférence encore de 2,5 à 4,5 secondes, de préférence encore de 3 à 4 secondes.

4. Méthode selon l'une quelconque des revendications précédentes, dans laquelle :
- ledit taux de réussite T1 est d'au moins 70%, de préférence d'au moins 75%, de préférence encore d'au moins 80%, de préférence encore d'au moins 85%, de préférence encore d'au moins 90%, de préférence encore d'au moins 95%, de préférence encore de 100% ; et/ou
- ledit taux de réussite T1 est atteint pour au moins deux sessions consécutives, ledit taux de réussite T1 étant de préférence atteint pour au moins deux sessions consécutives dès la quinzième session réalisée, de préférence encore dès la douzième session réalisée, de préférence encore dès la onzième session réalisée, de préférence encore dès la dixième session réalisée, de préférence encore dès la neuvième session réalisée, de préférence encore dès la huitième session réalisée

5. Méthode selon la revendication 2, dans laquelle ledit taux de réussite T2 est d'au moins 70%, de préférence d'au moins 75%, de préférence encore d'au moins 80%, de préférence encore d'au moins 85%, de préférence encore d'au moins 90%, de préférence encore d'au moins 95%, de préférence encore de 100%.

6. Méthode selon l'une quelconque des revendications précédentes, dans laquelle ladite durée Dᵣₐ va de 1 seconde à 300 secondes, de préférence de 3 secondes à 240 secondes, de préférence de 5 secondes à 200 secondes, de préférence de 10 secondes à 180 secondes, de préférence encore de 15 secondes à 120 secondes, de préférence de 20 secondes à 100 secondes, de préférence de 25 secondes à 90 secondes, de préférence de 30 secondes à 60 secondes, de préférence encore de 25 secondes à 35 secondes.

7. Méthode selon la revendication 2, dans laquelle ladite durée D_{rb} va de 1 seconde à 300 secondes, de préférence de 3 secondes à 240 secondes, de préférence de 5 secondes à 200 secondes, de préférence de 10 secondes à 180 secondes, de préférence encore de 15 secondes à 120 secondes, de préférence de 20 secondes à 100 secondes, de préférence de 25 secondes à 90 secondes, de préférence de 30 secondes à 60 secondes, de préférence encore de 25 secondes à 35 secondes.

8. Méthode selon l'une quelconque des revendications précédentes, dans laquelle ladite consigne et/ou ladite au moins une réponse est un stimulus visuel, de préférence un stimulus visuel choisi parmi une couleur, une forme, une image, un objet, un signal lumineux ou une combinaison quelconque de l'un de ceux-ci.

9. Méthode selon l'une quelconque des revendications précédentes, dans laquelle l'étape de détection de la réponse choisie par le poisson modèle comprend une détection du passage dudit poisson modèle à travers une zone de réponse, ladite zone de réponse comprenant ou consistant de préférence en une ligne de réponse, ladite ligne de réponse comprenant ou consistant de préférence en un orifice de réponse.

10. Méthode selon l'une quelconque des revendications précédentes, comprenant une étape additionnelle (a-iv) et/ou (b-v), ladite étape additionnelle comprenant :
- la distribution d'une récompense audit poisson modèle lorsqu'il a choisi au moins une de ladite ou desdites réponses identiques à ladite consigne pendant ladite durée Dᵣₐ, ladite récompense comprenant de préférence de la nourriture adaptée audit poisson modèle, de préférence encore de la nourriture appétente et/ou pauvre sur le plan nutritionnel pour ledit poisson modèle; ou
- la non distribution de récompense audit poisson modèle lorsqu'il a choisi au moins une de ladite ou desdites réponses différentes de ladite consigne ; ou optionnellement, lorsqu'il n'a choisi aucune réponse) pendant ladite durée Dᵣₐ ; et, optionnellement, l'isolement et/ou la mise en obscurité dudit poisson modèle dudit poisson modèle, lorsqu'il a choisi au moins une de ladite ou desdites réponses différentes de ladite consigne ;ou optionnellement, lorsqu'il n'a choisi aucune réponse ; pendant ladite durée Dᵣₐ.

11. Méthode selon l'une quelconque des revendications 2 à 10, comprenant une étape additionnelle (b-v), ladite étape additionnelle comprenant :
- la distribution d'une récompense audit poisson modèle lorsqu'il a choisi au moins une de ladite ou desdites réponses identiques à ladite consigne pendant ladite durée (D_{rb}), ladite récompense comprenant de préférence de la nourriture adaptée audit poisson modèle, de préférence encore de la nourriture appétente et/ou pauvre sur le plan nutritionnel pour ledit poisson modèle; ou
- la non distribution de récompense audit poisson modèle lorsqu'il a choisi au moins une de ladite ou desdites réponses différentes de ladite consigne ; ou optionnellement, lorsqu'il n'a choisi aucune réponse ; pendant ladite durée (D_{rb}), et, optionnellement, l'isolement et/ou la mise en obscurité dudit poisson modèle dudit poisson modèle, lorsqu'il a choisi au moins une de ladite ou desdites réponses différentes de ladite consigne ; ou optionnellement, lorsqu'il n'a choisi aucune réponse ; pendant ladite durée (D_{rb}).

12. Méthode selon l'une quelconque des revendications précédentes, dans laquelle ledit poisson modèle est choisi parmi les poissons modèles appartenant aux genres *Danio* sp., *Pseudotropheus* sp., *Gnathonemus* sp., *Pomacentrus* sp., *Phoxinus* sp., *Xenotoca* sp., *Astronotus* sp., *Oryzias sp., Nothobranchius sp., Astyanax sp.* et *Carassius* sp., de préférence parmi le *Danio rerio, Metriaclima zebra, Gnathonemus petersii, Pomacentrus amboinensis, Phoxinus phoxinus, Xenotoca eiseni, Astronotus ocellatus, Oryzias latipes, Nothobranchius furzeri, Astyanax mexicanus* et *Carassius auratus.*

13. Dispositif d'obtention de poisson modèles conditionnés (1), comprenant :
- un bac (2) comprenant une paroi (5) délimitant une cavité (6) propre à contenir de l'eau pour le poisson, la cavité (6) comprenant une zone de départ (7) et une zone de choix (8),
- une première cloison (3) mobile entre une position fermée dans laquelle la première cloison (3) empêche une circulation du poisson modèle entre la zone de départ (7) et la zone de choix (8), et entre une position ouverte dans laquelle la première cloison (3) autorise une circulation du poisson modèle entre la zone de départ (7) et la zone de choix (8),
- une deuxième cloison (4) séparant la zone de choix (8) en un premier compartiment (9) et un deuxième compartiment (10), et
- un premier élément de présentation (11) pour présenter une consigne dans la zone de départ (7), un deuxième élément de présentation (12) pour présenter une première réponse dans le premier compartiment (9) et un troisième élément de présentation (13) pour présenter une deuxième réponse dans le deuxième compartiment (10),
ledit dispositif étant **caractérisé en ce que** le premier élément de présentation comprend un premier panneau d'affichage, sur lequel figure la consigne, le deuxième élément de présentation comprend un deuxième panneau d'affichage, sur lequel figure la première réponse et le troisième élément de présentation comprend un troisième panneau d'affichage, sur lequel figure la deuxième réponse ; et
dans lequel l'une de la première réponse et de la deuxième réponse est identique à la consigne et l'autre de la première réponse et de la deuxième réponse est différente de la consigne.

14. Dispositif selon la revendication 13, dans lequel la consigne est une couleur, et l'un de la première réponse et de la deuxième réponse est une couleur identique à la couleur de consigne et l'autre de la première réponse et de la deuxième réponse est une couleur différente de la couleur de consigne ; et/ou
dans lequel la première cloison mobile est opaque à un rayonnement lumineux dans une gamme de longueurs d'onde dans le vide comprise entre 380 à 780 nanomètres.

15. Utilisation d'un poisson modèle conditionné obtenu par la méthode selon l'une quelconque des revendications 1 à 12, pour caractériser une atteinte ou une déficience cognitive, résultant par exemple d'une maladie neuropsychiatrique ou neurologique ;telle qu'une maladie neurodégénérative, une addiction ou une lésion au cerveau ; notamment choisie parmi l'hyperactivité, l'autisme, la schizophrénie, la maladie de Parkinson, la maladie d'Alzheimer, et d'une lésion au cerveau ; s'agissant notamment des effets sur la mémoire de travail et/ou sur les capacités cognitives et/ou sur les capacités d'apprentissage chez un poisson modèle ; ou pour étudier la capacité de régénération du cerveau chez un poisson modèle ; ou encore pour caractériser la mémoire de travail et/ou les capacités cognitives et/ou les capacités d'apprentissage chez un poisson modèle ; ou encore pour identifier des molécules, des composés, des compositions ou des formulations modifiant la mémoire de travail et/ou les capacités cognitives et/ou les capacités d'apprentissage chez un poisson modèle ;
dans laquelle ledit poisson modèle est choisi parmi des poissons modèles de l'hyperactivité, de l'autisme, de la schizophrénie, de la maladie de Parkinson, de la maladie d'Alzheimer et d'une lésion au cerveau.

## Patentansprüche

1. Verfahren zur Erzeugung von konditionierten Modellfischen, umfassend eine Konditionierungsphase a), wobei die Konditionierungsphase aus mindestens einer Sitzung von mindestens zwei aufeinanderfolgenden Versuchen besteht;
wobei jeder Versuch die folgenden Schritte umfasst:
(a-i) Präsentieren einer Anweisung gegenüber dem Modellfisch;
(a-ii) gleichzeitiges Präsentieren von mindestens zwei unterschiedlichen Antworten gegenüber dem Modellfisch aus Schritt (a-i) während einer Dauer Dra, wobei die mindestens zwei unterschiedlichen Antworten mindestens eine mit der Anweisung identische Antwort und mindestens eine von der Anweisung verschiedene Antwort umfassen; und
(a-iii) Erkennen der von dem Modellfisch aus Schritt (a-ii) gewählten Antwort aus den mindestens zwei unterschiedlichen Antworten, während der Dauer Dᵣₐ, zum Beispiel mittels Sensoren;
wobei der Modellfisch bei der Auswahl mindestens einer der Antwort oder der Antworten, die mit der Anweisung identisch sind, mit einer vorbestimmten Erfolgsquote T1 pro Sitzung konditioniert wird.

2. Verfahren nach Anspruch 1, das ferner eine Phase der vertieften Konditionierung b) umfasst, **dadurch gekennzeichnet, dass** sie aus mindestens einer Sitzung von mindestens zwei aufeinanderfolgenden Versuchen besteht;
wobei jeder Versuch die folgenden Schritte umfasst:
(b-i) Präsentieren einer Anweisung gegenüber dem konditionierten Modellfisch während einer bestimmten Dauer;
(b-ii) Wegnehmen der Anweisung und dann Beobachten einer Verzögerung von einer vorbestimmten Dauer D1, während der keine Anweisung gegenüber dem Modellfisch aus Schritt (b-i) präsentiert wird;
(b-iii) gleichzeitiges Präsentieren von mindestens zwei unterschiedlichen Antworten gegenüber dem Modellfisch aus Schritt (b-ii) während einer Dauer D_{rb}, wobei die mindestens zwei unterschiedlichen Antworten mindestens eine Antwort, die mit der Anweisung identisch ist, und mindestens eine Antwort, die von der Anweisung verschieden ist, umfassen; und
(b-iv) Erkennen der von dem Modellfisch aus Schritt (b-iii) aus den mindestens zwei unterschiedlichen Antworten gewählten Antwort, während der Dauer D_{rb}, zum Beispiel mithilfe von Sensoren;
wobei der Modellfisch beim Auswählen von mindestens einer der Antwort oder der Antworten, die mit der Anweisung identisch sind, mit einer vorbestimmten Erfolgsquote T2 pro Sitzung vertieft konditioniert wird.

3. Verfahren nach Anspruch 2, wobei die Dauer D1 der Verzögerung von 0,5 bis 10 Sekunden, vorzugsweise von 0,7 bis 9 Sekunden, weiterhin vorzugsweise von 0,9 bis 8 Sekunden, weiterhin vorzugsweise von 1 bis 7 Sekunden, weiterhin vorzugsweise von 1,5 bis 6 Sekunden, weiterhin vorzugsweise von 2 bis 5 Sekunden, weiterhin vorzugsweise von 2,5 bis 4,5 Sekunden, weiterhin vorzugsweise von 3 bis 4 Sekunden reicht.

4. Verfahren nach einem der vorstehenden Ansprüche, wobei:
- die Erfolgsquote T1 mindestens 70 %, vorzugsweise mindestens 75 %, weiterhin vorzugsweise mindestens 80 %, weiterhin vorzugsweise mindestens 85 %, weiterhin vorzugsweise mindestens 90 %, weiterhin vorzugsweise mindestens 95 %, weiterhin vorzugsweise 100 % beträgt; und/oder
- die Erfolgsquote T1 für mindestens zwei aufeinanderfolgende Sitzungen erreicht wird, wobei die Erfolgsquote T1 vorzugsweise für mindestens zwei aufeinanderfolgende Sitzungen ab der fünfzehnten durchgeführten Sitzung, weiterhin vorzugsweise ab der zwölften durchgeführten Sitzung, weiterhin vorzugsweise ab der elften durchgeführten Sitzung, weiterhin vorzugsweise ab der zehnten durchgeführten Sitzung, weiterhin vorzugsweise ab der neunten durchgeführten Sitzung, weiterhin vorzugsweise ab der achten durchgeführten Sitzung erreicht wird.

5. Verfahren nach Anspruch 2, wobei die Erfolgsquote T2 mindestens 70 %, vorzugsweise mindestens 75 %, weiterhin vorzugsweise mindestens 80 %, weiterhin vorzugsweise mindestens 85 %, weiterhin vorzugsweise mindestens 90 %, weiterhin vorzugsweise mindestens 95 %, weiterhin vorzugsweise 100 % beträgt.

6. Verfahren nach einem der vorstehenden Ansprüche, wobei die Dauer Dᵣₐ von 1 Sekunde bis 300 Sekunden reicht, vorzugsweise von 3 Sekunden bis 240 Sekunden, vorzugsweise von 5 Sekunden bis 200 Sekunden, vorzugsweise von 10 Sekunden bis 180 Sekunden, weiterhin vorzugsweise von 15 Sekunden bis 120 Sekunden, vorzugsweise von 20 Sekunden bis 100 Sekunden, vorzugsweise von 25 Sekunden bis 90 Sekunden, vorzugsweise von 30 Sekunden bis 60 Sekunden, weiterhin vorzugsweise von 25 Sekunden bis 35 Sekunden.

7. Verfahren nach Anspruch 2, wobei die Dauer D_{rb} von 1 Sekunde bis 300 Sekunden reicht, vorzugsweise von 3 Sekunden bis 240 Sekunden, vorzugsweise von 5 Sekunden bis 200 Sekunden, vorzugsweise von 10 Sekunden bis 180 Sekunden, weiterhin vorzugsweise von 15 Sekunden bis 120 Sekunden, vorzugsweise von 20 Sekunden bis 100 Sekunden, vorzugsweise von 25 Sekunden bis 90 Sekunden, vorzugsweise von 30 Sekunden bis 60 Sekunden, weiterhin vorzugsweise von 25 Sekunden bis 35 Sekunden.

8. Verfahren nach einem der vorstehenden Ansprüche, wobei die Anweisung und/oder die mindestens eine Antwort ein visueller Reiz ist, vorzugsweise ein visueller Reiz, ausgewählt aus einer Farbe, einer Form, einem Bild, einem Objekt, einem Lichtsignal oder einer beliebigen Kombination davon.

9. Verfahren nach einem der vorstehenden Ansprüche, wobei der Schritt des Erkennens der von dem Modellfisch ausgewählten Antwort ein Erkennen des Durchgangs des Modellfischs durch einen Antwortbereich umfasst, wobei der Antwortbereich vorzugsweise eine Antwortlinie umfasst oder aus dieser besteht, wobei die Antwortlinie vorzugsweise eine Antwortöffnung umfasst oder aus dieser besteht.

10. Verfahren nach einem der vorstehenden Ansprüche, umfassend einen zusätzlichen Schritt (a-iv) und/oder (b-v), wobei der zusätzliche Schritt umfasst:
- Verteilen einer Belohnung an den Modellfisch, wenn er mindestens eine der Antwort oder der Antworten ausgewählt hat, die mit der Anweisung identisch sind, während der Dauer Dra, wobei die Belohnung vorzugsweise Futter umfasst, das für den Modellfisch geeignet ist, und vorzugsweise Futter, das für den Modellfisch schmackhaft und/oder nährstoffarm ist; oder
- Nichtverteilen einer Belohnung an den Modellfisch, wenn er mindestens eine der Antwort oder der Antworten, die von der Anweisung verschieden sind, ausgewählt hat; oder gegebenenfalls, wenn er keine Antwort ausgewählt hat),, während der Dauer Dᵣₐ; und gegebenenfalls Isolieren und/oder Bringen in Dunkelheit des Modellfisches, wenn er mindestens eine der Antwort oder der Antworten, die von der Anweisung verschieden sind, ausgewählt hat; oder gegebenenfalls, wenn er keine Antwort ausgewählt hat, während der Dauer Dᵣₐ.

11. Verfahren nach einem der Ansprüche 2 bis 10, umfassend einen zusätzlichen Schritt (b-v), wobei der zusätzliche Schritt umfasst:
- Verteilen einer Belohnung an den Modellfisch, wenn er mindestens eine der Antwort oder der Antworten ausgewählt hat, die mit der Anweisung identisch ist/sind, während der Dauer (D_{rb}), wobei die Belohnung vorzugsweise Futter umfasst, das für den Modellfisch geeignet ist, vorzugsweise Futter, das für den Modellfisch schmackhaft und/oder nährstoffarm ist; oder
- Nichtverteilen einer Belohnung an den Modellfisch, wenn er mindestens eine der Antwort oder der Antworten ausgewählt hat, die von der Anweisung verschieden sind; oder gegebenenfalls, wenn er keine Antwort ausgewählt hat, während der Dauer (D_{rb}), und gegebenenfalls Isolieren und/oder Bringen in Dunkelheit des Modellfisches, wenn er mindestens eine der Antwort oder der Antworten ausgewählt hat, die von der Anweisung verschieden sind; oder gegebenenfalls, wenn er keine Antwort ausgewählt hat, während der Dauer (D_{rb}).

12. Verfahren nach einem der vorstehenden Ansprüche, wobei der Modellfisch ausgewählt ist aus Modellfischen der Gattungen *Danio* sp., *Pseudotropheus* sp., *Gnathonemus* sp., *Pomacentrus* sp., *Phoxinus* sp., *Xenotoca* sp., *Astronotus* sp., *Oryzias sp., Nothobranchius sp., Astyanax sp.* und *Carassius* sp., vorzugsweise aus *Danio rerio, Metriaclima zebra, Gnathonemus petersii, Pomacentrus amboinensis, Phoxinus phoxinus, Xenotoca eiseni, Astronotus ocellatus, Oryzias latipes, Nothobranchius furzeri, Astyanax mexicanus* und *Carassius auratus.*

13. Vorrichtung zur Erzeugung von konditionierten Modellfischen (1), umfassend:
- einen Behälter (2) umfassend eine Wand (5), die einen Hohlraum (6) begrenzt, der geeignet ist, Wasser für den Fisch zu enthalten, wobei der Hohlraum (6) eine Startzone (7) und eine Auswahlzone (8) umfasst,
- eine erste Trennwand (3), die zwischen einer geschlossenen Position, in der die erste Trennwand (3) eine Zirkulation des Modellfisches zwischen dem Startbereich (7) und dem Auswahlbereich (8) verhindert, und zwischen einer offenen Position, in der die erste Trennwand (3) eine Zirkulation des Modellfisches zwischen dem Startbereich (7) und dem Auswahlbereich (8) zulässt, beweglich ist,
- eine zweite Trennwand (4), die den Auswahlbereich (8) in ein erstes Abteil (9) und ein zweites Abteil (10) unterteilt, und
- ein erstes Präsentationselement (11) zum Präsentieren einer Anweisung im Startbereich (7), ein zweites Präsentationselement (12) zum Präsentieren einer ersten Antwort im ersten Abteil (9) und ein drittes Präsentationselement (13) zum Präsentieren einer zweiten Antwort im zweiten Abteil (10),
wobei die Vorrichtung **dadurch gekennzeichnet ist, dass** das erste Präsentationselement eine erste Anzeigetafel umfasst, auf der die Anweisung steht, das zweite Darstellungselement eine zweite Anzeigetafel umfasst, auf der die erste Antwort steht, und das dritte Präsentationselement eine dritte Anzeigetafel umfasst, auf der die zweite Antwort steht; und
wobei eine der ersten Antwort und der zweiten Antwort mit der Anweisung identisch ist und die andere der ersten Antwort und der zweiten Antwort von der Anweisung verschieden ist.

14. Vorrichtung nach Anspruch 13, wobei die Anweisung eine Farbe ist und eine von der ersten Antwort und der zweiten Antwort eine Farbe ist, die mit der Anweisungsfarbe identisch ist, und die andere von der ersten Antwort und der zweiten Antwort eine Farbe ist, die von der Anweisungsfarbe verschieden ist; und/oder
wobei die erste bewegliche Trennwand für eine Lichtstrahlung in einem Vakuumwellenlängenbereich von 380 bis 780 Nanometer undurchlässig ist.

15. Verwendung eines konditionierten Modellfischs, der durch das Verfahren nach einem der Ansprüche 1 bis 12 erzeugt wird, zur Charakterisierung einer kognitiven Beeinträchtigung oder eines kognitiven Defizits, das beispielsweise das Ergebnis einer neuropsychiatrischen oder neurologischen Erkrankung ist; wie einer neurodegenerativen Erkrankung, einer Sucht oder einer Hirnverletzung; insbesondere ausgewählt aus Hyperaktivität, Autismus, Schizophrenie, Parkinson-Krankheit, Alzheimer-Krankheit, und einer Hirnverletzung; insbesondere hinsichtlich der Auswirkungen auf das Arbeitsgedächtnis und/oder die kognitiven Fähigkeiten und/oder die Lernfähigkeit bei einem Modellfisch; oder zur Untersuchung der Regenerationsfähigkeit des Gehirns bei einem Modellfisch; oder zur Charakterisierung des Arbeitsgedächtnisses und/oder der kognitiven Fähigkeiten und/oder der Lernfähigkeit bei einem Modellfisch; oder zur Identifizierung von Molekülen, Verbindungen, Zusammensetzungen oder Formulierungen, die das Arbeitsgedächtnis und/oder die kognitiven Fähigkeiten und/oder die Lernfähigkeit bei einem Modellfisch verändern;
wobei der Modellfisch ausgewählt ist aus Modellfischen für Hyperaktivität, Autismus, Schizophrenie, Parkinson-Krankheit, Alzheimer-Krankheit und einer Gehirnverletzung.

## Claims

1. A method for obtaining conditioned model fish comprising a conditioning phase a), said conditioning phase consisting of at least one session of at least two consecutive trials;
each trial comprising the steps of:
(a-i) presenting an instruction to the model fish ;
(a-ii) simultaneously present at least two distinct responses to the model fish of step (a-i) for a duration Dᵣₐ , said at least two distinct responses comprising at least one response identical to said instruction and at least one response different from said instruction; and
(a-iii) detecting the response chosen by the model fish in step (a-ii) among said at least two distinct responses, during said duration Dᵣₐ, for example by means of sensors;
said model fish being conditioned when it chooses at least one of said response(s) identical to said instruction with a predetermined success rate T1 per session.

2. The method according to claim 1, further comprising a deep conditioning phase b) **characterized in that** it consists of at least one session of at least two consecutive trials;
each trial comprising the steps consisting of :
(b-i) presenting an instruction to said conditioned model fish for specified duration;
(b-ii) removing said instruction and then observing a delay of a predetermined duration D1, during which no instruction is presented to the model fish of step (b-i);
(b-iii) simultaneously presenting at least two distinct responses to the model fish of step (b-ii) for a duration D_{rb}, said at least two distinct responses comprising at least one response identical to said instruction and at least one response different from said instruction; and
(b-iv) detecting the response chosen by the model fish in step (b-iii) from said at least two distinct responses, during said duration D_{rb}, for example by means of sensors;
said model fish being deeply conditioned when it chooses at least one of said identical response to said instruction with a predetermined success rate T2 per session.

3. The method according to claim 2, wherein the duration D1 of said delay ranges from 0.5 to 10 seconds, preferably from 0.7 to 9 seconds, more preferably from 0.9 to 8 seconds, more preferably from 1 to 7 seconds, more preferably from 1.5 to 6 seconds, more preferably from 2 to 5 seconds, more preferably from 2.5 to 4.5 seconds, more preferably from 3 to 4 seconds.

4. The method according to any one of the preceding claims, wherein:
- said success rate T1 is at least 70%, preferably at least 75%, even more preferably at least 80%, even more preferably at least 85%, even more preferably at least 90%, even more preferably at least 95%, even more preferably 100%; and/or
- said success rate T1 is achieved for at least two consecutive sessions, said success rate T1 preferably being achieved for at least two consecutive sessions as early as the fifteenth session carried out, more preferably as early as the twelfth session carried out, more preferably as early as the eleventh session carried out, more preferably as early as the tenth session carried out, more preferably as early as the ninth session carried out, more preferably as early as the eighth session carried out.

5. The method according to claim 2 wherein said success rate T2 is at least 70%, preferably at least 75%, more preferably at least 80%, more preferably at least 85%, more preferably at least 90%, more preferably at least 95%, more preferably 100%.

6. The method according to any one of the preceding claims, wherein said duration Dᵣₐ ranges from 1 second to 300 seconds, preferably from 3 seconds to 240 seconds, preferably from 5 seconds to 200 seconds, preferably from 10 seconds to 180 seconds, more preferably from 15 seconds to 120 seconds, preferably from 20 seconds to 100 seconds, preferably from 25 seconds to 90 seconds, preferably from 30 seconds to 60 seconds , more preferably from 25 seconds to 35 seconds.

7. The method according to claim 2 wherein said duration D_{rb}, ranges from 1 second to 300 seconds, preferably from 3 seconds to 240 seconds, preferably from 5 seconds to 200 seconds, preferably from 10 seconds to 180 seconds, more preferably from 15 seconds to 120 seconds, preferably from 20 seconds to 100 seconds, preferably from 25 seconds to 90 seconds, preferably from 30 seconds to 60 seconds, more preferably from 25 seconds to 35 seconds.

8. The method according to any one of the preceding claims, wherein said instruction and/or said at least one response is a visual stimulus, preferably a visual stimulus selected from a color, a shape, an image, an object, a light signal or any combination thereof.

9. The method according to any one of the preceding claims, wherein the step of detecting the response chosen by the model fish comprises detecting the passage of said model fish through a response zone, said response zone preferably comprising or consisting of a response line, said response line preferably comprising or consisting of a response orifice.

10. The method according to any one of the preceding claims, comprising an additional step (a-iv) and/or (b-v), said additional step comprising:
- dispensing a reward to said model fish when it has chosen at least one of said response(s) identical to said instruction during said duration Dᵣₐ , said reward preferably comprising food suitable for said model fish, more preferably food that is palatable and/or nutritionally poor for said model fish; or
- not dispensing any reward to said model fish when it has chosen at least one of said response(s) different from said instruction; or optionally, when it has chosen no response) during said duration Dᵣₐ, and, optionally, isolating and/or putting in the dark said model fish from said model fish, when it has chosen at least one of said response(s) different from said instruction; or optionally, when it has chosen no response; during said duration Dᵣₐ .

11. Method according to any one of claims 2 to 10, comprising an additional step (a-iv) and/or (b-v), said additional step comprising:
- dispensing a reward to said model fish when it has chosen at least one of said response(s) identical to said instruction during said duration (D_{rb}), said reward preferably comprising food suitable for said model fish, more preferably food that is palatable and/or nutritionally poor for said model fish; or
- not dispensing any reward to said model fish when it has chosen at least one of said response(s) different from said instruction; or optionally, when it has chosen no response; during said duration (D_{rb} ), and, optionally, isolating and/or putting in the dark said model fish from said model fish, when it has chosen at least one of said response(s) different from said instruction; or optionally, when it has chosen no response; during said duration (D_{rb} ).

12. The method according to any one of the preceding claims, wherein said model fish is selected from model fish belonging to the genera *Danio* sp., *Pseudotropheus* sp., *Gnathonemus* sp., *Pomacentrus* sp., *Phoxinus* sp., *Xenotoca* sp., *Astronotus* sp., *Oryzias sp., Nothobranchius sp., Astyanax sp.* and *Carassius* sp., preferably *Danio rerio, Metriaclima zebra, Gnathonemus petersii, Pomacentrus amboinensis, Phoxinus phoxinus, Xenotoca eiseni, Astronotus ocellatus, Oryzias latipes, Nothobranchius furzeri, Astyanax mexicanus* and *Carassius auratus.*

13. A device for obtaining conditioned model fish (1), comprising :
- a tank (2) comprising a wall (5) delimiting a cavity (6) suitable for containing water for fish, the cavity (6) comprising a starting zone (7) and a selection zone (8),
- a first partition (3) movable between a closed position in which the first partition (3) prevents movement of the model fish between the start zone (7) and the choice zone (8), and an open position in which the first partition (3) allows movement of the model fish between the start zone (7) and the choice zone (8),
- a second partition (4) separating the selection zone (8) into a first compartment (9) and a second compartment (10), and
- a first presentation element (11) for presenting an instruction in the start zone (7), a second presentation element (12) for presenting a first response in the first compartment (9) and a third presentation element (13) for presenting a second response in the second compartment (10), said device being **characterized in that** the first presentation element comprises a first display panel, on which the instruction is shown, the second presentation element comprises a second display panel, on which the first response is shown, and the third presentation element comprises a third display panel, on which the second response is shown ; and
wherein one of the first response and the second response is identical to the instruction and the other of the first response and the second response is different from the instruction.

14. The device according to claim 13, wherein the instruction is a color, and one of the first response and the second response is a color identical to the instruction color and the other of the first response and the second response is a color different from the instruction color; and/or
in which the first movable partition is opaque to light radiation in a vacuum wavelength range comprised between 380 and 780 nanometers.

15. Use of a conditioned model fish obtainable by the method according to any one of claims 1 to 12, to characterize a cognitive impairment or deficiency, resulting for example from a neuropsychiatric or neurological disease; such as a neurodegenerative disease, an addiction or a brain lesion; in particular selected from hyperactivity, autism, schizophrenia, Parkinson's disease, Alzheimer's disease, and a brain lesion; in particular with regard to effects on working memory and/or cognitive abilities and/or learning abilities in a model fish; or to study brain regeneration capacity in a model fish; or to characterize working memory and/or cognitive abilities and/or learning abilities in a model fish; or to identify molecules, compounds, compositions or formulations modifying working memory and/or cognitive abilities and/or learning abilities in a model fish;
wherein said model fish is selected from fish models of hyperactivity, autism, schizophrenia, Parkinson's disease, Alzheimer's disease and brain injury.
